# EUROPEAN PATENT SPECIFICATION

(11) **EP 3 263 590 B1**
(45) Date of publication and mention of the grant of the patent: **16.12.2020**
(21) Application number: 17001115.9
(22) Date of filing: 29.06.2017
(51) Int. Cl.: C07K 14/18

(54) **FISH VIRUS**
FISCHVIRUS
VIRUS DE POISSON

(30) Priority: 30.06.2016 GB 201611512
(43) Date of publication of application: 03.01.2018
(60) Divisional application: 20176389.3
(73) Proprietor: Pharmaq AS, 7863 Overhalla (NO)
(72) Inventor: BRUDAL, Espen, 7863 Overhalla (NO); HAUGLAND, Oyvind, 7863 Overhalla (NO); JOHANSEN, Renate, 7863 Overhalla (NO); VESTVIK, Nils Frederik, 7863 Overhalla (NO)
(74) Representative: Mannion, Sally Kim

(56) References cited:
- WO-A1-2011/131600
- DATABASE Geneseq [Online] 9 July 2009 (2009-07-09), "Human autosome 18 nucleotide sequence SEQ ID NO:23389.", XP002773428, retrieved from EBI accession no. GSN:AWG07403 Database accession no. AWG07403

<110> Pharmaq AS
   Johansen, Renate
   Brudal, Espen
   Vestvik, Nils Fredrik
   Haugland, Oyvind
<120> Fish Virus
<130> ZP000160A
<150> GB1611512.3
   <151> 2016-06-30
<160> 26
<170> PatentIn version 3.5
<210> 1
   <211> 11336
   <212> DNA
   <213> Flaviviridae Virus
<400> 1
   aatttacatt ctgacacgcc aatttagtcg ctaccaacat ttaggtcgct acacagttta      60
   accactggtt caacgtttca gaatattgtt aaattacacc aattcagaat tgaacaacac     120
   ccaatattga atggtattat aaaacaaaca caatataaca taatataata aattataata     180
   aacaaaaaac acacacataa aacaaacaaa caaaaaacaa aacactcaaa caacatactt     240
   agaagaataa cccggctggg tccacttctc aagacaatga gttacagagg aatcatgcgt     300
   ctcgccggaa cccccttcag ggttgcccgc cgtgtcaccc aaggctttgg gggcaccaag       360
   agagccatcc accatgccag gcaatcagcc ggccgtggtc tgcgtcacgt ccagagagga     420
   cgcggcaaaa tacagcagac ctttgagatc ctgctcaaga gagcagccgt cattctgagg     480
   agaatctgga ataaaccacc acaaatcccc aagaatatca acggtgtgag gcgactcaga     540
   aagatgataa ccaacctcaa ctccgtgcag caggccatca aaggcccaac gaagcgtaag     600
   aagaagagtt ccggaatggg cacagcaggc ttgctgaccc tcctcatgat cttctttgcc     660
   acccaagtga tccctggagg gacctccaca tcaccaacac cttcacccac caccccaaca     720
   gacatctcac caatggtgat agcatgtgtc cgcaaggaca agacttcctc tatggatcta     780
   ctactggtga atgaggaagg actttttgca aagatggaca acaccaaaga tcctgaactt     840
   ttcatacgca acaaccaaac ctatggcatt gatctcacag agtgttccac cggcttccag     900
   ccggacacca actactcatc accagaattg aaatacgatg aacatgactg tgccctgatc     960
   agagcaggag tgctaccaac ggcagcagac acatgtttcg gaactacatc tctgaggcag    1020
   agacacatcc attcaaccct ctatcaagac ctttcaagga agactagaaa cagacgcgat    1080
   ctgtcacgtg atactattca gaagtatgag atacacacca accagccatc agaaggctgg 1140
   atgaagaccc tatcaacatg ggagaccaaa gtgctatcaa acacagacat aaccatagcc 1200
   cttctggccc tggttatggc ctattttaaa gtacccactg tccacctgct gctgatcatc 1260
   ctcttttcca aggtttacct cggggtagcc gattcgacct cttcaaccac tgaaatgagc 1320
   ggaaaaacac tctgctctga cccggaagaa atgtcaacca tgactggagt agcacacacc 1380
   tcagtcagcc tccgcatgga ctacaagcat tgctatcaga tccaaccggt aggcatggac 1440
   acaatcacgg ccagaatgag ctacccctat atagacaatg cactcacagg atacaaaata 1500
   tcctggttgg acattagttc caactcagtt tcatccgaca gatgcccagg agatggagaa 1560
   cccgcctgcc caaaatcagg accatattcc atctgcgcta gaggatacca tgatcgtggc 1620
   tggaccactg gctgcttcct cttcggacaa ggaccggtat gtacttgtgc tgaactagcc 1680
   ctcaaacatc cgatacaggt ctatacaatc gacccaacaa agatgggagc ccgcttaacg 1740
   gtctcctttg gacgcacaga aaatcagaac tattacaaca tggacacaaa cacaggaagt 1800
   ttcgacatca cagacaaagc cgactcgtcc tcacgagtca gagtaacatg ccagctacca 1860
   cacgcagaac ttgccaccaa gtacctgata aagatggcac caaatgatgg cgaatcatat 1920
   ctcgtagata gattggcctt tgaatcaatg aaactcccct ggtccgtatg gaccacgtct 1980
   atcccaacca gccttagcaa gattgaagat ggcaacgtcc atgatgccga gacctatata 2040
   acttggagag acaccaccct ccattacgtg agattttcag aagcccataa cgtcgaatcc 2100
   atggtcaaga atttcctgga caagaacggg aagataatca tagccgagat ggccaaagga 2160
   tcaggaagag cctccccagg caccaagatg cccttagaca ttagccccac agactgcagt 2220
   ttcatgctta attcccttaa aatacacacc accagtgtga aggactgtgc agacacaacc 2280
   aactacgcat tcaaactgat cgaaggcagc agaatactca ccactcacga tggagttgtc 2340
   acctttaact tagatatggc agttcctagc cagtgcaggt tagtcgtcaa gggacgtagc 2400
   cgcagccaag ttggctctgg atacacctcc aactactgca cccttttcga tcaggcacac 2460
   atagtcactt caaccaaaga agtactgaaa atgcactgcc cacccggaga gcatgaaatt 2520
   aaactaggca acaacaagca tcaagtgaat gtcccccaaa tgggtatcac aggatggctt 2580
   agcagatgga ccactaacgt tctggaagtc aaagacagaa taaccataga tggagcagtt 2640
   aagagaccag tactagacat gttcaagaac ttctttgcca aaggagcagg cttcctagga 2700
   agccttttcg gaatgacatc aacaatcatg atggcagtga cgggaggagc catagtatgg 2760
   ctcggaaccc taatgacagg taacatgaag aatctcacag tcatcatagg acttatcctc 2820
   atggccccct tagtaataac ggaggtagaa gcagagaagt atggttgcgt ggctgacaca 2880
   gatgaatggt caatggaatg tggcagtatc aaggacgtca tgttcgattc atcaatcaag 2940
   ctgtatgtcc ctcaattagg agacagctac aacatagact tagacctcat ggacggctta 3000
   acaatcctta ggatgccaaa cagagaacaa atccagaaag ccgccaaagc cttaggctcc 3060
   gtactccaca aagtctacac agatgaagga gaagaatgcg ccactgatgg tacagaccat 3120
   gacgtgaatg acaccacctc ttgggatcaa acctcccgta aactcaaaga tgatgggaaa 3180
   caaggagcac taatcacctg ccataattca gaaaaacact gcaagaactg ccgagaaatc 3240
   tttttggaag tcaatgaggt agacgatgtc gatggatgga tgtatgacac caaagagtta 3300
   gaaaccacca cctccacacc accagtctac tataaaggcg aagaaagacc agaatcttac 3360
   ctcaaagacc tcacagtgga ctgccgtagg gcagggaaac attataaatg ttcggcccac 3420
   ggagttacct ggtttggaac catccacact gatgaagaga aggaatactg cctgacacac 3480
   aaagtcgaag gcactgtcac ccaaatattt ggagcaaaaa ggagaagttg gaggagctgc 3540
   ggaatcagct gctcctctct ctgcgagaga gtcaacacca ccacaaaagg acacatctgg 3600
   cccaagagat ttggaggacc agaatgcaac ttcaactgct ggccaatcac caaaaaacag 3660
   aacgaagaag atgagaagaa agaaatgtca gcagccgaga acgcctatag aggcggtatc 3720
   atcctacgca aggaagctag cgagagactc ctcaagagat ctctctataa agacagagcc 3780
   ctgaccttta actccacctg gtgcaaacag aagaaatgca acaccactgt aatcaaagcc 3840
   aacgatggcg acgaggaaat agatgaaggt tttacccccg tagacaagga agaagaacac 3900
   gaccatgatg aagatttcat atccggaaaa actagcctag ccacagccat gcaagagaac 3960
   ggccacaacc aagccacaat tgatgcagcc actggtttct acaagatgca gagggccacc 4020
   ggcaccaaag ccgttaacca gctcatcaat gagctagtca aagcctcagg aggtgagaga 4080
   acagccagca atgacaattt tatcaattct ccagcaacat tgaagagtga gatggagttg 4140
   ctttcatact cccccacccg aattgaaaaa gccctcagga gctacaagaa aggaacagaa 4200
   gcgaaaggca ccaaacagat taggaactta attagagaac tactgacaga tggagaacaa 4260
   ccactcacta acggtgcaag cgaactaacc accacagcca ctgttgttct gggttttcct 4320
   tagcttcatt cgcctttgag cccgcgactg aagcctttga atggctcctt aagacttttt 4380
   tgacactaac aaccctcata tttaaccatg ggtttcgtgt aagaccggtt ggagctaaga 4440
   cccagatcac catcttcgca acagcactac tccaccacct aacaccatta tcaatggaac 4500
   caatcagtca atttggttgg tatgcagtac agactgccat tgatagtccg actaggttta 4560
   tcatcagctt gctcatcaca ctcgaagtca tacttatctt catgttccct aaaccgattt 4620
   acaacatgac acgatggtat tggtcttcct acaaaaaaga gagcattatg cagatggtgg 4680
   caggaggagc gatctgtctg gccttagcac aagcacccaa gaacctttcc tacatcctgt 4740
   tcccagtggc ggccctctac atcctcatga ggaggggcgc tactgttcga acacggcacc 4800
   tcacatccca actgttagta cccatagaca aagacgatga tccaccgaca ggagtcggta 4860
   ccataaccct taaggaaggc aagttacaat atgatatgag tagcctccac gaccacaggg 4920
   tcgtgacgga ttccactagg gttttcgtgg tggtattaat tgccattttt ggcctcctag 4980
   actacagggc tggaatagcc gccctcatcc tggcctttgg ttactttaag aatgccaaag 5040
   ccgacctgtt cgacaccttc aacattgccg gacccgagag agtggagaaa gatactttct 5100
   ctggccccga aggcatatat gaggtataca ataggacacc attaggagat gaacatcttg 5160
   gttatggtta caacaagaac aacatttttc acaccatgta ccacattaca aaaggactgc 5220
   ccctagaatt cgaagatgaa gtgaccaccc cctacgatta tagtgatgaa ctggattgga 5280
   tcacctacgg tggcccctgg gctttcgcca aacgggaagg cagggacctc ttcgtctatc 5340
   tccacaggga gcatagactt agaccactaa aaatcagttc agaagacgat aaagtctcac 5400
   tgggaacaac gaggaccata gcatttggcg actctggctc accagtacta gctagagacg 5460
   ccgacggaac actgacaccc gtctcattag ctggacacac ggtaccacta caggatccaa 5520
   catatgaagc catgatttta cccaccccta ctacaggcat ggacctttca aaatttcaga 5580
   ggaaattcga cacaagaggc ccaggatgga atccaatagt gttaagatgt ggagcaggaa 5640
   aaacaagaca cgttctgaga agagccgtgg ttagatctac ggaaacagga cgtaactgtc 5700
   tacttttagc acccaccagg acggtagcag gagaaatgta tgaagcatta aaagatttag 5760
   atgtagggat tgatatcaca ggcaaggtcg aaagaagaaa caagagaaac cttatcatgt 5820
   gccactcaac agcagtaaac cgcctcctcc accatagcct caacatccac aactacaaga 5880
   acgtctttgt ggacgaagca cacatgcaag accccatgac tatagcattg ctaggttttc 5940
   tcgaagaaag aaccagagac accagcaatg acatccaggt ctttccgatg acggccacct 6000
   ggtgggacca ctatgaaact gaaactaatt ttccagtgat tgacaccaaa cttaacgatg 6060
   aaaaaatgat cgttgatgca ataaaagact cacttagtaa gaatagaaga agcattacat 6120
   tcgtccccac catcaagaaa gctgaagaac tctacaccag tgtaggtgaa ggagaaagaa 6180
   tcatcctcaa tagatatacc tacagaagtg aaatcagcaa aatcaaaaca gataaggaca 6240
   agcccatagc catctttgca accaatatag ccgaggtcgg cctaaacgcc aatttagacg 6300
   acgtttttga cctccagcaa cagatagttt ttaaggagag tgtggccggt gtaatcacta 6360
   gagaaacatc cggcgcctcc aaagcatcca ctacacagag aaggggaaga gttggaagag 6420
   tcagatcagg cacttatcag tacttagacc aagccgcaac acacaacatc atgcatgact 6480
   cagatatcat ccgtaaggag gctgccatag tattaaaaat gttaggagtc gacagtgaga 6540
   gcacccgatt ttgccaactc ccccagatcc cacccatctc cctatcaaaa aagaaaggca 6600
   taatgaggtt gaccaacgat aatgataaca taacaatcta cttagcccac tacgtcactg 6660
   acgattcagg aatagaagta gattggacga ccggagtaca cccttgctca tgtaacgatt 6720
   gcaaaacagc caataataag ttcatgatag tagaccccag atcacactca tacatgatgc 6780
   acaagcagaa tggagagcca ctcatcaaag gcgacacccg catttacgaa ctccgtggat 6840
   cctccactat cacaggtagc atacttcgct acattaaagc gtctgccaac ccaacatcca 6900
   gactaatgat gtcagtattc gcggtggagg tggattggac ctgggctgac ccttacgtga 6960
   ctagtttcaa ccaaggctac atagccatca ttaaacaact ccccatatgg ataggagtcc 7020
   acctaaaaat agaaagggta gcagctgcta tcctgtggct cctgatttgg aagatcataa 7080
   catacatttt tgggggaaga agcaatgaca gtaaagtcgt cataaacaat ggaaacatga 7140
   gttcactacc cggcttagcc atattagcaa caggcattgc cctacacttc gttgacaaat 7200
   tcaactcagg tcatttacca gtagccctag tatcggtggg gtgcatctac atcttaggtg 7260
   tcctctacta ctcacagaga caggttagct ataagaccac agcgagtgag gctgcttttt 7320
   acctctttgc cgtctttgcc acgctagtag gactattcat ctatatactt agaagccaca 7380
   gggacgtttt tgatgatttt acatcaccca ggaaacgcta ctaccacacg gaagaagaca 7440
   agaaagaaca tgagcccata actgaggctc cacctcttag cattagcaag ggatttacct 7500
   ttttcctggg gttagtgccc ctcatcaacc tcctggcgga gacaatgaca cagagcaaag 7560
   gcctacaaaa catgaatgtc aacactctac gtcattcact tagcggtggt gcagaagtca 7620
   aaccccccgt acttatcacc atcttcaccg tctttgccat gttccagggt tcctcgctca 7680
   cagactgcat cgtcacactt tgcctctctg tcctttatta ccttttctat ttcggacact 7740
   cagcacacgt taacaccaaa agtgcagtca cagccagccc gggaaacacc accggagatt 7800
   cactaggttt ctccaatgca ccccttaaag aagatggggt gattaaggtg tttaccggtc 7860
   tggtactact gacagacgcc ctacttagac aggaaatgac ggtggctcta gcacttaatg 7920
   cttttttcac actgataccc ctcttcctct ctaaaggaga ggaacccagc tacgcattct 7980
   acatgataat ccaagccggt ttagaacaag aacttctcct ggtcataggc tccgtagcaa 8040
   cagccctctt ttgcagatcc ataagccgga ccagaaaaac agggggagcc gtgctatcca 8100
   tgagttttaa ctcagtcgtt ggacgacata aaaatctcga accaggtttc caagactaca 8160
   agacgagaat gaacggactt ggcaagaaca ccttctacaa cctgagaaaa cacatggtcc 8220
   ccgtacacga caaggggaca gtatgctcca gaggctacca caaaatgaat gaactcctca 8280
   gataccacga tttagaaatt agcggaaaaa tcttagaatt aggatgtggt tctggtggct 8340
   ttacccaaag aatggtatta gagaaaaaag tcgatagaat tgacgccatc tcttggggac 8400
   cagataagaa agaccacaat ctgtttgaac tattcaaaaa atcaacacca ggccatggaa 8460
   aagttaggta ctcagtagga gaagcattca ccaacaggga ttttgagaac tacgactggg 8520
   tcgtgatgga cataggagaa caaagtccag acatggcccg ggagaggacc agagacctgt 8580
   ggaggttaga ctggatcacc aagaaagtca tcccccatgc taaagtcatc atgaagatct 8640
   tgtcccctac agatatagac gtcctaagag ccataccacc aggttacagg atcgttagac 8700
   tatatcattc ttggaactca aattttgaaa tgtacatgat cccgggcgac ccctcagata 8760
   acatcactag gatgaaccag ctcctcaaaa accttaacat caggctccag caaacactgg 8820
   aaggagaaca tgtggagtta ggggcagaac aagtaaaact gccttcagtc gtggagaaaa 8880
   taggcactaa ccttggaaga gaatatgagg attggtgcct ggcagagaga ttagaacaac 8940
   tccctgccct cacacaggtc gaaaggccta aattggacca ctttcagtat ctggccagtt 9000
   tcttcactga ggcccgcaag ctacctggca catgctctaa ccccattatc atgaccttct 9060
   ggaatggact caccaaactt attcctgggg ctgggggatt caccatgaca gacaccacac 9120
   acgatggtag ttacaagatg ttccaaaaga agatggataa cccaccaagc accgatcaaa 9180
   cctacatccc tgaactccat agagtttttg acaagatgag tgactttctc tttaaaagaa 9240
   acaaaccaag aagactcacc agagaggaaa tcatccaaat agttaggaac gacgcagccg 9300
   tcggaaacat acatccagac atcatttggc caacagcact ggaagcccta gaatcagaaa 9360
   gtttctggaa agtagttgag gaagaaacac acctccattc ccaaggaaaa tgccgctatg 9420
   gcatattcaa caccatgggc aagagggaaa agaaggacac agacggtgaa agaagtggct 9480
   cacgcataat atgctaccta ccacttgtgg aaagagtcat tgagatggac gtcctaggtt 9540
   tcctcaataa agatcacatc gcaggaccgg aaagtctgcc atgtggggtg tcaggagtta 9600
   gtccctatca ttacccagac atattttcga agaaagcagg actagatcca gaaacaggag 9660
   acatgaccaa agcagtcatc caagatgaca cagcagcctg ggacaccaga gtaagaaaag 9720
   atgtcctgca catggaacgc gacttcatcc ttaaacacac cacagacccc taccacgtca 9780
   acctcataaa aacacagtac aaaatctacg gcgaaccgat ccttacgatc accagaccct 9840
   tcaccgttga caggtcggta gttgacgtcc tccaaggcta cggacagaga tgttctggca 9900
   cagtagtcac ctacgccatg aacaccatca caaatgcatg tgtccagaca ctcaggttct 9960
   gggaggccat ggacgtagac ttggaggaag tcctaaatga catgccccac atcctcaaca 10020
   gtatgatgat cagtggagac gacatgctct tcatggtaga accatggcta gccaaaaaac 10080
   tcagttcatc tctcaaagtc atcaattcac tcggcttccc tcgcaaagga ctcacaacat    10140
   ggcaggaatc acccataatc acccagtttg ataaagtcta cttctgcagt cataagttca    10200
   ccatcgccag agtggccggt gaaagacgag cattcttaga caaagacctt ttcgagatca    10260
   tcgggaaaac ccaactcgtt ctgggaggac tacacagccc acacgagata gccggacacg    10320
   caaaaagcgt agccatctac atgttcgtca ccttcttcca caacagagcc gtgagaaagg    10380
   tggcattggc catcctatca gcactgcccg acgaactggt gcccatgggg aatgtcgcca    10440
   accccttctt tctggatcac tcctggatca gcaatgaaga cctaatcact atcttcaaca    10500
   atgtccacgg cacacatgcc aggagctaca ctgacattgg ttacacgtac aaaacgttgg    10560
   ccaagatcag aggagaccca ttggatcatg aagaacggac cacatggaac aaagacctca    10620
   agagaaaaat cgagtcgatc tccacccact acaacagtgg tataacaaat gggagtttct    10680
   ggcacgagca actccgaaca aacgttagtt accacagcct attctaggac tgtagcacca    10740
   cttagtttag ataagaaaac aaacaaaaaa ccttactata aaaaataaaa aataagaaaa    10800
   tacttgctat gaaataactt agatttagaa aatgtattaa cttagattta gagaatgtat    10860
   tcatttactg tttagttagg accaagagga ctgctactct aagacgtgag gtcgttaaat    10920
   aacatcgacc aagtacgaac acttacaact agtggtcgga agataacaaa aagccgtaac    10980
   ctatctccgg tttttcaata aatagagtag aaagaaaaga accctgaatc ccccgcgtga    11040
   tcaggtttaa ataaaagcaa cgtggaaaaa ttattagctt aatgaaaatg tttattaaaa    11100
   ttacgcttat attatgtaaa tattattgtt ttataaaatg ttgtactaaa tgtaaatgac    11160
   atgtatatag tcaccttacc actccagtat tacacaaaca ccattacagt atcgggcgag    11220
   ttgttacatt cttatgttca tggaactaga attggtgtat tcctaccaag ctgtaggttt    11280
   aacggaaaca ctaattttgg aactacttaa tatacagaag tagaaacaag cccccc        11336
<210> 2
   <211> 4047
   <212> DNA
   <213> Flaviviridae Virus
<400> 2
   atgagttaca gaggaatcat gcgtctcgcc ggaaccccct tcagggttgc ccgccgtgtc      60
   acccaaggct ttgggggcac caagagagcc atccaccatg ccaggcaatc agccggccgt     120
   ggtctgcgtc acgtccagag aggacgcggc aaaatacagc agacctttga gatcctgctc     180
   aagagagcag ccgtcattct gaggagaatc tggaataaac caccacaaat ccccaagaat     240
   atcaacggtg tgaggcgact cagaaagatg ataaccaacc tcaactccgt gcagcaggcc     300
   atcaaaggcc caacgaagcg taagaagaag agttccggaa tgggcacagc aggcttgctg 360
   accctcctca tgatcttctt tgccacccaa gtgatccctg gagggacctc cacatcacca 420
   acaccttcac ccaccacccc aacagacatc tcaccaatgg tgatagcatg tgtccgcaag 480
   gacaagactt cctctatgga tctactactg gtgaatgagg aaggactttt tgcaaagatg 540
   gacaacacca aagatcctga acttttcata cgcaacaacc aaacctatgg cattgatctc 600
   acagagtgtt ccaccggctt ccagccggac accaactact catcaccaga attgaaatac 660
   gatgaacatg actgtgccct gatcagagca ggagtgctac caacggcagc agacacatgt 720
   ttcggaacta catctctgag gcagagacac atccattcaa ccctctatca agacctttca 780
   aggaagacta gaaacagacg cgatctgtca cgtgatacta ttcagaagta tgagatacac 840
   accaaccagc catcagaagg ctggatgaag accctatcaa catgggagac caaagtgcta 900
   tcaaacacag acataaccat agcccttctg gccctggtta tggcctattt taaagtaccc 960
   actgtccacc tgctgctgat catcctcttt tccaaggttt acctcggggt agccgattcg 1020
   acctcttcaa ccactgaaat gagcggaaaa acactctgct ctgacccgga agaaatgtca 1080
   accatgactg gagtagcaca cacctcagtc agcctccgca tggactacaa gcattgctat 1140
   cagatccaac cggtaggcat ggacacaatc acggccagaa tgagctaccc ctatatagac 1200
   aatgcactca caggatacaa aatatcctgg ttggacatta gttccaactc agtttcatcc 1260
   gacagatgcc caggagatgg agaacccgcc tgcccaaaat caggaccata ttccatctgc 1320
   gctagaggat accatgatcg tggctggacc actggctgct tcctcttcgg acaaggaccg 1380
   gtatgtactt gtgctgaact agccctcaaa catccgatac aggtctatac aatcgaccca 1440
   acaaagatgg gagcccgctt aacggtctcc tttggacgca cagaaaatca gaactattac 1500
   aacatggaca caaacacagg aagtttcgac atcacagaca aagccgactc gtcctcacga 1560
   gtcagagtaa catgccagct accacacgca gaacttgcca ccaagtacct gataaagatg 1620
   gcaccaaatg atggcgaatc atatctcgta gatagattgg cctttgaatc aatgaaactc 1680
   ccctggtccg tatggaccac gtctatccca accagcctta gcaagattga agatggcaac 1740
   gtccatgatg ccgagaccta tataacttgg agagacacca ccctccatta cgtgagattt 1800
   tcagaagccc ataacgtcga atccatggtc aagaatttcc tggacaagaa cgggaagata 1860
   atcatagccg agatggccaa aggatcagga agagcctccc caggcaccaa gatgccctta 1920
   gacattagcc ccacagactg cagtttcatg cttaattccc ttaaaataca caccaccagt 1980
   gtgaaggact gtgcagacac aaccaactac gcattcaaac tgatcgaagg cagcagaata 2040
   ctcaccactc acgatggagt tgtcaccttt aacttagata tggcagttcc tagccagtgc 2100
   aggttagtcg tcaagggacg tagccgcagc caagttggct ctggatacac ctccaactac 2160
   tgcacccttt tcgatcaggc acacatagtc acttcaacca aagaagtact gaaaatgcac 2220
   tgcccacccg gagagcatga aattaaacta ggcaacaaca agcatcaagt gaatgtcccc 2280
   caaatgggta tcacaggatg gcttagcaga tggaccacta acgttctgga agtcaaagac 2340
   agaataacca tagatggagc agttaagaga ccagtactag acatgttcaa gaacttcttt 2400
   gccaaaggag caggcttcct aggaagcctt ttcggaatga catcaacaat catgatggca 2460
   gtgacgggag gagccatagt atggctcgga accctaatga caggtaacat gaagaatctc 2520
   acagtcatca taggacttat cctcatggcc cccttagtaa taacggaggt agaagcagag 2580
   aagtatggtt gcgtggctga cacagatgaa tggtcaatgg aatgtggcag tatcaaggac 2640
   gtcatgttcg attcatcaat caagctgtat gtccctcaat taggagacag ctacaacata 2700
   gacttagacc tcatggacgg cttaacaatc cttaggatgc caaacagaga acaaatccag 2760
   aaagccgcca aagccttagg ctccgtactc cacaaagtct acacagatga aggagaagaa 2820
   tgcgccactg atggtacaga ccatgacgtg aatgacacca cctcttggga tcaaacctcc 2880
   cgtaaactca aagatgatgg gaaacaagga gcactaatca cctgccataa ttcagaaaaa 2940
   cactgcaaga actgccgaga aatctttttg gaagtcaatg aggtagacga tgtcgatgga 3000
   tggatgtatg acaccaaaga gttagaaacc accacctcca caccaccagt ctactataaa 3060
   ggcgaagaaa gaccagaatc ttacctcaaa gacctcacag tggactgccg tagggcaggg 3120
   aaacattata aatgttcggc ccacggagtt acctggtttg gaaccatcca cactgatgaa 3180
   gagaaggaat actgcctgac acacaaagtc gaaggcactg tcacccaaat atttggagca 3240
   aaaaggagaa gttggaggag ctgcggaatc agctgctcct ctctctgcga gagagtcaac 3300
   accaccacaa aaggacacat ctggcccaag agatttggag gaccagaatg caacttcaac 3360
   tgctggccaa tcaccaaaaa acagaacgaa gaagatgaga agaaagaaat gtcagcagcc 3420
   gagaacgcct atagaggcgg tatcatccta cgcaaggaag ctagcgagag actcctcaag 3480
   agatctctct ataaagacag agccctgacc tttaactcca cctggtgcaa acagaagaaa 3540
   tgcaacacca ctgtaatcaa agccaacgat ggcgacgagg aaatagatga aggttttacc 3600
   cccgtagaca aggaagaaga acacgaccat gatgaagatt tcatatccgg aaaaactagc 3660
   ctagccacag ccatgcaaga gaacggccac aaccaagcca caattgatgc agccactggt 3720
   ttctacaaga tgcagagggc caccggcacc aaagccgtta accagctcat caatgagcta 3780
   gtcaaagcct caggaggtga gagaacagcc agcaatgaca attttatcaa ttctccagca 3840
   acattgaaga gtgagatgga gttgctttca tactccccca cccgaattga aaaagccctc 3900
   aggagctaca agaaaggaac agaagcgaaa ggcaccaaac agattaggaa cttaattaga      3960
   gaactactga cagatggaga acaaccactc actaacggtg caagcgaact aaccaccaca      4020
   gccactgttg ttctgggttt tccttag                                          4047
<210> 3
   <211> 1348
   <212> PRT
   <213> Flaviviridae Virus
<400> 3
   Met Ser Tyr Arg Gly Ile Met Arg Leu Ala Gly Thr Pro Phe Arg Val
   1               5                   10                  15
   Ala Arg Arg Val Thr Gln Gly Phe Gly Gly Thr Lys Arg Ala Ile His
               20                  25                  30
   His Ala Arg Gln Ser Ala Gly Arg Gly Leu Arg His Val Gln Arg Gly
           35                  40                  45
   Arg Gly Lys Ile Gln Gln Thr Phe Glu Ile Leu Leu Lys Arg Ala Ala
       50                  55                  60
   Val Ile Leu Arg Arg Ile Trp Asn Lys Pro Pro Gln Ile Pro Lys Asn
   65                  70                  75                  80
   Ile Asn Gly Val Arg Arg Leu Arg Lys Met Ile Thr Asn Leu Asn Ser
                   85                  90                  95
   Val Gln Gln Ala Ile Lys Gly Pro Thr Lys Arg Lys Lys Lys Ser Ser
               100                 105                 110
   Gly Met Gly Thr Ala Gly Leu Leu Thr Leu Leu Met Ile Phe Phe Ala
           115                 120                 125
   Thr Gln Val Ile Pro Gly Gly Thr Ser Thr Ser Pro Thr Pro Ser Pro
       130                 135                 140
   Thr Thr Pro Thr Asp Ile Ser Pro Met Val Ile Ala Cys Val Arg Lys
   145                 150                 155                 160
   Asp Lys Thr Ser Ser Met Asp Leu Leu Leu Val Asn Glu Glu Gly Leu
                   165                 170                 175
   Phe Ala Lys Met Asp Asn Thr Lys Asp Pro Glu Leu Phe Ile Arg Asn
               180                 185                 190
   Asn Gln Thr Tyr Gly Ile Asp Leu Thr Glu Cys Ser Thr Gly Phe Gln
           195                 200                 205
   Pro Asp Thr Asn Tyr Ser Ser Pro Glu Leu Lys Tyr Asp Glu His Asp
       210                 215                 220
   Cys Ala Leu Ile Arg Ala Gly Val Leu Pro Thr Ala Ala Asp Thr Cys
   225                 230                 235                 240
   Phe Gly Thr Thr Ser Leu Arg Gln Arg His Ile His Ser Thr Leu Tyr
                   245                 250                 255
   Gln Asp Leu Ser Arg Lys Thr Arg Asn Arg Arg Asp Leu Ser Arg Asp
               260                 265                 270
   Thr Ile Gln Lys Tyr Glu Ile His Thr Asn Gln Pro Ser Glu Gly Trp
           275                 280                 285
   Met Lys Thr Leu Ser Thr Trp Glu Thr Lys Val Leu Ser Asn Thr Asp
       290                 295                 300
   Ile Thr Ile Ala Leu Leu Ala Leu Val Met Ala Tyr Phe Lys Val Pro
   305                 310                 315                 320
   Thr Val His Leu Leu Leu Ile Ile Leu Phe Ser Lys Val Tyr Leu Gly
                   325                 330                 335
   Val Ala Asp Ser Thr Ser Ser Thr Thr Glu Met Ser Gly Lys Thr Leu
               340                 345                 350
   Cys Ser Asp Pro Glu Glu Met Ser Thr Met Thr Gly Val Ala His Thr
           355                 360                 365
   Ser Val Ser Leu Arg Met Asp Tyr Lys His Cys Tyr Gln Ile Gln Pro
       370                 375                 380
   Val Gly Met Asp Thr Ile Thr Ala Arg Met Ser Tyr Pro Tyr Ile Asp
   385                 390                 395                 400
   Asn Ala Leu Thr Gly Tyr Lys Ile Ser Trp Leu Asp Ile Ser Ser Asn
                   405                 410                 415
   Ser Val Ser Ser Asp Arg Cys Pro Gly Asp Gly Glu Pro Ala Cys Pro
               420                 425                 430
   Lys Ser Gly Pro Tyr Ser Ile Cys Ala Arg Gly Tyr His Asp Arg Gly
           435                 440                 445
   Trp Thr Thr Gly Cys Phe Leu Phe Gly Gln Gly Pro Val Cys Thr Cys
       450                 455                 460
   Ala Glu Leu Ala Leu Lys His Pro Ile Gln Val Tyr Thr Ile Asp Pro
   465                 470                 475                 480
   Thr Lys Met Gly Ala Arg Leu Thr Val Ser Phe Gly Arg Thr Glu Asn
                   485                 490                 495
   Gln Asn Tyr Tyr Asn Met Asp Thr Asn Thr Gly Ser Phe Asp Ile Thr
               500                 505                 510
   Asp Lys Ala Asp Ser Ser Ser Arg Val Arg Val Thr Cys Gln Leu Pro
           515                 520                 525
   His Ala Glu Leu Ala Thr Lys Tyr Leu Ile Lys Met Ala Pro Asn Asp
       530                 535                 540
   Gly Glu Ser Tyr Leu Val Asp Arg Leu Ala Phe Glu Ser Met Lys Leu
   545                 550                 555                 560
   Pro Trp Ser Val Trp Thr Thr Ser Ile Pro Thr Ser Leu Ser Lys Ile
                   565                 570                 575
   Glu Asp Gly Asn Val His Asp Ala Glu Thr Tyr Ile Thr Trp Arg Asp
               580                 585                 590
   Thr Thr Leu His Tyr Val Arg Phe Ser Glu Ala His Asn Val Glu Ser
           595                 600                 605
   Met Val Lys Asn Phe Leu Asp Lys Asn Gly Lys Ile Ile Ile Ala Glu
       610                 615                 620
   Met Ala Lys Gly Ser Gly Arg Ala Ser Pro Gly Thr Lys Met Pro Leu
   625                 630                 635                 640
   Asp Ile Ser Pro Thr Asp Cys Ser Phe Met Leu Asn Ser Leu Lys Ile
                   645                 650                 655
   His Thr Thr Ser Val Lys Asp Cys Ala Asp Thr Thr Asn Tyr Ala Phe
               660                 665                 670
   Lys Leu Ile Glu Gly Ser Arg Ile Leu Thr Thr His Asp Gly Val Val
   675 680 685
   Thr Phe Asn Leu Asp Met Ala Val Pro Ser Gln Cys Arg Leu Val Val
   690 695 700
   Lys Gly Arg Ser Arg Ser Gln Val Gly Ser Gly Tyr Thr Ser Asn Tyr
   705 710 715 720
   Cys Thr Leu Phe Asp Gln Ala His Ile Val Thr Ser Thr Lys Glu Val
   725 730 735
   Leu Lys Met His Cys Pro Pro Gly Glu His Glu Ile Lys Leu Gly Asn
   740 745 750
   Asn Lys His Gln Val Asn Val Pro Gln Met Gly Ile Thr Gly Trp Leu
   755 760 765
   Ser Arg Trp Thr Thr Asn Val Leu Glu Val Lys Asp Arg Ile Thr Ile
   770 775 780
   Asp Gly Ala Val Lys Arg Pro Val Leu Asp Met Phe Lys Asn Phe Phe
   785 790 795 800
   Ala Lys Gly Ala Gly Phe Leu Gly Ser Leu Phe Gly Met Thr Ser Thr
   805 810 815
   Ile Met Met Ala Val Thr Gly Gly Ala Ile Val Trp Leu Gly Thr Leu
   820 825 830
   Met Thr Gly Asn Met Lys Asn Leu Thr Val Ile Ile Gly Leu Ile Leu
   835 840 845
   Met Ala Pro Leu Val Ile Thr Glu Val Glu Ala Glu Lys Tyr Gly Cys
   850 855 860
   Val Ala Asp Thr Asp Glu Trp Ser Met Glu Cys Gly Ser Ile Lys Asp
   865 870 875 880
   Val Met Phe Asp Ser Ser Ile Lys Leu Tyr Val Pro Gln Leu Gly Asp
   885 890 895
   Ser Tyr Asn Ile Asp Leu Asp Leu Met Asp Gly Leu Thr Ile Leu Arg
   900 905 910
   Met Pro Asn Arg Glu Gln Ile Gln Lys Ala Ala Lys Ala Leu Gly Ser
   915 920 925
   Val Leu His Lys Val Tyr Thr Asp Glu Gly Glu Glu Cys Ala Thr Asp
   930 935 940
   Gly Thr Asp His Asp Val Asn Asp Thr Thr Ser Trp Asp Gln Thr Ser
   945 950 955 960
   Arg Lys Leu Lys Asp Asp Gly Lys Gln Gly Ala Leu Ile Thr Cys His
   965 970 975
   Asn Ser Glu Lys His Cys Lys Asn Cys Arg Glu Ile Phe Leu Glu Val
   980 985 990
   Asn Glu Val Asp Asp Val Asp Gly Trp Met Tyr Asp Thr Lys Glu Leu
   995 1000 1005
   Glu Thr Thr Thr Ser Thr Pro Pro Val Tyr Tyr Lys Gly Glu Glu
   1010 1015 1020
   Arg Pro Glu Ser Tyr Leu Lys Asp Leu Thr Val Asp Cys Arg Arg
   1025 1030 1035
   Ala Gly Lys His Tyr Lys Cys Ser Ala His Gly Val Thr Trp Phe
   1040 1045 1050
   Gly Thr Ile His Thr Asp Glu Glu Lys Glu Tyr Cys Leu Thr His
   1055 1060 1065
   Lys Val Glu Gly Thr Val Thr Gln Ile Phe Gly Ala Lys Arg Arg
   1070 1075 1080
   Ser Trp Arg Ser Cys Gly Ile Ser Cys Ser Ser Leu Cys Glu Arg
   1085 1090 1095
   Val Asn Thr Thr Thr Lys Gly His Ile Trp Pro Lys Arg Phe Gly
   1100 1105 1110
   Gly Pro Glu Cys Asn Phe Asn Cys Trp Pro Ile Thr Lys Lys Gln
   1115 1120 1125
   Asn Glu Glu Asp Glu Lys Lys Glu Met Ser Ala Ala Glu Asn Ala
   1130 1135 1140
   Tyr Arg  Gly Gly Ile Ile Leu  Arg Lys Glu Ala Ser  Glu Arg Leu
       1145                 1150                 1155
   Leu Lys  Arg Ser Leu Tyr Lys  Asp Arg Ala Leu Thr  Phe Asn Ser
       1160                 1165                 1170
   Thr Trp  Cys Lys Gln Lys Lys  Cys Asn Thr Thr Val  Ile Lys Ala
       1175                 1180                 1185
   Asn Asp  Gly Asp Glu Glu Ile  Asp Glu Gly Phe Thr  Pro Val Asp
       1190                 1195                 1200
   Lys Glu  Glu Glu His Asp His  Asp Glu Asp Phe Ile  Ser Gly Lys
       1205                 1210                 1215
   Thr Ser  Leu Ala Thr Ala Met  Gln Glu Asn Gly His  Asn Gln Ala
       1220                 1225                 1230
   Thr Ile  Asp Ala Ala Thr Gly  Phe Tyr Lys Met Gln  Arg Ala Thr
       1235                 1240                 1245
   Gly Thr  Lys Ala Val Asn Gln  Leu Ile Asn Glu Leu  Val Lys Ala
       1250                 1255                 1260
   Ser Gly  Gly Glu Arg Thr Ala  Ser Asn Asp Asn Phe  Ile Asn Ser
       1265                 1270                 1275
   Pro Ala  Thr Leu Lys Ser Glu  Met Glu Leu Leu Ser  Tyr Ser Pro
       1280                 1285                 1290
   Thr Arg  Ile Glu Lys Ala Leu  Arg Ser Tyr Lys Lys  Gly Thr Glu
       1295                 1300                 1305
   Ala Lys  Gly Thr Lys Gln Ile  Arg Asn Leu Ile Arg  Glu Leu Leu
       1310                 1315                 1320
   Thr Asp  Gly Glu Gln Pro Leu  Thr Asn Gly Ala Ser  Glu Leu Thr
       1325                 1330                 1335
   Thr Thr  Ala Thr Val Val Leu  Gly Phe Pro
       1340                 1345
<210> 4
   <211> 6408
   <212> DNA
   <213> Flaviviridae Virus
<400> 4
   ttagcttcat tcgcctttga gcccgcgact gaagcctttg aatggctcct taagactttt      60
   ttgacactaa caaccctcat atttaaccat gggtttcgtg taagaccggt tggagctaag     120
   acccagatca ccatcttcgc aacagcacta ctccaccacc taacaccatt atcaatggaa     180
   ccaatcagtc aatttggttg gtatgcagta cagactgcca ttgatagtcc gactaggttt     240
   atcatcagct tgctcatcac actcgaagtc atacttatct tcatgttccc taaaccgatt     300
   tacaacatga cacgatggta ttggtcttcc tacaaaaaag agagcattat gcagatggtg     360
   gcaggaggag cgatctgtct ggccttagca caagcaccca agaacctttc ctacatcctg     420
   ttcccagtgg cggccctcta catcctcatg aggaggggcg ctactgttcg aacacggcac     480
   ctcacatccc aactgttagt acccatagac aaagacgatg atccaccgac aggagtcggt     540
   accataaccc ttaaggaagg caagttacaa tatgatatga gtagcctcca cgaccacagg     600
   gtcgtgacgg attccactag ggttttcgtg gtggtattaa ttgccatttt tggcctccta     660
   gactacaggg ctggaatagc cgccctcatc ctggcctttg gttactttaa gaatgccaaa     720
   gccgacctgt tcgacacctt caacattgcc ggacccgaga gagtggagaa agatactttc     780
   tctggccccg aaggcatata tgaggtatac aataggacac cattaggaga tgaacatctt     840
   ggttatggtt acaacaagaa caacattttt cacaccatgt accacattac aaaaggactg     900
   cccctagaat tcgaagatga agtgaccacc ccctacgatt atagtgatga actggattgg     960
   atcacctacg gtggcccctg ggctttcgcc aaacgggaag gcagggacct cttcgtctat    1020
   ctccacaggg agcatagact tagaccacta aaaatcagtt cagaagacga taaagtctca    1080
   ctgggaacaa cgaggaccat agcatttggc gactctggct caccagtact agctagagac    1140
   gccgacggaa cactgacacc cgtctcatta gctggacaca cggtaccact acaggatcca    1200
   acatatgaag ccatgatttt acccacccct actacaggca tggacctttc aaaatttcag    1260
   aggaaattcg acacaagagg cccaggatgg aatccaatag tgttaagatg tggagcagga    1320
   aaaacaagac acgttctgag aagagccgtg gttagatcta cggaaacagg acgtaactgt    1380
   ctacttttag cacccaccag gacggtagca ggagaaatgt atgaagcatt aaaagattta    1440
   gatgtaggga ttgatatcac aggcaaggtc gaaagaagaa acaagagaaa ccttatcatg    1500
   tgccactcaa cagcagtaaa ccgcctcctc caccatagcc tcaacatcca caactacaag    1560
   aacgtctttg tggacgaagc acacatgcaa gaccccatga ctatagcatt gctaggtttt    1620
   ctcgaagaaa gaaccagaga caccagcaat gacatccagg tctttccgat gacggccacc    1680
   tggtgggacc actatgaaac tgaaactaat tttccagtga ttgacaccaa acttaacgat 1740
   gaaaaaatga tcgttgatgc aataaaagac tcacttagta agaatagaag aagcattaca 1800
   ttcgtcccca ccatcaagaa agctgaagaa ctctacacca gtgtaggtga aggagaaaga 1860
   atcatcctca atagatatac ctacagaagt gaaatcagca aaatcaaaac agataaggac 1920
   aagcccatag ccatctttgc aaccaatata gccgaggtcg gcctaaacgc caatttagac 1980
   gacgtttttg acctccagca acagatagtt tttaaggaga gtgtggccgg tgtaatcact 2040
   agagaaacat ccggcgcctc caaagcatcc actacacaga gaaggggaag agttggaaga 2100
   gtcagatcag gcacttatca gtacttagac caagccgcaa cacacaacat catgcatgac 2160
   tcagatatca tccgtaagga ggctgccata gtattaaaaa tgttaggagt cgacagtgag 2220
   agcacccgat tttgccaact cccccagatc ccacccatct ccctatcaaa aaagaaaggc 2280
   ataatgaggt tgaccaacga taatgataac ataacaatct acttagccca ctacgtcact 2340
   gacgattcag gaatagaagt agattggacg accggagtac acccttgctc atgtaacgat 2400
   tgcaaaacag ccaataataa gttcatgata gtagacccca gatcacactc atacatgatg 2460
   cacaagcaga atggagagcc actcatcaaa ggcgacaccc gcatttacga actccgtgga 2520
   tcctccacta tcacaggtag catacttcgc tacattaaag cgtctgccaa cccaacatcc 2580
   agactaatga tgtcagtatt cgcggtggag gtggattgga cctgggctga cccttacgtg 2640
   actagtttca accaaggcta catagccatc attaaacaac tccccatatg gataggagtc 2700
   cacctaaaaa tagaaagggt agcagctgct atcctgtggc tcctgatttg gaagatcata 2760
   acatacattt ttgggggaag aagcaatgac agtaaagtcg tcataaacaa tggaaacatg 2820
   agttcactac ccggcttagc catattagca acaggcattg ccctacactt cgttgacaaa 2880
   ttcaactcag gtcatttacc agtagcccta gtatcggtgg ggtgcatcta catcttaggt 2940
   gtcctctact actcacagag acaggttagc tataagacca cagcgagtga ggctgctttt 3000
   tacctctttg ccgtctttgc cacgctagta ggactattca tctatatact tagaagccac 3060
   agggacgttt ttgatgattt tacatcaccc aggaaacgct actaccacac ggaagaagac 3120
   aagaaagaac atgagcccat aactgaggct ccacctctta gcattagcaa gggatttacc 3180
   tttttcctgg ggttagtgcc cctcatcaac ctcctggcgg agacaatgac acagagcaaa 3240
   ggcctacaaa acatgaatgt caacactcta cgtcattcac ttagcggtgg tgcagaagtc 3300
   aaaccccccg tacttatcac catcttcacc gtctttgcca tgttccaggg ttcctcgctc 3360
   acagactgca tcgtcacact ttgcctctct gtcctttatt accttttcta tttcggacac 3420
   tcagcacacg ttaacaccaa aagtgcagtc acagccagcc cgggaaacac caccggagat 3480
   tcactaggtt tctccaatgc accccttaaa gaagatgggg tgattaaggt gtttaccggt 3540
   ctggtactac tgacagacgc cctacttaga caggaaatga cggtggctct agcacttaat 3600
   gcttttttca cactgatacc cctcttcctc tctaaaggag aggaacccag ctacgcattc 3660
   tacatgataa tccaagccgg tttagaacaa gaacttctcc tggtcatagg ctccgtagca 3720
   acagccctct tttgcagatc cataagccgg accagaaaaa cagggggagc cgtgctatcc 3780
   atgagtttta actcagtcgt tggacgacat aaaaatctcg aaccaggttt ccaagactac 3840
   aagacgagaa tgaacggact tggcaagaac accttctaca acctgagaaa acacatggtc 3900
   cccgtacacg acaaggggac agtatgctcc agaggctacc acaaaatgaa tgaactcctc 3960
   agataccacg atttagaaat tagcggaaaa atcttagaat taggatgtgg ttctggtggc 4020
   tttacccaaa gaatggtatt agagaaaaaa gtcgatagaa ttgacgccat ctcttgggga 4080
   ccagataaga aagaccacaa tctgtttgaa ctattcaaaa aatcaacacc aggccatgga 4140
   aaagttaggt actcagtagg agaagcattc accaacaggg attttgagaa ctacgactgg 4200
   gtcgtgatgg acataggaga acaaagtcca gacatggccc gggagaggac cagagacctg 4260
   tggaggttag actggatcac caagaaagtc atcccccatg ctaaagtcat catgaagatc 4320
   ttgtccccta cagatataga cgtcctaaga gccataccac caggttacag gatcgttaga 4380
   ctatatcatt cttggaactc aaattttgaa atgtacatga tcccgggcga cccctcagat 4440
   aacatcacta ggatgaacca gctcctcaaa aaccttaaca tcaggctcca gcaaacactg 4500
   gaaggagaac atgtggagtt aggggcagaa caagtaaaac tgccttcagt cgtggagaaa 4560
   ataggcacta accttggaag agaatatgag gattggtgcc tggcagagag attagaacaa 4620
   ctccctgccc tcacacaggt cgaaaggcct aaattggacc actttcagta tctggccagt 4680
   ttcttcactg aggcccgcaa gctacctggc acatgctcta accccattat catgaccttc 4740
   tggaatggac tcaccaaact tattcctggg gctgggggat tcaccatgac agacaccaca 4800
   cacgatggta gttacaagat gttccaaaag aagatggata acccaccaag caccgatcaa 4860
   acctacatcc ctgaactcca tagagttttt gacaagatga gtgactttct ctttaaaaga 4920
   aacaaaccaa gaagactcac cagagaggaa atcatccaaa tagttaggaa cgacgcagcc 4980
   gtcggaaaca tacatccaga catcatttgg ccaacagcac tggaagccct agaatcagaa 5040
   agtttctgga aagtagttga ggaagaaaca cacctccatt cccaaggaaa atgccgctat 5100
   ggcatattca acaccatggg caagagggaa aagaaggaca cagacggtga aagaagtggc 5160
   tcacgcataa tatgctacct accacttgtg gaaagagtca ttgagatgga cgtcctaggt 5220
   ttcctcaata aagatcacat cgcaggaccg gaaagtctgc catgtggggt gtcaggagtt 5280
   agtccctatc attacccaga catattttcg aagaaagcag gactagatcc agaaacagga 5340
   gacatgacca aagcagtcat ccaagatgac acagcagcct gggacaccag agtaagaaaa 5400
   gatgtcctgc acatggaacg cgacttcatc cttaaacaca ccacagaccc ctaccacgtc 5460
   aacctcataa aaacacagta caaaatctac ggcgaaccga tccttacgat caccagaccc 5520
   ttcaccgttg acaggtcggt agttgacgtc ctccaaggct acggacagag atgttctggc 5580
   acagtagtca cctacgccat gaacaccatc acaaatgcat gtgtccagac actcaggttc 5640
   tgggaggcca tggacgtaga cttggaggaa gtcctaaatg acatgcccca catcctcaac 5700
   agtatgatga tcagtggaga cgacatgctc ttcatggtag aaccatggct agccaaaaaa 5760
   ctcagttcat ctctcaaagt catcaattca ctcggcttcc ctcgcaaagg actcacaaca 5820
   tggcaggaat cacccataat cacccagttt gataaagtct acttctgcag tcataagttc 5880
   accatcgcca gagtggccgg tgaaagacga gcattcttag acaaagacct tttcgagatc 5940
   atcgggaaaa cccaactcgt tctgggagga ctacacagcc cacacgagat agccggacac 6000
   gcaaaaagcg tagccatcta catgttcgtc accttcttcc acaacagagc cgtgagaaag 6060
   gtggcattgg ccatcctatc agcactgccc gacgaactgg tgcccatggg gaatgtcgcc 6120
   aaccccttct ttctggatca ctcctggatc agcaatgaag acctaatcac tatcttcaac 6180
   aatgtccacg gcacacatgc caggagctac actgacattg gttacacgta caaaacgttg 6240
   gccaagatca gaggagaccc attggatcat gaagaacgga ccacatggaa caaagacctc 6300
   aagagaaaaa tcgagtcgat ctccacccac tacaacagtg gtataacaaa tgggagtttc 6360
   tggcacgagc aactccgaac aaacgttagt taccacagcc tattctag 6408
<210> 5
   <211> 2135
   <212> PRT
   <213> Flaviviridae Virus
<400> 5
   Leu Ala Ser Phe Ala Phe Glu Pro Ala Thr Glu Ala Phe Glu Trp Leu
   1               5                   10                  15
   Leu Lys Thr Phe Leu Thr Leu Thr Thr Leu Ile Phe Asn His Gly Phe
               20                  25                  30
   Arg Val Arg Pro Val Gly Ala Lys Thr Gln Ile Thr Ile Phe Ala Thr
           35                  40                  45
   Ala Leu Leu His His Leu Thr Pro Leu Ser Met Glu Pro Ile Ser Gln
       50                  55                  60
   Phe Gly Trp Tyr Ala Val Gln Thr Ala Ile Asp Ser Pro Thr Arg Phe
   65 70 75 80
   Ile Ile Ser Leu Leu Ile Thr Leu Glu Val Ile Leu Ile Phe Met Phe
   85 90 95
   Pro Lys Pro Ile Tyr Asn Met Thr Arg Trp Tyr Trp Ser Ser Tyr Lys
   100 105 110
   Lys Glu Ser Ile Met Gln Met Val Ala Gly Gly Ala Ile Cys Leu Ala
   115 120 125
   Leu Ala Gln Ala Pro Lys Asn Leu Ser Tyr Ile Leu Phe Pro Val Ala
   130 135 140
   Ala Leu Tyr Ile Leu Met Arg Arg Gly Ala Thr Val Arg Thr Arg His
   145 150 155 160
   Leu Thr Ser Gln Leu Leu Val Pro Ile Asp Lys Asp Asp Asp Pro Pro
   165 170 175
   Thr Gly Val Gly Thr Ile Thr Leu Lys Glu Gly Lys Leu Gln Tyr Asp
   180 185 190
   Met Ser Ser Leu His Asp His Arg Val Val Thr Asp Ser Thr Arg Val
   195 200 205
   Phe Val Val Val Leu Ile Ala Ile Phe Gly Leu Leu Asp Tyr Arg Ala
   210 215 220
   Gly Ile Ala Ala Leu Ile Leu Ala Phe Gly Tyr Phe Lys Asn Ala Lys
   225 230 235 240
   Ala Asp Leu Phe Asp Thr Phe Asn Ile Ala Gly Pro Glu Arg Val Glu
   245 250 255
   Lys Asp Thr Phe Ser Gly Pro Glu Gly Ile Tyr Glu Val Tyr Asn Arg
   260 265 270
   Thr Pro Leu Gly Asp Glu His Leu Gly Tyr Gly Tyr Asn Lys Asn Asn
   275 280 285
   Ile Phe His Thr Met Tyr His Ile Thr Lys Gly Leu Pro Leu Glu Phe
   290 295 300
   Glu Asp Glu Val Thr Thr Pro Tyr Asp Tyr Ser Asp Glu Leu Asp Trp
   305 310 315 320
   Ile Thr Tyr Gly Gly Pro Trp Ala Phe Ala Lys Arg Glu Gly Arg Asp
   325 330 335
   Leu Phe Val Tyr Leu His Arg Glu His Arg Leu Arg Pro Leu Lys Ile
   340 345 350
   Ser Ser Glu Asp Asp Lys Val Ser Leu Gly Thr Thr Arg Thr Ile Ala
   355 360 365
   Phe Gly Asp Ser Gly Ser Pro Val Leu Ala Arg Asp Ala Asp Gly Thr
   370 375 380
   Leu Thr Pro Val Ser Leu Ala Gly His Thr Val Pro Leu Gln Asp Pro
   385 390 395 400
   Thr Tyr Glu Ala Met Ile Leu Pro Thr Pro Thr Thr Gly Met Asp Leu
   405 410 415
   Ser Lys Phe Gln Arg Lys Phe Asp Thr Arg Gly Pro Gly Trp Asn Pro
   420 425 430
   Ile Val Leu Arg Cys Gly Ala Gly Lys Thr Arg His Val Leu Arg Arg
   435 440 445
   Ala Val Val Arg Ser Thr Glu Thr Gly Arg Asn Cys Leu Leu Leu Ala
   450 455 460
   Pro Thr Arg Thr Val Ala Gly Glu Met Tyr Glu Ala Leu Lys Asp Leu
   465 470 475 480
   Asp Val Gly Ile Asp Ile Thr Gly Lys Val Glu Arg Arg Asn Lys Arg
   485 490 495
   Asn Leu Ile Met Cys His Ser Thr Ala Val Asn Arg Leu Leu His His
   500 505 510
   Ser Leu Asn Ile His Asn Tyr Lys Asn Val Phe Val Asp Glu Ala His
   515 520 525
   Met Gln Asp Pro Met Thr Ile Ala Leu Leu Gly Phe Leu Glu Glu Arg
   530 535 540
   Thr Arg Asp Thr Ser Asn Asp Ile Gln Val Phe Pro Met Thr Ala Thr
   545 550 555 560
   Trp Trp Asp His Tyr Glu Thr Glu Thr Asn Phe Pro Val Ile Asp Thr
   565 570 575
   Lys Leu Asn Asp Glu Lys Met Ile Val Asp Ala Ile Lys Asp Ser Leu
   580 585 590
   Ser Lys Asn Arg Arg Ser Ile Thr Phe Val Pro Thr Ile Lys Lys Ala
   595 600 605
   Glu Glu Leu Tyr Thr Ser Val Gly Glu Gly Glu Arg Ile Ile Leu Asn
   610 615 620
   Arg Tyr Thr Tyr Arg Ser Glu Ile Ser Lys Ile Lys Thr Asp Lys Asp
   625 630 635 640
   Lys Pro Ile Ala Ile Phe Ala Thr Asn Ile Ala Glu Val Gly Leu Asn
   645 650 655
   Ala Asn Leu Asp Asp Val Phe Asp Leu Gln Gln Gln Ile Val Phe Lys
   660 665 670
   Glu Ser Val Ala Gly Val Ile Thr Arg Glu Thr Ser Gly Ala Ser Lys
   675 680 685
   Ala Ser Thr Thr Gln Arg Arg Gly Arg Val Gly Arg Val Arg Ser Gly
   690 695 700
   Thr Tyr Gln Tyr Leu Asp Gln Ala Ala Thr His Asn Ile Met His Asp
   705 710 715 720
   Ser Asp Ile Ile Arg Lys Glu Ala Ala Ile Val Leu Lys Met Leu Gly
   725 730 735
   Val Asp Ser Glu Ser Thr Arg Phe Cys Gln Leu Pro Gln Ile Pro Pro
   740 745 750
   Ile Ser Leu Ser Lys Lys Lys Gly Ile Met Arg Leu Thr Asn Asp Asn
   755 760 765
   Asp Asn Ile Thr Ile Tyr Leu Ala His Tyr Val Thr Asp Asp Ser Gly
   770 775 780
   Ile Glu Val Asp Trp Thr Thr Gly Val His Pro Cys Ser Cys Asn Asp
   785                 790                 795                 800
   Cys Lys Thr Ala Asn Asn Lys Phe Met Ile Val Asp Pro Arg Ser His
                   805                 810                 815
   Ser Tyr Met Met His Lys Gln Asn Gly Glu Pro Leu Ile Lys Gly Asp
               820                 825                 830
   Thr Arg Ile Tyr Glu Leu Arg Gly Ser Ser Thr Ile Thr Gly Ser Ile
           835                 840                 845
   Leu Arg Tyr Ile Lys Ala Ser Ala Asn Pro Thr Ser Arg Leu Met Met
       850                 855                 860
   Ser Val Phe Ala Val Glu Val Asp Trp Thr Trp Ala Asp Pro Tyr Val
   865                 870                 875                 880
   Thr Ser Phe Asn Gln Gly Tyr Ile Ala Ile Ile Lys Gln Leu Pro Ile
                   885                 890                 895
   Trp Ile Gly Val His Leu Lys Ile Glu Arg Val Ala Ala Ala Ile Leu
               900                 905                 910
   Trp Leu Leu Ile Trp Lys Ile Ile Thr Tyr Ile Phe Gly Gly Arg Ser
           915                 920                 925
   Asn Asp Ser Lys Val Val Ile Asn Asn Gly Asn Met Ser Ser Leu Pro
       930                 935                 940
   Gly Leu Ala Ile Leu Ala Thr Gly Ile Ala Leu His Phe Val Asp Lys
   945                 950                 955                 960
   Phe Asn Ser Gly His Leu Pro Val Ala Leu Val Ser Val Gly Cys Ile
                   965                 970                 975
   Tyr Ile Leu Gly Val Leu Tyr Tyr Ser Gln Arg Gln Val Ser Tyr Lys
               980                 985                 990
   Thr Thr Ala Ser Glu Ala Ala Phe  Tyr Leu Phe Ala Val  Phe Ala Thr
           995                 1000                 1005
   Leu Val  Gly Leu Phe Ile Tyr  Ile Leu Arg Ser His  Arg Asp Val
       1010                 1015                 1020
   Phe Asp Asp Phe Thr Ser Pro Arg Lys Arg Tyr Tyr His Thr Glu
       1025                1030                1035
   Glu Asp Lys Lys Glu His Glu Pro Ile Thr Glu Ala Pro Pro Leu
       1040                1045                1050
   Ser Ile Ser Lys Gly Phe Thr Phe Phe Leu Gly Leu Val Pro Leu
       1055                1060                1065
   Ile Asn Leu Leu Ala Glu Thr Met Thr Gln Ser Lys Gly Leu Gln
       1070                1075                1080
   Asn Met Asn Val Asn Thr Leu Arg His Ser Leu Ser Gly Gly Ala
       1085                1090                1095
   Glu Val Lys Pro Pro Val Leu Ile Thr Ile Phe Thr Val Phe Ala
       1100                1105                1110
   Met Phe Gln Gly Ser Ser Leu Thr Asp Cys Ile Val Thr Leu Cys
       1115                1120                1125
   Leu Ser Val Leu Tyr Tyr Leu Phe Tyr Phe Gly His Ser Ala His
       1130                1135                1140
   Val Asn Thr Lys Ser Ala Val Thr Ala Ser Pro Gly Asn Thr Thr
       1145                1150                1155
   Gly Asp Ser Leu Gly Phe Ser Asn Ala Pro Leu Lys Glu Asp Gly
       1160                1165                1170
   Val Ile Lys Val Phe Thr Gly Leu Val Leu Leu Thr Asp Ala Leu
       1175                1180                1185
   Leu Arg Gln Glu Met Thr Val Ala Leu Ala Leu Asn Ala Phe Phe
       1190                1195                1200
   Thr Leu Ile Pro Leu Phe Leu Ser Lys Gly Glu Glu Pro Ser Tyr
       1205                1210                1215
   Ala Phe Tyr Met Ile Ile Gln Ala Gly Leu Glu Gln Glu Leu Leu
       1220                1225                1230
   Leu Val Ile Gly Ser Val Ala Thr Ala Leu Phe Cys Arg Ser Ile
       1235                1240                1245
   Ser Arg Thr Arg Lys Thr Gly Gly Ala Val Leu Ser Met Ser Phe
       1250                1255                1260
   Asn Ser Val Val Gly Arg His Lys Asn Leu Glu Pro Gly Phe Gln
       1265                1270                1275
   Asp Tyr Lys Thr Arg Met Asn Gly Leu Gly Lys Asn Thr Phe Tyr
       1280                1285                1290
   Asn Leu Arg Lys His Met Val Pro Val His Asp Lys Gly Thr Val
       1295                1300                1305
   Cys Ser Arg Gly Tyr His Lys Met Asn Glu Leu Leu Arg Tyr His
       1310                1315                1320
   Asp Leu Glu Ile Ser Gly Lys Ile Leu Glu Leu Gly Cys Gly Ser
       1325                1330                1335
   Gly Gly Phe Thr Gln Arg Met Val Leu Glu Lys Lys Val Asp Arg
       1340                1345                1350
   Ile Asp Ala Ile Ser Trp Gly Pro Asp Lys Lys Asp His Asn Leu
       1355                1360                1365
   Phe Glu Leu Phe Lys Lys Ser Thr Pro Gly His Gly Lys Val Arg
       1370                1375                1380
   Tyr Ser Val Gly Glu Ala Phe Thr Asn Arg Asp Phe Glu Asn Tyr
       1385                1390                1395
   Asp Trp Val Val Met Asp Ile Gly Glu Gln Ser Pro Asp Met Ala
       1400                1405                1410
   Arg Glu Arg Thr Arg Asp Leu Trp Arg Leu Asp Trp Ile Thr Lys
       1415                1420                1425
   Lys Val Ile Pro His Ala Lys Val Ile Met Lys Ile Leu Ser Pro
       1430                1435                1440
   Thr Asp Ile Asp Val Leu Arg Ala Ile Pro Pro Gly Tyr Arg Ile
       1445                1450                1455
   Val Arg Leu Tyr His Ser Trp Asn Ser Asn Phe Glu Met Tyr Met
       1460                1465                1470
   Ile Pro Gly Asp Pro Ser Asp Asn Ile Thr Arg Met Asn Gln Leu
   1475 1480 1485
   Leu Lys Asn Leu Asn Ile Arg Leu Gln Gln Thr Leu Glu Gly Glu
   1490 1495 1500
   His Val Glu Leu Gly Ala Glu Gln Val Lys Leu Pro Ser Val Val
   1505 1510 1515
   Glu Lys Ile Gly Thr Asn Leu Gly Arg Glu Tyr Glu Asp Trp Cys
   1520 1525 1530
   Leu Ala Glu Arg Leu Glu Gln Leu Pro Ala Leu Thr Gln Val Glu
   1535 1540 1545
   Arg Pro Lys Leu Asp His Phe Gln Tyr Leu Ala Ser Phe Phe Thr
   1550 1555 1560
   Glu Ala Arg Lys Leu Pro Gly Thr Cys Ser Asn Pro Ile Ile Met
   1565 1570 1575
   Thr Phe Trp Asn Gly Leu Thr Lys Leu Ile Pro Gly Ala Gly Gly
   1580 1585 1590
   Phe Thr Met Thr Asp Thr Thr His Asp Gly Ser Tyr Lys Met Phe
   1595 1600 1605
   Gln Lys Lys Met Asp Asn Pro Pro Ser Thr Asp Gln Thr Tyr Ile
   1610 1615 1620
   Pro Glu Leu His Arg Val Phe Asp Lys Met Ser Asp Phe Leu Phe
   1625 1630 1635
   Lys Arg Asn Lys Pro Arg Arg Leu Thr Arg Glu Glu Ile Ile Gln
   1640 1645 1650
   Ile Val Arg Asn Asp Ala Ala Val Gly Asn Ile His Pro Asp Ile
   1655 1660 1665
   Ile Trp Pro Thr Ala Leu Glu Ala Leu Glu Ser Glu Ser Phe Trp
   1670 1675 1680
   Lys Val Val Glu Glu Glu Thr His Leu His Ser Gln Gly Lys Cys
   1685 1690 1695
   Arg Tyr Gly Ile Phe Asn Thr Met Gly Lys Arg Glu Lys Lys Asp
   1700 1705 1710
   Thr Asp Gly Glu Arg Ser Gly Ser Arg Ile Ile Cys Tyr Leu Pro
   1715 1720 1725
   Leu Val Glu Arg Val Ile Glu Met Asp Val Leu Gly Phe Leu Asn
   1730 1735 1740
   Lys Asp His Ile Ala Gly Pro Glu Ser Leu Pro Cys Gly Val Ser
   1745 1750 1755
   Gly Val Ser Pro Tyr His Tyr Pro Asp Ile Phe Ser Lys Lys Ala
   1760 1765 1770
   Gly Leu Asp Pro Glu Thr Gly Asp Met Thr Lys Ala Val Ile Gln
   1775 1780 1785
   Asp Asp Thr Ala Ala Trp Asp Thr Arg Val Arg Lys Asp Val Leu
   1790 1795 1800
   His Met Glu Arg Asp Phe Ile Leu Lys His Thr Thr Asp Pro Tyr
   1805 1810 1815
   His Val Asn Leu Ile Lys Thr Gln Tyr Lys Ile Tyr Gly Glu Pro
   1820 1825 1830
   Ile Leu Thr Ile Thr Arg Pro Phe Thr Val Asp Arg Ser Val Val
   1835 1840 1845
   Asp Val Leu Gln Gly Tyr Gly Gln Arg Cys Ser Gly Thr Val Val
   1850 1855 1860
   Thr Tyr Ala Met Asn Thr Ile Thr Asn Ala Cys Val Gln Thr Leu
   1865 1870 1875
   Arg Phe Trp Glu Ala Met Asp Val Asp Leu Glu Glu Val Leu Asn
   1880 1885 1890
   Asp Met Pro His Ile Leu Asn Ser Met Met Ile Ser Gly Asp Asp
   1895 1900 1905
   Met Leu Phe Met Val Glu Pro Trp Leu Ala Lys Lys Leu Ser Ser
   1910 1915 1920
   Ser Leu  Lys Val Ile Asn Ser  Leu Gly Phe Pro Arg  Lys Gly Leu
       1925                 1930                 1935
   Thr Thr  Trp Gln Glu Ser Pro  Ile Ile Thr Gln Phe  Asp Lys Val
       1940                 1945                 1950
   Tyr Phe  Cys Ser His Lys Phe  Thr Ile Ala Arg Val  Ala Gly Glu
       1955                 1960                 1965
   Arg Arg  Ala Phe Leu Asp Lys  Asp Leu Phe Glu Ile  Ile Gly Lys
       1970                 1975                 1980
   Thr Gln  Leu Val Leu Gly Gly  Leu His Ser Pro His  Glu Ile Ala
       1985                 1990                 1995
   Gly His  Ala Lys Ser Val Ala  Ile Tyr Met Phe Val  Thr Phe Phe
       2000                 2005                 2010
   His Asn  Arg Ala Val Arg Lys  Val Ala Leu Ala Ile  Leu Ser Ala
       2015                 2020                 2025
   Leu Pro  Asp Glu Leu Val Pro  Met Gly Asn Val Ala  Asn Pro Phe
       2030                 2035                 2040
   Phe Leu  Asp His Ser Trp Ile  Ser Asn Glu Asp Leu  Ile Thr Ile
       2045                 2050                 2055
   Phe Asn  Asn Val His Gly Thr  His Ala Arg Ser Tyr  Thr Asp Ile
       2060                 2065                 2070
   Gly Tyr  Thr Tyr Lys Thr Leu  Ala Lys Ile Arg Gly  Asp Pro Leu
       2075                 2080                 2085
   Asp His  Glu Glu Arg Thr Thr  Trp Asn Lys Asp Leu  Lys Arg Lys
       2090                 2095                 2100
   Ile Glu  Ser Ile Ser Thr His  Tyr Asn Ser Gly Ile  Thr Asn Gly
       2105                 2110                 2115
   Ser Phe  Trp His Glu Gln Leu  Arg Thr Asn Val Ser  Tyr His Ser
       2120                 2125                 2130
   Leu Phe
       2135
<210> 6
   <211> 1557
   <212> DNA
   <213> Flaviviridae Virus
<400> 6
   acctcttcaa ccactgaaat gagcggaaaa acactctgct ctgacccgga agaaatgtca      60
   accatgactg gagtagcaca cacctcagtc agcctccgca tggactacaa gcattgctat     120
   cagatccaac cggtaggcat ggacacaatc acggccagaa tgagctaccc ctatatagac     180
   aatgcactca caggatacaa aatatcctgg ttggacatta gttccaactc agtttcatcc     240
   gacagatgcc caggagatgg agaacccgcc tgcccaaaat caggaccata ttccatctgc     300
   gctagaggat accatgatcg tggctggacc actggctgct tcctcttcgg acaaggaccg     360
   gtatgtactt gtgctgaact agccctcaaa catccgatac aggtctatac aatcgaccca     420
   acaaagatgg gagcccgctt aacggtctcc tttggacgca cagaaaatca gaactattac     480
   aacatggaca caaacacagg aagtttcgac atcacagaca aagccgactc gtcctcacga     540
   gtcagagtaa catgccagct accacacgca gaacttgcca ccaagtacct gataaagatg     600
   gcaccaaatg atggcgaatc atatctcgta gatagattgg cctttgaatc aatgaaactc     660
   ccctggtccg tatggaccac gtctatccca accagcctta gcaagattga agatggcaac     720
   gtccatgatg ccgagaccta tataacttgg agagacacca ccctccatta cgtgagattt     780
   tcagaagccc ataacgtcga atccatggtc aagaatttcc tggacaagaa cgggaagata     840
   atcatagccg agatggccaa aggatcagga agagcctccc caggcaccaa gatgccctta     900
   gacattagcc ccacagactg cagtttcatg cttaattccc ttaaaataca caccaccagt     960
   gtgaaggact gtgcagacac aaccaactac gcattcaaac tgatcgaagg cagcagaata    1020
   ctcaccactc acgatggagt tgtcaccttt aacttagata tggcagttcc tagccagtgc    1080
   aggttagtcg tcaagggacg tagccgcagc caagttggct ctggatacac ctccaactac    1140
   tgcacccttt tcgatcaggc acacatagtc acttcaacca aagaagtact gaaaatgcac    1200
   tgcccacccg gagagcatga aattaaacta ggcaacaaca agcatcaagt gaatgtcccc    1260
   caaatgggta tcacaggatg gcttagcaga tggaccacta acgttctgga agtcaaagac    1320
   agaataacca tagatggagc agttaagaga ccagtactag acatgttcaa gaacttcttt    1380
   gccaaaggag caggcttcct aggaagcctt ttcggaatga catcaacaat catgatggca    1440
   gtgacgggag gagccatagt atggctcgga accctaatga caggtaacat gaagaatctc    1500
   acagtcatca taggacttat cctcatggcc cccttagtaa taacggaggt agaagca       1557
<210> 7
   <211> 519
   <212> PRT
   <213> Flaviviridae Virus
<400> 7
   Thr Ser Ser Thr Thr Glu Met Ser Gly Lys Thr Leu Cys Ser Asp Pro
   1               5                   10                  15
   Glu Glu Met Ser Thr Met Thr Gly Val Ala His Thr Ser Val Ser Leu
               20                  25                  30
   Arg Met Asp Tyr Lys His Cys Tyr Gln Ile Gln Pro Val Gly Met Asp
           35                  40                  45
   Thr Ile Thr Ala Arg Met Ser Tyr Pro Tyr Ile Asp Asn Ala Leu Thr
       50                  55                  60
   Gly Tyr Lys Ile Ser Trp Leu Asp Ile Ser Ser Asn Ser Val Ser Ser
   65                  70                  75                  80
   Asp Arg Cys Pro Gly Asp Gly Glu Pro Ala Cys Pro Lys Ser Gly Pro
                   85                  90                  95
   Tyr Ser Ile Cys Ala Arg Gly Tyr His Asp Arg Gly Trp Thr Thr Gly
               100                 105                 110
   Cys Phe Leu Phe Gly Gln Gly Pro Val Cys Thr Cys Ala Glu Leu Ala
           115                 120                 125
   Leu Lys His Pro Ile Gln Val Tyr Thr Ile Asp Pro Thr Lys Met Gly
       130                 135                 140
   Ala Arg Leu Thr Val Ser Phe Gly Arg Thr Glu Asn Gln Asn Tyr Tyr
   145                 150                 155                 160
   Asn Met Asp Thr Asn Thr Gly Ser Phe Asp Ile Thr Asp Lys Ala Asp
                   165                 170                 175
   Ser Ser Ser Arg Val Arg Val Thr Cys Gln Leu Pro His Ala Glu Leu
               180                 185                 190
   Ala Thr Lys Tyr Leu Ile Lys Met Ala Pro Asn Asp Gly Glu Ser Tyr
           195                 200                 205
   Leu Val Asp Arg Leu Ala Phe Glu Ser Met Lys Leu Pro Trp Ser Val
   210 215 220
   Trp Thr Thr Ser Ile Pro Thr Ser Leu Ser Lys Ile Glu Asp Gly Asn
   225 230 235 240
   Val His Asp Ala Glu Thr Tyr Ile Thr Trp Arg Asp Thr Thr Leu His
   245 250 255
   Tyr Val Arg Phe Ser Glu Ala His Asn Val Glu Ser Met Val Lys Asn
   260 265 270
   Phe Leu Asp Lys Asn Gly Lys Ile Ile Ile Ala Glu Met Ala Lys Gly
   275 280 285
   Ser Gly Arg Ala Ser Pro Gly Thr Lys Met Pro Leu Asp Ile Ser Pro
   290 295 300
   Thr Asp Cys Ser Phe Met Leu Asn Ser Leu Lys Ile His Thr Thr Ser
   305 310 315 320
   Val Lys Asp Cys Ala Asp Thr Thr Asn Tyr Ala Phe Lys Leu Ile Glu
   325 330 335
   Gly Ser Arg Ile Leu Thr Thr His Asp Gly Val Val Thr Phe Asn Leu
   340 345 350
   Asp Met Ala Val Pro Ser Gln Cys Arg Leu Val Val Lys Gly Arg Ser
   355 360 365
   Arg Ser Gln Val Gly Ser Gly Tyr Thr Ser Asn Tyr Cys Thr Leu Phe
   370 375 380
   Asp Gln Ala His Ile Val Thr Ser Thr Lys Glu Val Leu Lys Met His
   385 390 395 400
   Cys Pro Pro Gly Glu His Glu Ile Lys Leu Gly Asn Asn Lys His Gln
   405 410 415
   Val Asn Val Pro Gln Met Gly Ile Thr Gly Trp Leu Ser Arg Trp Thr
   420 425 430
   Thr Asn Val Leu Glu Val Lys Asp Arg Ile Thr Ile Asp Gly Ala Val
   435 440 445
   Lys Arg Pro Val Leu Asp Met Phe Lys Asn Phe Phe Ala Lys Gly Ala
       450                 455                 460
   Gly Phe Leu Gly Ser Leu Phe Gly Met Thr Ser Thr Ile Met Met Ala
   465                 470                 475                 480
   Val Thr Gly Gly Ala Ile Val Trp Leu Gly Thr Leu Met Thr Gly Asn
                   485                 490                 495
   Met Lys Asn Leu Thr Val Ile Ile Gly Leu Ile Leu Met Ala Pro Leu
               500                 505                 510
   Val Ile Thr Glu Val Glu Ala
           515
<210> 8
   <211> 20
   <212> DNA
   <213> Flaviviridae Virus
<400> 8
   cctccattcc caaggaaaat 20
<210> 9
   <211> 20
   <212> DNA
   <213> Flaviviridae Virus
<400> 9
   ggtcctgcga tgtgatcttt 20
<210> 10
   <211> 20
   <212> DNA
   <213> Flaviviridae Virus
<400> 10
   gagaacagcc agcaatgaca 20
<210> 11
   <211> 20
   <212> DNA
   <213> Flaviviridae Virus
<400> 11
   ttcgagtgtg atgagcaagc 20
<210> 12
   <211> 23
   <212> DNA
   <213> Flaviviridae Virus
<400> 12
   acatacttag aagaataacc cgg 23
<210> 13
   <211> 19
   <212> DNA
   <213> Flaviviridae Virus
<400> 13
   tgccgttggt agcactcct 19
<210> 14
   <211> 22
   <212> DNA
   <213> Flaviviridae Virus
<400> 14
   tacgcaacaa ccaaacctat gg 22
<210> 15
   <211> 20
   <212> DNA
   <213> Flaviviridae Virus
<400> 15
   gcagatggaa tatggtcctg 20
<210> 16
   <211> 21
   <212> DNA
   <213> Flaviviridae Virus
<400> 16
   gacaatgcac tcacaggata c 21
<210> 17
   <211> 20
   <212> DNA
   <213> Flaviviridae Virus
<400> 17
   gcatgaaact gcagtctgtg 20
<210> 18
   <211> 20
   <212> DNA
   <213> Flaviviridae Virus
<400> 18
   tcagaagccc ataacgtcga 20
<210> 19
   <211> 21
   <212> DNA
   <213> Flaviviridae Virus
<400> 19
   gatgaatcga acatgacgtc c 21
<210> 20
   <211> 11336
   <212> RNA
   <213> Flaviviridae
<400> 20
   ggggggcuug uuucuacuuc uguauauuaa guaguuccaa aauuaguguu uccguuaaac 60
   cuacagcuug guaggaauac accaauucua guuccaugaa cauaagaaug uaacaacucg     120
   cccgauacug uaaugguguu uguguaauac uggaguggua aggugacuau auacauguca     180
   uuuacauuua guacaacauu uuauaaaaca auaauauuua cauaauauaa gcguaauuuu 240
   aauaaacauu uucauuaagc uaauaauuuu uccacguugc uuuuauuuaa accugaucac 300
   gcgggggauu caggguucuu uucuuucuac ucuauuuauu gaaaaaccgg agauagguua 360
   cggcuuuuug uuaucuuccg accacuaguu guaaguguuc guacuugguc gauguuauuu 420
   aacgaccuca cgucuuagag uagcaguccu cuugguccua acuaaacagu aaaugaauac 480
   auucucuaaa ucuaaguuaa uacauuuucu aaaucuaagu uauuucauag caaguauuuu 540
   cuuauuuuuu auuuuuuaua guaagguuuu uuguuuguuu ucuuaucuaa acuaaguggu 600
   gcuacagucc uagaauaggc ugugguaacu aacguuuguu cggaguugcu cgugccagaa 660
   acucccauuu guuauaccac uguuguagug gguggagauc gacucgauuu uucucuugag 720
   gucuuuguuc cauguggucc guucuucaug auccaauggg ucuccucuga ucuuggccaa 780
   cguuuuguac guguaaccaa ugucagugua gcuccuggca ugugugccgu ggacauuguu 840
   gaagauagug auuaggucuu cauugcugau ccaggaguga uccagaaaga agggguuggc 900
   gacauucccc augggcacca guucgucggg cagugcugau aggauggcca augccaccuu 960
   ucucacggcu cuguugugga agaaggugac gaacauguag auggcuacgc uuuuugcgug 1020
   uccggcuauc ucgugugggc uguguagucc ucccagaacg aguuggguuu ucccgaugau 1080
   cucgaaaagg ucuuugucua agaaugcucg ucuuucaccg gccacucugg cgauggugaa 1140
   cuuaugacug cagaaguaga cuuuaucaaa cugggugauu augggugauu ccugccaugu 1200
   ugugaguccu uugcgaggga agccgaguga auugaugacu uugagagaug aacugaguuu 1260
   uuuggcuagc caugguucua ccaugaagag caugucgucu ccacugauca ucauacuguu 1320
   gaggaugugg ggcaugucau uuaggacuuc cuccaagucu acguccaugg ccucccagaa 1380
   ccugaguguc uggacacaug cauuugugau gguguucaug gcguagguga cuacugugcc 1440
   agaacaucuc uguccguagc cuuggaggac gucaacuacc gaccugucaa cggugaaggg 1500
   ucuggugauc guaaggaucg guucgccgua gauuuuguac uguguuuuua ugagguugac 1560
   gugguagggg ucuguggugu guuuaaggau gaagucgcgu uccaugugca ggacaucuuu 1620
   ucuuacucug gugucccagg cugcuguguc aucuuggaug acugcuuugg ucaugucucc 1680
   uguuucugga ucuaguccug cuuucuucga aaauaugucu ggguaaugau agggacuaac 1740
   uccugacacc ccacauggca gacuuuccgg uccugcgaug ugaucuuuau ugaggaaacc 1800
   uaggacgucc aucucaauga cucuuuccac aagugguagg uagcauauua ugcgugagcc 1860
   acuucuuuca ccgucugugu ccuucuuuuc ccucuugccc augguguuga auaugccaua 1920
   gcggcauuuu ccuugggaau ggaggugugu uucuuccuca acuacuuucc agaaacuuuc 1980
   ugauucuagg gcuuccagug cuguuggcca aaugaugucu ggauguaugu uuccgacggc 2040
   ugcgucguuc cuaacuauuu ggaugauuuc cucucuggug agucuucuug guuuguuucu 2100
   uuuaaagaga aagucacuca ucuugucaaa aacucuaugg aguucaggga uguagguuug 2160
   aucggugcuu gguggguuau ccaucuucuu uuggaacauc uuguaacuac caucgugugu 2220
   ggugucuguc auggugaauc ccccagcccc aggaauaagu uuggugaguc cauuccagaa 2280
   ggucaugaua augggguuag agcaugugcc agguagcuug cgggccucag ugaagaaacu 2340
   ggccagauac ugaaaguggu ccaauuuagg ccuuucgacc ugugugaggg cagggaguug 2400
   uucuaaucuc ucugccaggc accaauccuc auauucucuu ccaagguuag ugccuauuuu 2460
   cuccacgacu gaaggcaguu uuacuuguuc ugccccuaac uccacauguu cuccuuccag 2520
   uguuugcugg agccugaugu uaagguuuuu gaggagcugg uucauccuag ugauguuauc 2580
   ugaggggucg cccgggauca uguacauuuc aaaauuugag uuccaagaau gauauagucu 2640
   aacgauccug uaaccuggug guauggcucu uaggacgucu auaucuguag gggacaagau 2700
   cuucaugaug acuuuagcau gggggaugac uuucuuggug auccagucua accuccacag 2760
   gucucugguc cucucccggg ccaugucugg acuuuguucu ccuaugucca ucacgaccca 2820
   gucguaguuc ucaaaauccc uguuggugaa ugcuucuccu acugaguacc uaacuuuucc 2880
   auggccuggu guugauuuuu ugaauaguuc aaacagauug uggucuuucu uaucuggucc 2940
   ccaagagaug gcgucaauuc uaucgacuuu uuucucuaau accauucuuu ggguaaagcc 3000
   accagaacca cauccuaauu cuaagauuuu uccgcuaauu ucuaaaucgu gguaucugag 3060
   gaguucauuc auuuuguggu agccucugga gcauacuguc cccuugucgu guacggggac 3120
   cauguguuuu cucagguugu agaagguguu cuugccaagu ccguucauuc ucgucuugua 3180
   gucuuggaaa ccugguucga gauuuuuaug ucguccaacg acugaguuaa aacucaugga 3240
   uagcacggcu cccccuguuu uucugguccg gcuuauggau cugcaaaaga gggcuguugc 3300
   uacggagccu augaccagga gaaguucuug uucuaaaccg gcuuggauua ucauguagaa    3360
   ugcguagcug gguuccucuc cuuuagagag gaagaggggu aucaguguga aaaaagcauu    3420
   aagugcuaga gccaccguca uuuccugucu aaguagggcg ucugucagua guaccagacc 3480
   gguaaacacc uuaaucaccc caucuucuuu aaggggugca uuggagaaac cuagugaauc 3540
   uccgguggug uuucccgggc uggcugugac ugcacuuuug guguuaacgu gugcugagug 3600
   uccgaaauag aaaagguaau aaaggacaga gaggcaaagu gugacgaugc agucugugag 3660
   cgaggaaccc uggaacaugg caaagacggu gaagauggug auaaguacgg gggguuugac 3720
   uucugcacca ccgcuaagug aaugacguag aguguugaca uucauguuuu guaggccuuu 3780
   gcucuguguc auugucuccg ccaggagguu gaugaggggc acuaacccca ggaaaaaggu 3840
   aaaucccuug cuaaugcuaa gagguggagc cucaguuaug ggcucauguu cuuucuuguc 3900
   uucuuccgug ugguaguagc guuuccuggg ugauguaaaa ucaucaaaaa cgucccugug 3960
   gcuucuaagu auauagauga auaguccuac uagcguggca aagacggcaa agagguaaaa 4020
   agcagccuca cucgcugugg ucuuauagcu aaccugucuc ugugaguagu agaggacacc 4080
   uaagauguag augcacccca ccgauacuag ggcuacuggu aaaugaccug aguugaauuu 4140
   gucaacgaag uguagggcaa ugccuguugc uaauauggcu aagccgggua gugaacucau 4200
   guuuccauug uuuaugacga cuuuacuguc auugcuucuu cccccaaaaa uguauguuau 4260
   gaucuuccaa aucaggagcc acaggauagc agcugcuacc cuuucuauuu uuagguggac 4320
   uccuauccau auggggaguu guuuaaugau ggcuauguag ccuugguuga aacuagucac 4380
   guaaggguca gcccaggucc aauccaccuc caccgcgaau acugacauca uuagucugga 4440
   uguuggguug gcagacgcuu uaauguagcg aaguaugcua ccugugauag uggaggaucc 4500
   acggaguucg uaaaugcggg ugucgccuuu gaugaguggc ucuccauucu gcuugugcau 4560
   cauguaugag ugugaucugg ggucuacuau caugaacuua uuauuggcug uuuugcaauc 4620
   guuacaugag caagggugua cuccggucgu ccaaucuacu ucuauuccug aaucgucagu 4680
   gacguagugg gcuaaguaga uuguuauguu aucauuaucg uuggucaacc ucauuaugcc 4740
   uuucuuuuuu gauagggaga ugggugggau cugggggagu uggcaaaauc gggugcucuc 4800
   acugucgacu ccuaacauuu uuaauacuau ggcagccucc uuacggauga uaucugaguc 4860
   augcaugaug uuguguguug cggcuugguc uaaguacuga uaagugccug aucugacucu 4920
   uccaacucuu ccccuucucu guguagugga ugcuuuggag gcgccggaug uuucucuagu 4980
   gauuacaccg gccacacucu ccuuaaaaac uaucuguugc uggaggucaa aaacgucguc 5040
   uaaauuggcg uuuaggccga ccucggcuau auugguugca aagauggcua ugggcuuguc 5100
   cuuaucuguu uugauuuugc ugauuucacu ucuguaggua uaucuauuga ggaugauucu 5160
   uucuccuuca ccuacacugg uguagaguuc uucagcuuuc uugauggugg ggacgaaugu 5220
   aaugcuucuu cuauucuuac uaagugaguc uuuuauugca ucaacgauca uuuuuucauc 5280
   guuaaguuug gugucaauca cuggaaaauu aguuucaguu ucauaguggu cccaccaggu 5340
   ggccgucauc ggaaagaccu ggaugucauu gcuggugucu cugguucuuu cuucgagaaa 5400
   accuagcaau gcuauaguca uggggucuug caugugugcu ucguccacaa agacguucuu 5460
   guaguugugg auguugaggc uaugguggag gaggcgguuu acugcuguug aguggcacau 5520
   gauaagguuu cucuuguuuc uucuuucgac cuugccugug auaucaaucc cuacaucuaa 5580
   aucuuuuaau gcuucauaca uuucuccugc uaccguccug gugggugcua aaaguagaca 5640
   guuacguccu guuuccguag aucuaaccac ggcucuucuc agaacguguc uuguuuuucc 5700
   ugcuccacau cuuaacacua uuggauucca uccugggccu cuugugucga auuuccucug 5760
   aaauuuugaa agguccaugc cuguaguagg gguggguaaa aucauggcuu cauauguugg 5820
   auccuguagu gguaccgugu guccagcuaa ugagacgggu gucaguguuc cgucggcguc 5880
   ucuagcuagu acuggugagc cagagucgcc aaaugcuaug guccucguug uucccaguga 5940
   gacuuuaucg ucuucugaac ugauuuuuag uggucuaagu cuaugcuccc uguggagaua 6000
   gacgaagagg ucccugccuu cccguuuggc gaaagcccag gggccaccgu aggugaucca 6060
   auccaguuca ucacuauaau cguagggggu ggucacuuca ucuucgaauu cuaggggcag 6120
   uccuuuugua augugguaca uggugugaaa aauguuguuc uuguuguaac cauaaccaag 6180
   auguucaucu ccuaauggug uccuauugua uaccucauau augccuucgg ggccagagaa 6240
   aguaucuuuc uccacucucu cggguccggc aauguugaag gugucgaaca ggucggcuuu 6300
   ggcauucuua aaguaaccaa aggccaggau gagggcggcu auuccagccc uguagucuag 6360
   gaggccaaaa auggcaauua auaccaccac gaaaacccua guggaauccg ucacgacccu 6420
   guggucgugg aggcuacuca uaucauauug uaacuugccu uccuuaaggg uuaugguacc 6480
   gacuccuguc gguggaucau cgucuuuguc uauggguacu aacaguuggg augugaggug 6540
   ccguguucga acaguagcgc cccuccucau gaggauguag agggccgcca cugggaacag 6600
   gauguaggaa agguucuugg gugcuugugc uaaggccaga cagaucgcuc cuccugccac 6660
   caucugcaua augcucucuu uuuuguagga agaccaauac caucguguca uguuguaaau 6720
   cgguuuaggg aacaugaaga uaaguaugac uucgagugug augagcaagc ugaugauaaa 6780
   ccuagucgga cuaucaaugg cagucuguac ugcauaccaa ccaaauugac ugauugguuc 6840
   cauugauaau gguguuaggu gguggaguag ugcuguugcg aagaugguga ucugggucuu 6900
   agcuccaacc ggucuuacac gaaacccaug guuaaauaug aggguuguua gugucaaaaa 6960
   agucuuaagg agccauucaa aggcuucagu cgcgggcuca aaggcgaaug aagcuaagga 7020
   aaacccagaa caacaguggc uguggugguu aguucgcuug caccguuagu gagugguugu 7080
   ucuccaucug ucaguaguuc ucuaauuaag uuccuaaucu guuuggugcc uuucgcuucu 7140
   guuccuuucu uguagcuccu gagggcuuuu ucaauucggg ugggggagua ugaaagcaac 7200
   uccaucucac ucuucaaugu ugcuggagaa uugauaaaau ugucauugcu ggcuguucuc 7260
   ucaccuccug aggcuuugac uagcucauug augagcuggu uaacggcuuu ggugccggug 7320
   gcccucugca ucuuguagaa accaguggcu gcaucaauug uggcuugguu guggccguuc 7380
   ucuugcaugg cuguggcuag gcuaguuuuu ccggauauga aaucuucauc auggucgugu 7440
   ucuucuuccu ugucuacggg gguaaaaccu ucaucuauuu ccucgucgcc aucguuggcu 7500
   uugauuacag ugguguugca uuucuucugu uugcaccagg uggaguuaaa ggucagggcu 7560
   cugucuuuau agagagaucu cuugaggagu cucucgcuag cuuccuugcg uaggaugaua 7620
   ccgccucuau aggcguucuc ggcugcugac auuucuuucu ucucaucuuc uucguucugu 7680
   uuuuugguga uuggccagca guugaaguug cauucugguc cuccaaaucu cuugggccag 7740
   auguguccuu uugugguggu guugacucuc ucgcagagag aggagcagcu gauuccgcag 7800
   cuccuccaac uucuccuuuu ugcuccaaau auuuggguga cagugccuuc gacuuugugu 7860
   gucaggcagu auuccuucuc uucaucagug uggaugguuc caaaccaggu aacuccgugg 7920
   gccgaacauu uauaauguuu cccugcccua cggcagucca cugugagguc uuugagguaa 7980
   gauucugguc uuucuucgcc uuuauaguag acugguggug uggagguggu gguuucuaac 8040
   ucuuuggugu cauacaucca uccaucgaca ucgucuaccu cauugacuuc caaaaagauu 8100
   ucucggcagu ucuugcagug uuuuucugaa uuauggcagg ugauuagugc uccuuguuuc 8160
   ccaucaucuu ugaguuuacg ggagguuuga ucccaagagg uggugucauu cacgucaugg 8220
   ucuguaccau caguggcgca uucuucuccu ucaucugugu agacuuugug gaguacggag 8280
   ccuaaggcuu uggcggcuuu cuggauuugu ucucuguuug gcauccuaag gauuguuaag 8340
   ccguccauga ggucuaaguc uauguuguag cugucuccua auugagggac auacagcuug 8400
   auugaugaau cgaacaugac guccuugaua cugccacauu ccauugacca uucaucugug 8460
   ucagccacgc aaccauacuu cucugcuucu accuccguua uuacuaaggg ggccaugagg 8520
   auaaguccua ugaugacugu gagauucuuc auguuaccug ucauuagggu uccgagccau 8580
   acuauggcuc cucccgucac ugccaucaug auuguugaug ucauuccgaa aaggcuuccu 8640
   aggaagccug cuccuuuggc aaagaaguuc uugaacaugu cuaguacugg ucucuuaacu 8700
   gcuccaucua ugguuauucu gucuuugacu uccagaacgu uaguggucca ucugcuaagc 8760
   cauccuguga uacccauuug ggggacauuc acuugaugcu uguuguugcc uaguuuaauu 8820
   ucaugcucuc cgggugggca gugcauuuuc aguacuucuu ugguugaagu gacuaugugu 8880
   gccugaucga aaagggugca guaguuggag guguauccag agccaacuug gcugcggcua 8940
   cgucccuuga cgacuaaccu gcacuggcua ggaacugcca uaucuaaguu aaaggugaca 9000
   acuccaucgu gaguggugag uauucugcug ccuucgauca guuugaaugc guaguugguu 9060
   gugucugcac aguccuucac acugguggug uguauuuuaa gggaauuaag caugaaacug 9120
   cagucugugg ggcuaauguc uaagggcauc uuggugccug gggaggcucu uccugauccu 9180
   uuggccaucu cggcuaugau uaucuucccg uucuugucca ggaaauucuu gaccauggau 9240
   ucgacguuau gggcuucuga aaaucucacg uaauggaggg uggugucucu ccaaguuaua 9300
   uaggucucgg caucauggac guugccaucu ucaaucuugc uaaggcuggu ugggauagac 9360
   gugguccaua cggaccaggg gaguuucauu gauucaaagg ccaaucuauc uacgagauau 9420
   gauucgccau cauuuggugc caucuuuauc agguacuugg uggcaaguuc ugcguguggu 9480
   agcuggcaug uuacucugac ucgugaggac gagucggcuu ugucugugau gucgaaacuu 9540
   ccuguguuug uguccauguu guaauaguuc ugauuuucug ugcguccaaa ggagaccguu 9600
   aagcgggcuc ccaucuuugu ugggucgauu guauagaccu guaucggaug uuugagggcu 9660
   aguucagcac aaguacauac cgguccuugu ccgaagagga agcagccagu gguccagcca 9720
   cgaucauggu auccucuagc gcagauggaa uaugguccug auuuugggca ggcggguucu 9780
   ccaucuccug ggcaucuguc ggaugaaacu gaguuggaac uaauguccaa ccaggauauu 9840
   uuguauccug ugagugcauu gucuauauag ggguagcuca uucuggccgu gauugugucc 9900
   augccuaccg guuggaucug auagcaaugc uuguagucca ugcggaggcu gacugaggug 9960
   ugugcuacuc cagucauggu ugacauuucu uccgggucag agcagagugu uuuuccgcuc 10020
   auuucagugg uugaagaggu cgaaucggcu accccgaggu aaaccuugga aaagaggaug 10080
   aucagcagca gguggacagu ggguacuuua aaauaggcca uaaccagggc cagaagggcu 10140
   augguuaugu cuguguuuga uagcacuuug gucucccaug uugauagggu cuucauccag 10200
   ccuucugaug gcugguuggu guguaucuca uacuucugaa uaguaucacg ugacagaucg 10260
   cgucuguuuc uagucuuccu ugaaaggucu ugauagaggg uugaauggau gugucucugc 10320
   cucagagaug uaguuccgaa acaugugucu gcugccguug guagcacucc ugcucugauc 10380
   agggcacagu cauguucauc guauuucaau ucuggugaug aguaguuggu guccggcugg 10440
   aagccggugg aacacucugu gagaucaaug ccauagguuu gguuguugcg uaugaaaagu 10500
   ucaggaucuu ugguguuguc caucuuugca aaaaguccuu ccucauucac caguaguaga 10560
   uccauagagg aagucuuguc cuugcggaca caugcuauca ccauugguga gaugucuguu 10620
   gggguggugg gugaaggugu uggugaugug gaggucccuc cagggaucac uuggguggca 10680
   aagaagauca ugaggagggu cagcaagccu gcugugccca uuccggaacu cuucuucuua 10740
   cgcuucguug ggccuuugau ggccugcugc acggaguuga gguugguuau caucuuucug 10800
   agucgccuca caccguugau auucuugggg auuuguggug guuuauucca gauucuccuc 10860
   agaaugacgg cugcucucuu gagcaggauc ucaaaggucu gcuguauuuu gccgcguccu 10920
   cucuggacgu gacgcagacc acggccggcu gauugccugg caugguggau ggcucucuug 10980
   gugcccccaa agccuugggu gacacggcgg gcaacccuga aggggguucc ggcgagacgc 11040
   augauuccuc uguaacucau ugucuugaga aguggaccca gccggguuau ucuucuaagu 11100
   auguuguuug aguguuuugu uuuuuguuug uuuguuuuau guguguguuu uuuguuuauu 11160
   auaauuuauu auauuauguu auauuguguu uguuuuauaa uaccauucaa uauugggugu 11220
   uguucaauuc ugaauuggug uaauuuaaca auauucugaa acguugaacc agugguuaaa 11280
   cuguguagcg accuaaaugu ugguagcgac uaaauuggcg ugucagaaug uaaauu 11336
<210> 21
   <211> 2659
   <212> DNA
   <213> Flaviviridae Virus
<400> 21
   ggtccacttc tcaagacaat gagttacaga ggaatcatgc gtctcgccgg aacccccttc      60
   agggttgccc gccgtgtcac ccaaggcttt gggggcacca agagagccat ccaccatgcc     120
   aggcaatcag ccggccgtgg tctgcgtcac gtccagagag gacgcggcaa aatacagcag     180
   acctttgaga tcctgctcaa gagagcagcc gtcattctga ggagaatctg gaataaacca     240
   ccacaaatcc ccaagaatat caacggtgtg aggcgactca gaaagatgat aaccaacctc     300
   aactccgtgc agcaggccat caaaggccca acgaagcgta agaagaagag ttccggaacg     360
   ggcacagcag gcttgctgac cctcctcatg atcttctttg ccacccaagt gatccctgga     420
   gggacctcca catcaccaac accttcaccc accaccccaa cagacatctc accaatggtg     480
   atagcatgtg tccgcaagga caagacttcc tctatggatc tactgctggt gaatgaggaa     540
   ggactttttg caaagatgga caacaccaaa gatcctgaac ttttcatacg caacaaccaa     600
   acctatggca ttgatctcac agagtgttcc accggcttcc agccggacac caactactca     660
   tcaccagaat tgaaatacga tgaacatgac tgtgccctga tcagagcagg agtgctacca      720
   acggcagcag acacatgttt cggaactaca tctctgaggc agagacacat ccattcaacc      780
   ctctatcaag acctttcaag gaagactaga aacagacgcg atctgtcacg tgatactatt      840
   cagaagtatg agatacacac caaccagcca tcagaaggct ggatgaagac cctatcaaca      900
   tgggagacca aagtgctatc aaacacagac ataaccatag cccttctggc cctggttatg      960
   gcctatttta aagtacccac tgtccacctg ctgctgatca tcctcttttc caaggtttac     1020
   ctcggggtag ccgattcgac ctcttcaacc actgaaatga gcggaaaaac actctgctct     1080
   gacccggaag aaatgtcaac catgactgga gtagcacaca cctcagtcag cctacgcatg     1140
   gactacaagc attgctatca gatccaaccg gtaggcatgg acacaatcac ggccagaatg     1200
   agctacccct acatagacaa tgcactcaca ggatacaaaa tatcctggtt ggacattagt     1260
   tccaactcag tttcatccga cagatgccca ggagatggag aacccgcctg cccaaaatca     1320
   ggaccatatt ccatctgcgc tagaggatac catgatcgtg gctggaccac tggctgcttc     1380
   ctcttcggac aaggaccggt atgtacttgt gctgaactaa ccctcaaaca tccgatacag     1440
   gtctatacaa tcgacccaac aaagatggga gcccgcttaa cggtctcctt tggacgcaca     1500
   gaaaatcaga actattacaa catggacaca aacacaggaa gtttcgacat cacagacaaa     1560
   gccgactcgt cctcacgagt cagagtaaca tgccagctac cacacgcaga acttgccacc     1620
   aagtacctga taaagatggc accaaatgat ggcgaatcat atctcgtaga tagattggcc     1680
   tttgaatcaa tgaaactccc ctggtccgta tggaccacgt ctatcccaac cagccttagc     1740
   aagattgaag atggcaacgt ccatgatgcc gagacctata taacttggag agacaccacc     1800
   ctccattacg tgagattttc agaagcccat aacgtcgaat ccatggtcaa gaatttcctg     1860
   gacaagaacg ggaagataat catagccgag atggccaaag gatcaggaag agcctcccca     1920
   ggcaccaaga tgcccttaga cattagcccc acagactgca gtttcatgct taattccctt     1980
   aaaatacaca ccaccagtgt gaaggactgt gcagacacaa ccaactacgc attcaaactg     2040
   atcgaaggca gcagaatact caccactcac gatggagtcg tcacctttaa cttagatatg     2100
   gcagttccta gccagtgcag gttagtcgtc aagggacgta gccgcagcca agttggctct     2160
   ggatacacct ccaactactg cacccttttc gatcaggcac acatagtcac ttcaaccaaa      2220
   gaagtactga aaatgcactg cccacccgga gagcatgaaa ttaaactagg caacaacaag     2280
   tatcaagtga atgtccccca aatgggtatc acaggatggc ttagcagatg gaccactaac     2340
   gttctggaag tcaaagacag aataaccata gatggagcag ttaagagacc agtactagac     2400
   atgttcaaga acttctttgc caaaggagca ggcttcctag gaagcctttt cggaatgaca     2460
   tcaacaatca tgatggcagt gacgggagga gccatagtat ggctcggaac cctaatgaca     2520
   ggtaacatga agaatctcac agtcatcata ggacttatcc tcatggcccc cttagtaata     2580
   acggaggtag aagcagagaa gtatggttgc gtggctgaca cagatgaatg gtcaatggaa     2640
   tgtggcagta tcaaggacg                                                  2659
<210> 22
   <211> 2661
   <212> DNA
   <213> Flaviviridae Virus
<400> 22
   ggtccacttc tcaagacaat gagttacaga ggaatcatgc gtctcgccgg aacccccttc        60
   agggttgccc gccgtgtcac ccaaggcttt gggggcacca agagagccat ccaccatgcc      120
   aggcaatcag ccggccgtgg tctgcgtcac gtccagagag gacgcggcaa aatacagcag      180
   acctttgaga tcctgctcaa gagagcagcc gtcattctga ggagaatctg gaataaacca      240
   ccacaaatcc ccaagaatat caacggtgtg aggcgactca gaaagatgat aaccaacctc      300
   aactccgtgc agcaggccat caaaggccca acgaagcgta agaagaagag ttccggaacg      360
   ggcacagcag gcttgctgac cctcctcatg atcttctttg ccacccaagt gatccctgga      420
   gggacctcca catcaccaac accttcaccc accaccccaa cagacatctc accaatggtg      480
   atagcatgtg tccgcaagga caagacttcc tctatggatc tactactggt gaatgaggaa      540
   ggactttttg caaagatgga caacaccaaa gatcctgaac ttttcatacg caacaaccaa      600
   acctatggca ttgatctcac agagtgttcc accggcttcc agccggacac caactactca      660
   tcaccagaat tgaaatacga tgaacatgac tgtgccctga tcagagcagg agtgctacca      720
   acggcagcag acacatgttt cggaactaca tctctgaggc agagacacat ccattcaacc      780
   ctctatcaag acctttcaag gaagactaga aacagacgcg atctgtcacg tgatactatt      840
   cagaagtatg agatacacac caaccagcca tcagaaggct ggatgaagac cctatcaaca      900
   tgggagacca aagtgctatc aaacacagac ataaccatag cccttctggc cctggttatg      960
   gcctatttta aagtacccac tgtccacctg ctgctgatca tcctcttttc caaggtttac     1020
   ctcggggtag ccgattcgac ctcttcaacc actgaaatga gcggaaaaac actctgctct     1080
   gacccggaag aaatgtcaac catgactgga gtagcacaca cctcagtcag cctccgcatg     1140
   gactacaagc attgctatca gatccaaccg gtaggcatgg acacaatcac ggccagaatg     1200
   agctacccct acatagacaa tgcactcaca ggatacaaaa tatcctggtt ggacattagt     1260
   tccaactcag tttcatccga cagatgccca ggagatggag aacccgcctg cccaaaatca     1320
   ggaccatatt ccatctgcgc tagaggatac catgatcgtg gctggaccac tggctgcttc 1380
   ctcttcggac aaggaccggt atgtacttgt gctgaactaa ccctcaaaca tccgatacag 1440
   gtctatacaa tcgacccaac aaagatggga gcccgcttaa cggtctcctt tggacgcaca 1500
   gaaaatcaga actattacaa catggacaca aacacaggaa gtttcgacat cacagacaaa 1560
   gccgactcgt cctcacgagt cagagtaaca tgccagctac cacacgcaga acttgccacc 1620
   aagtacctga taaagatggc accaaatgat ggcgaatcat atctcgtaga tagattggcc 1680
   tttgaatcaa tgaaactccc ctggtccgta tggaccacgt ctatcccaac cagccttagc 1740
   aagattgaag atggcaacgt ccatgatgcc gagacctata taacttggag agacaccacc 1800
   ctccattacg tgagattttc agaagcccat aacgtcgaat ccatggtcaa gaatttcctg 1860
   gacaagaacg ggaagataat catagccgag atggccaaag gatcaggaag agcctcccca 1920
   ggcaccaaga tgcccttaga cattagcccc acagactgca gtttcatgct taattccctt 1980
   aaaatacaca ccaccagtgt gaaggactgt gcagacacaa ccaactacgc attcaaactg 2040
   atcgaaggca gcagaatact caccactcac gatggagtcg tcacctttaa cttagatatg 2100
   gcagttccta gccagtgcag gttagtcgtc aagggacgta gccgcagcca agttggctct 2160
   ggatacacct ccaactactg cacccttttc gatcaggcac acatagtcac ttcaaccaaa 2220
   gaagtactga aaatgcactg cccacccgga gagcatgaaa ttaaactagg caacaacaag 2280
   tatcaagtga atgtccccca aatgggtatc acaggatggc ttagcagatg gaccactaac 2340
   gttctggaag tcaaagacag aataaccata gatggagcag ttaagagacc agtactagac 2400
   atgttcaaga acttctttgc caaaggagca ggcttcctag gaagcctttt cggaatgaca 2460
   tcaacaatca tgatggcagt gacgggagga gccatagtat ggctcggaac cctaatgaca 2520
   ggtaacatga agaatctcac agtcatcata ggacttatcc tcatggcccc cttagtaata 2580
   acggaggtag aagcagagaa gtatggttgc gtggctgaca cagatgaatg gtcaatggaa 2640
   tgtggcagta tcaaggacgt c 2661
<210> 23
   <211> 2644
   <212> DNA
   <213> Flaviviridae Virus
<400> 23
   ctcaagacaa tgagttacag aggaatcatg cgtctcgccg gaaccccctt cagggttgcc      60
   cgccgtgtca cccaaggctt tgggggcacc aagagagcca tccaccatgc caggcaatca     120
   gccggccgtg gtctgcgtca cgtccagaga ggacgcggca aaatacagca gacctttgag     180
   atcctgctca agagagcagc cgtcattctg aggagaatct ggaataaacc accacaaatc     240
   cccaagaata tcaacggtgt gaggcgactc agaaagatga taaccaacct caactccgtg     300
   cagcaggcca tcaaaggccc aacgaagcgt aagaagaaga gttccggaac gggcacagca     360
   ggcttgctga ccctcctcat gatcttcttt gccacccaag tgatccctgg agggacctcc     420
   acatcaccaa caccttcacc caccacccca acagacatct caccaatggt gatagcatgt     480
   gtccgcaagg acaagacttc ctctatggat ctactactgg tgaatgagga aggacttttt     540
   gcaaagatgg acaacaccaa agatcctgaa cttttcatac gcaacaacca aacctatggc     600
   attgatctca cagagtgttc caccggcttc cagccggaca ccaactactc atcaccagaa     660
   ttgaaatacg atgaacatga ctgtgccctg atcagagcag gagtgctacc aacggcagca     720
   gacacatgtt tcggaactac atctctgagg cagagacaca tccattcaac cctctatcaa     780
   gacctttcaa ggaagactag aaacagacgc gatctgtcac gtgatactat tcagaagtat     840
   gagatacaca ccaaccagcc atcagaaggc tggatgaaga ccctatcaac atgggagacc     900
   aaagtgctat caaacacaga cataaccata gcccttctgg ccctggttat ggcctatttt     960
   aaagtaccca ctgtccacct gctgctgatc atcctctttt ccaaggttta cctcggggta    1020
   gccgattcga cctcttcaac cactgaaatg agcggaaaaa cactctgctc tgacccggaa    1080
   gaaatgtcaa ccatgactgg agtagcacac acctcagtca gcctccgcat ggactacaag    1140
   cattgctatc agatccaacc ggtaggcatg gacacaatca cggccagaat gagctacccc    1200
   tacatagaca atgcactcac aggatacaaa atatcctggt tggacattag ttccaactca    1260
   gtttcatccg acagatgccc aggagatgga gaacccgcct gcccaaaatc aggaccatat    1320
   tccatctgcg ctagaggata ccatgatcgt ggctggacca ctggctgctt cctcttcgga    1380
   caaggaccgg tatgtacttg tgctgaacta accctcaaac atccgataca ggtctataca    1440
   atcgacccaa caaagatggg agcccgctta acggtctcct ttggacgcac agaaaatcag    1500
   aactattaca acatggacac aaacacagga agtttcgaca tcacagacaa agccgactcg    1560
   tcctcacgag tcagagtaac atgccagcta ccacacgcag aacttgccac caagtacctg    1620
   ataaagatgg caccaaatga tggcgaatca tatctcgtag atagattggc ctttgaatca    1680
   atgaaactcc cctggtccgt atggaccacg tctatcccaa ccagccttag caagattgaa    1740
   gatggcaacg tccatgatgc cgagacctat ataacttgga gagacaccac cctccattac    1800
   gtgagatttt cagaagccca taacgtcgaa tccatggtca agaatttcct ggacaagaac    1860
   gggaagataa tcatagccga gatggccaaa ggatcaggaa gagcctcccc aggcaccaag    1920
   atgcccttag acattagccc cacagactgc agtttcatgc ttaattccct taaaatacac    1980
   accaccagtg tgaaggactg tgcagacaca accaactacg cattcaaact gatcgaaggc    2040
   agcagaatac tcaccactca cgatggagtc gtcaccttta acttagatat ggcagttcct 2100
   agccagtgca ggttagtcgt caagggacgt agccgcagcc aagttggctc tggatacacc 2160
   tccaactact gcaccctttt cgatcaggca cacatagtca cttcaaccaa agaagtactg 2220
   aaaatgcact gcccacccgg agagcatgaa attaaactag gcaacaacaa gtatcaagtg 2280
   aatgtccccc aaatgggtat cacaggatgg cttagcagat ggaccactaa cgttctggaa 2340
   gtcaaagaca gaataaccat agatggagca gttaagagac cagtactaga catgttcaag 2400
   aacttctttg ccaaaggagc aggcttccta ggaagccttt tcggaatgac atcaacaatc 2460
   atgatggcag tgacgggagg agccatagta tggctcggaa ccctaatgac aggtaacatg 2520
   aagaatctca cagtcatcat aggacttatc ctcatggccc ccttagtaat aacggaggta 2580
   gaagcagaga agtatggttg cgtggctgac acagatgaat ggtcaatgga atgtggcagt 2640
   atca 2644
<210> 24
   <211> 2673
   <212> DNA
   <213> Flaviviridae Virus
<400> 24
   agaataaccc ggctgggtcc acttctcaag acaatgagtt acagaggaat catgcgtctc 60
   gccggaaccc ccttcagggt tgcccgccgt gtcacccaag gctttggggg caccaagaga 120
   gccatccacc atgccaggca atcagccggc cgtggtctgc gtcacgtcca gagaggacgc 180
   ggcaaaatac agcagacctt tgagatcctg ctcaagagag cagccgtcat tctgaggaga 240
   atctggaata aaccaccaca aatccccaag aatatcaacg gtgtgaggcg actcagaaag 300
   atgataacca acctcaactc cgtgcagcag gccatcaaag gcccaacgaa gcgtaagaag 360
   aagagttccg gaacgggcac agcaggcttg ctgaccctcc tcatgatctt ctttgccacc 420
   caagtgatcc ctggagggac ctccacatca ccaacacctt cacccaccac cccaacagac 480
   atctcaccaa tggtgatagc atgtgtccgc aaggacaaga cttcctctat ggatctacta 540
   ctggtgaatg aggaaggact ttttgcaaag atggacaaca ccaaagatcc tgaacttttc 600
   atacgcaaca accaaaccta tggcattgat ctcacagagt gttccaccgg cttccagccg 660
   gacaccaact actcatcacc agaattgaaa tacgatgaac atgactgtgc cctgatcaga 720
   gcaggagtgc taccaacggc agcagacaca tgtttcggaa ctacatctct gaggcagaga 780
   cacatccatt caaccctcta tcaagacctt tcaaggaaga ctagaaacag acgcgatctg 840
   tcacgtgata ctattcagaa gtatgagata cacaccaacc agccatcaga aggctggatg 900
   aagaccctat caacatggga gaccaaagtg ctatcaaaca cagacataac catagccctt 960
   ctggccctgg ttatggccta ttttaaagta cccactgtcc acctgctgct gatcatcctc 1020
   ttttccaagg tttacctcgg ggtagccgat tcgacctctt caaccactga aatgagcgga 1080
   aaaacactct gctctgaccc ggaagaaatg tcaaccatga ctggagtagc acacacctca 1140
   gtcagcctcc gcatggacta caagcattgc tatcagatcc aaccggtagg catggacaca 1200
   atcacggcca gaatgagcta cccctacata gacaatgcac tcacaggata caaaatatcc 1260
   tggttggaca ttagttccaa ctcagtttca tccgacagat gcccaggaga tggagaaccc 1320
   gcctgcccaa aatcaggacc atattccatc tgcgctagag gataccatga tcgtggctgg 1380
   accactggct gcttcctctt cggacaagga ccggtatgta cttgtgctga actaaccctc 1440
   aaacatccga tacaggtcta tacaatcgac ccaacaaaga tgggagcccg cctaacggtc 1500
   tcctttggac gcacagaaaa tcagaactat tacaacatgg acacaaacac aggaagtttc 1560
   gacatcacag acaaagccga ctcgtcctca cgagtcagag taacatgcca gctaccacac 1620
   gcagaacttg ccaccaagta cctgataaag atggcaccaa atgatggcga atcatatctc 1680
   gtagatagat tggcctttga atcaatgaaa ctcccctggt ccgtatggac cacgtctatc 1740
   ccaaccagcc ttagcaagat tgaagatggc aacgtccatg atgccgagac ctatataact 1800
   tggagagaca ccaccctcca ttacgtgaga ttttcagaag cccataacgt cgaatccatg 1860
   gtcaagaatt tcctggacaa gaacgggaag ataatcatag ccgagatggc caaaggatca 1920
   ggaagagcct ccccaggcac caagatgccc ttagacatta gccccacaga ctgcagtttc 1980
   atgcttaatt cccttaaaat acacaccacc agtgtgaagg actgtgcaga cacaaccaac 2040
   tacgcattca aactgatcga aggcagcaga atactcacca ctcacgatgg agtcgtcacc 2100
   tttaacttag atatggcagt tcctagccag tgcaggttag tcgtcaaggg acgtagccgc 2160
   agccaagttg gctctggata cacctccaac tactgcaccc ttttcgatca ggcacacata 2220
   gtcacttcaa ccaaagaagt actgaaaatg cactgcccac ccggagagca tgaaattaaa 2280
   ctaggcaaca acaagtatca agtgaatgtc ccccaaatgg gtatcacagg atggcttagc 2340
   agatggacca ctaacgttct ggaagtcaaa gacagaataa ccatagatgg agcagttaag 2400
   agaccagtac tagacatgtt caagaacttc tttgccaaag gagcaggctt cctaggaagc 2460
   cttttcggaa tgacatcaac aatcatgatg gcagtgacgg gaggagccat agtatggctc 2520
   ggaaccctaa tgacaggtaa catgaagaat ctcacagtca tcataggact tatcctcatg 2580
   gcccccttag taataacgga ggtagaagca gagaagtatg gttgcgtggc tgacacagat 2640
   gaatggtcaa tggaatgtgg cagtatcaag gac 2673
<210> 25
   <211> 2645
   <212> DNA
   <213> Flaviviridae Virus
<400> 25
   tcaagacaat gagttacaga ggaatcatgc gtctcgccgg aacccccttc agggttgccc      60
   gccgtgtcac ccaaggcttt gggggcacca agagagccat ccaccatgcc aggcaatcag     120
   ccggccgtgg tctgcgtcac gtccagagag gacgcggcaa aatacagcag acctttgaga     180
   tcctgctcaa gagagcagcc gtcattctga ggagaatctg gaataaacca ccacaaatcc     240
   ccaagaatat caacggtgtg aggcgactca gaaagatgat aaccaacctc aactccgtgc     300
   agcaggccat caaaggccca acgaagcgta agaagaagag ttccggaatg ggcacagcag     360
   gcttgctgac cctcctcatg atcttctttg ccacccaagt gatccctgga gggacctcca     420
   catcaccaac accttcaccc accaccccaa cagacatctc accaatggtg atagcatgtg     480
   tccgcaagga caagacttcc tctatggatc tactactggt gaatgaggaa ggactttttg     540
   caaagatgga caacaccaaa gatcctgaac ttttcatacg caacaaccaa acctatggca     600
   ttgatctcac agagtgttcc accggcttcc agccggacac caactactca tcaccagaat     660
   tgaaatacga tgaacatgac tgtgccctga tcagagcagg agtgctacca acggcagcag     720
   acacatgttt cggaactaca tctctgaggc agagacacat ccattcaacc ctctatcaag     780
   acctttcaag gaagactaga aacagacgcg atctgtcacg tgatactatt cagaagtatg     840
   agatacacac caaccagcca tcagaaggct ggatgaagac cctatcaaca tgggagacca     900
   aagtgctatc aaacacagac ataaccatag cccttctggc cctggttatg gcctatttta     960
   aagtacccac tgtccacctg ctgctgatca tcctcttttc caaggtttac ctcggggtag    1020
   ccgattcgac ctcttcaacc actgaaatga gcggaaaaac actctgctct gacccggaag    1080
   aaatgtcaac catgactgga gtagcacaca cctcagtcag cctccgcatg gactacaagc    1140
   attgctatca gatccaaccg gtaggcatgg acacaatcac ggccagaatg agctacccct    1200
   acatagacaa tgcactcaca ggatacaaaa tatcctggtt ggacattagt tccaactcag    1260
   tttcatccga cagatgccca ggagatggag aacccgcctg cccaaaatca ggaccatatt    1320
   ccatctgcgc tagaggatac catgatcgtg gctggaccac cggctgcttc ctcttcggac    1380
   aaggaccggt atgtacttgt gctgaactaa ccctcaaaca tccgatacag gtctatacaa    1440
   tcgacccaac aaagatggga gcccgcttaa cggtctcctt tggacgcaca gaaaatcaga    1500
   actattacaa catggacaca aacacaggaa gtttcgacat cacagacaaa gccgactcgt    1560
   cctcacgagt cagagtaaca tgccagctac cacacgcaga acttgccacc aagtacctga    1620
   taaagatggc accaaatgat ggcgaatcat atctcgtaga tagattggcc tttgaatcaa 1680
   tgaaactccc ctggtccgta tggaccacgt ctatcccaac cagccttagc aagattgaag 1740
   atggcaacgt ccatgatgcc gagacctata taacttggag agacaccacc ctccattacg 1800
   tgagattttc agaagcccat aacgtcgaat ccatggtcaa gaatttcctg gacaagaacg 1860
   ggaagataat catagccgag atggccaaag gatcaggaag agcctcccca ggcaccaaga 1920
   tgcccttaga cattagcccc acagactgca gtttcatgct taattccctt aaaatacaca 1980
   ccaccagtgt gaaggactgt gcagatacaa ccaactacgc attcaaactg atcgaaggca 2040
   gcagaatact caccactcac gatggagtcg tcacctttaa cttagatatg gcagttccta 2100
   gccagtgcag gttagtcgtc aagggacgta gccgcagcca agttggctct ggatacacct 2160
   ccaactactg cacccttttc gatcaggcac acatagtcac ttcaaccaaa gaagtactga 2220
   aaatgcactg cccacccgga gagcatgaaa ttaaactagg caacaacaag tatcaagtga 2280
   atgtccccca aatgggtatc acaggatggc ttagcagatg gaccactaac gttctggaag 2340
   tcaaagacag aataaccata gatggagcag ttaagagacc agtactagac atgttcaaga 2400
   acttctttgc caaaggagca ggcttcctag gaagcctttt cggaatgaca tcaacaatca 2460
   tgatggcagt gacgggagga gccatagtat ggctcggaac cctaatgaca ggtaacatga 2520
   agaatctcac agtcatcata ggacttatcc tcatggcccc cttagtaata acggaggtag 2580
   aagcagagaa gtatggttgc gtggctgaca cagatgaatg gtcaatggaa tgtggcagta 2640
   tcaag 2645
<210> 26
   <211> 350
   <212> DNA
   <213> Flaviviridae Virus
<400> 26
   atgagttaca gaggaatcat gcgtctcgcc ggaaccccct tcagggttgc ccgccgtgtc 60
   acccaaggct ttgggggcac caagagagcc atccaccatg ccaggcaatc agccggccgt 120
   ggtctgcgtc acgtccagag aggacgcggc aaaatacagc agacctttga gatcctgctc 180
   aagagagcag ccgtcattct gaggagaatc tggaataaac caccacaaat ccccaagaat 240
   atcaacggtg tgaggcgact cagaaagatg ataaccaacc tcaactccgt gcagcaggcc 300
   atcaaaggcc caacgaagcg taagaagaag agttccggaa cgggcacagc 350

## Description

### FIELD OF THE INVENTION

The present invention relates to a newly discovered virus (Lumpfish flavivirus), which causes disease in fish, and the use of the isolated virus within veterinary immunology and diagnosis. More specifically, the invention provides nucleic acid sequences coding proteins and the use thereof in inter alia recombinant vaccines, DNA vaccines, live recombinant vaccines, and vaccines comprising attenuated or inactivated Lumpfish flavivirus. The protein coding sequences are also useful in respect of developing methods providing for immunological determination of virus infection. The invention thus also relates to antibodies, and the use thereof in immunoassays. The present invention also relates to primers useful in methods for detection of virus in a test sample. The present invention also provides diagnostic kits comprising recombinant proteins, antibodies and primers, respectively.

### BACKGROUND

The lumpfish (Cyclopterus lumpus L.) has become an important aquaculture species due to its efficacy as a cleaner fish for Atlantic salmon. This species has shown a high appetite for the ectoparasitic copepod, the sea louse *Lepeophtherius salmonis* Krøyer, representing a non-pharmaceutical and therefore an environmentally friendly delousing method when compared to chemical treatments. It is therefore important to ensure a stable supply of lumpfish for use as cleaner fish in the salmon industry.

Lumpfish, a common species along the Norwegian coast, has a broad geographical distribution, from Greenland in the north to the coast of Portugal in the east and Cape Cod in the south-west, indicating a broad temperature tolerance. This species in addition seems to be more robust and with a less complicated intensive production of larvae and juveniles compared to other cleaner fish species such as the ballan wrasse *Labrus bergylta,* goldsinny wrasse *Ctenolabrus rupestris* L. and corkwing wrasse *Symphodus melops* L.

Since the intensive production of this species is relatively new (around 2012), very little is known about the disease problems potentially faced by this species when being produced at high densities. At present, the main causes of death in cleaner fish are related to bacterial diseases. A number of bacteria with pathogenic potential have been isolated from farmed and wild caught cleaner fish including such species as *Vibrio anguillarum, Vibrio ordalii* and *Pseudomonas anguilliseptica,* and there are indications that primary pathogens of cleaner fish are also *Aeromonas salmonicida, Vibrio splendidus* and *Vibrio tapetis.* A few vaccines have been available for vaccinating lumpfish against bacterial diseases.

No viral diseases have been characterized in lumpfish until the description given in this patent application.

Elevated levels of mortalities were observed in a rearing facility for lumpfish in Norway. Histology samples were collected, and these showed mild to severe necrosis in the liver from all sampled lumpfish. The genome of a novel virus, most closely related to the virus family *Flaviviridae* was characterized. This novel virus is referred to herein as Lumpfish flavivirus.

It is therefore an object of the present invention to provide nucleic acid sequences originating from Lumpfish flavivirus, and various useful applications thereof.

### SUMMARY OF THE INVENTION

In a first aspect, the present invention provides an isolated flavivirus comprising a ribonucleic acid genome having at least 70% sequence identity to SEQ ID NO. 20. In an embodiment, the virus of the invention comprises a ribonucleic acid genome sequence having at least 80%, at least 85%, at least 90%, at least 91%, at least 92%, at least 93%, at least 94%, at least 95%, at least 96%, at least 97%, at least 98%, at least 99%, at least 99.5% or at least 99.9% sequence identity to SEQ ID NO. 20. The virus of the invention belongs to the family flaviviridae. In some embodiments, the virus of the invention is isolated from lumpfish. In some embodiments, the virus of the invention is a flavivirus isolated from a lumpfish. In some embodiment, the virus of the invention is a lumpfish flavivirus (LFV).

In a second aspect, the present invention provides an isolated DNA nucleic acid sequence originating from the virus of the invention having a sequence selected from the group consisting of: SEQ ID NO. 1, SEQ ID NO. 2, SEQ ID NO. 4, SEQ ID NO. 6, and variants thereof having at least 70% sequence identity to any of the sequences SEQ ID NO. 1, SEQ ID NO. 2, SEQ ID NO. 4 or SEQ ID NO. 6.

In some embodiments, the isolated DNA nucleic acid sequence originating from the virus of the invention may have at least 80%, at least 85%,at least 90%, at least 91%, at least 92%, at least 93%, at least 94%, at least 95%, at least 96%, at least 97%, at least 98% or at least 99% sequence identity to at least 99.5 %, or at least 99.9% sequence identity to a nucleic acid selected from the group consisting of: SEQ ID NO. 1, SEQ ID NO. 2, SEQ ID NO. 4, and SEQ ID NO. 6.

A third aspect of the invention relates to a vector comprising a nucleic acid sequence according to the second aspect of the present invention.

In some embodiments, a vector is provided comprising a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4 or SEQ ID NO. 6, wherein the nucleic acid sequence is operably or operatively linked to control sequences directing the expression of said nucleic acid sequences. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

In a fourth aspect of the invention, a host cell is provided comprising a vector of the third aspect.

DNA vaccines are disclosedcomprising a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4, or SEQ ID NO. 6. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

Disclosed herein are recombinant proteins encoded by a nucleic acid sequence selected from the group consisting of: SEQ ID NO. 2, SEQ ID NO. 4, SEQ ID NO. 6, and variants or fragments thereof which have at least 70 % sequence identity to any of the sequences SEQ ID NO. 2, SEQ ID NO. 4, or SEQ ID NO. 6. In some embodiments, the variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 2, SEQ ID NO. 4, and SEQ ID NO. 6.

Recombinant proteins are disclosed which have an amino acid sequence selected from the group consisting of: SEQ ID NO. 3, SEQ ID NO. 5, SEQ ID NO. 7, and variants or fragments thereof which have at least 70 % sequence identity to any of the sequences SEQ ID NO. 3, SEQ ID NO. 5, or SEQ ID NO. 7. The variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 3, SEQ ID NO. 5, and SEQ ID NO. 7.

A recombinant vaccine is disclosed comprising at least one of the recombinant proteins disclosed herein.

An antibody is disclosed that specifically recognises and binds to a recombinant protein disclosed herein. The antibody may be a monoclonal antibody or a polyclonal antibody.

An immunoassay is disclosed for the detection of antibodies specific for the virus of the invention in a biological sample. The immunoassay comprises the following steps:
a) contacting a biological sample suspected to comprise antibodies raised against the virus of the invention with at least one of the recombinant proteins disclosed herein; and
(b) determining whether the at least one recombinant protein binds to antibodies present in the biological sample wherein said binding represents an indication that the sample contains antibodies specific for the virus of the invention.

IThe virus of the invention may be isolated from lumpfish. The virus of the invention is from the family flaviviridae, The virus of the invention may be a Lumpfish flavivirus.

An immunoassay is disclosed herein for detection of the virus of the invention in a biological sample. The immunoassay comprises the following steps:
(a) contacting a biological sample suspected to comprise the virus of the invention with one or more antibodies that specifically recognise and bind to a recombinant protein as disclosed herein (such as an antibody as disclosed herein); and
(b) determining whether the one or more antibody binds to the virus of the invention present in the biological sample wherein said binding represents an indication that the sample contains the virus of the invention.

The one or more antibody may be a monoclonal antibody.

The virus of the invention may be isolated from lumpfish. The virus of the invention is from the family flaviviridae, The virus of the invention may be a Lumpfish flavivirus (LFV).

Also disclosed herein is a primer comprising a sequence of at least about 10 nucleotides, wherein said primer hybridizes to a nucleic acid sequence originating from the virus of the invention having a sequence selected from the group consisting of: SEQ ID NO. 1, SEQ ID NO. 2, SEQ ID NO. 4, SEQ ID NO. 6, and variants or fragments thereof which have at least 70 % sequence identity to any of the sequences SEQ ID NO. 1, SEQ ID NO. 2, SEQ ID NO. 4, or SEQ ID NO. 6. The primer may be suitable for use in PCR and hybridizes to the nucleic acid sequence at a temperature of at least 50 °C.The variants or fragments may have at least 80%, preferably at least 90 %, at least 92 %, at least 95 %, at least 97 %, at least 99 %, at least 99.5 %, or at least 99.9% sequence identity to a nucleic acid selected from the group consisting of: SEQ ID NO. 1, SEQ ID NO. 2, SEQ ID NO. 4, and SEQ ID NO. 6.

Also disclosed herein, the primer may consist of or comprise at least 15 nucleotides, such as at least 20 nucleotides.

The virus of the invention may be isolated from lumpfish. The virus of the invention is from the family flaviviridae, The virus of the invention may be a Lumpfish flavivirus (LFV).

A method for the detection of the virus of the invention in a biological sample is disclosed herein. The method comprises the following steps:
a) isolating nucleic acids from a biological sample suspected to comprise the virus of the invention;
b) contacting the nucleic acids with at least one primer as disclosed herein;
c) subjecting the mixture of b) to stringent hybridisation conditions; and,
d) determining whether hybridisation between the primer and the nucleic acids has occurred, wherein hybridisation indicates the presence of the virus of the invention in the biological sample.

The mixture of b) may be also subjected to reverse transcription and amplification conditions, preferably suitable PCR conditions.

The stringent hybridisation conditions may comprise hybridization in a buffer consisting of 6 X SSC, 50 mM Tris-HCL (pH=7.5), 1 mM EDTA, 0.02% PVP, 0.02% Ficoll, 0.02% BSA and 100 µg/ml denatured salmon sperm DNA, for 48 hours at 65°C, washing in a buffer consisting of 2 X SSC, 25 0.01% PVP, 0.01% Ficoll, 0.01% BSA for 45 minutes at 37°C, and washing in a buffer consisting of 0.1 X SSC, for 45 minutes at 50°C.
The virus of the invention may be isolated from lumpfish. The virus of the invention is from the family flaviviridae, The virus of the invention may be a Lumpfish flavivirus (LFV).

Also disclosed herein is a diagnostic kit comprising at least one primer sequence as disclosed herein.

The use of the nucleic acid sequences of the second aspect of the invention for the preparation of a DNA vaccine, a recombinant vaccine, or a live recombinant microorganism is also disclosed herein.

The isolated virus of the invention may be used in medicine. For example, the isolated virus may be used in inactivated or attenuated form as a vaccine.

The isolated virus of the invention may be used as part of a vaccine formulation in a method of preventing an infection in a lumpfish. The isolated virus may be attenutated or killed. The attenuated or killed virus of the invention may be formulated with an adjuvant.

Also disclosed herein is a vaccine comprising the virus of the invention in inactivated or attenuated form.

The virus of the invention may be isolated from lumpfish. The virus of the invention is from the family flaviviridae, The virus of the invention may be a Lumpfish flavivirus (LFV).

The vaccine may be used in the treatment or prevention of liver necrosis in lumpfish.

### BRIEF DESCRIPTION OF THE FIGURES

Figures 1A-1D discloses the DNA sequence (SEQ ID NO. 1) corresponding to the genomic sequence of the Lumpfish flavivirus, which is 11336 nucleotides in length.
Figure 2A and 2B discloses the DNA sequence (SEQ ID NO. 2) corresponding to the open reading frame 1 of the Lumpfish flavivirus. The open reading frame 1 comprises the sequence starting at the nucleotide in position 277 and ending at the nucleotide in position 4323 of SEQ ID NO. 1.
Figure 3 discloses the amino acid sequence coded by the sequence depicted in figure 2, i.e. the amino acid sequence corresponding to SEQ ID NO. 3.
Figure 4A-4C discloses the DNA sequence (SEQ ID NO. 4) corresponding to the open reading frame 2 of the Lumpfish flavivirus. The open reading frame 2 comprises the sequence starting at the nucleotide in position 4320 and ending at the nucleotide in position 10727 of SEQ ID NO. 1.
Figure 5 discloses the amino acid sequence coded by the sequence depicted in figure 4, i.e. the amino acid sequence corresponding to SEQ ID NO. 5.
Figure 6 discloses the DNA sequence (SEQ ID NO. 6) corresponding to the putative envelope protein of the Lumpfish flavivirus. This sequence starts at the nucleotide in position 1297 and ends at the nucleotide in position 2853 of SEQ ID NO. 1.
Figure 7 discloses the amino acid sequence coded by the sequence depicted in figure 6, i.e. the amino acid sequence corresponding to the putative envelope protein, SEQ ID NO. 7.

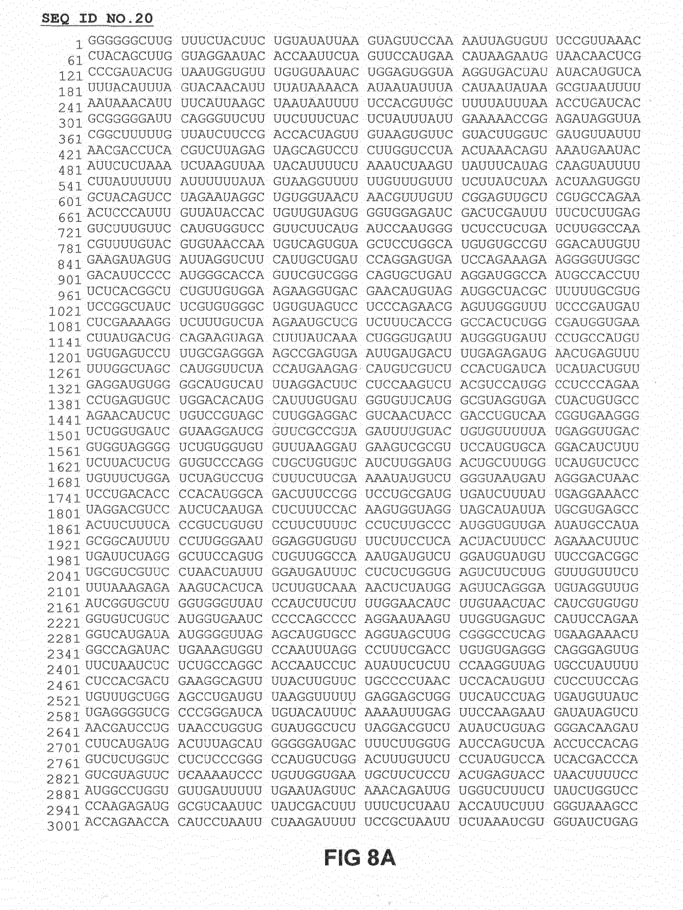

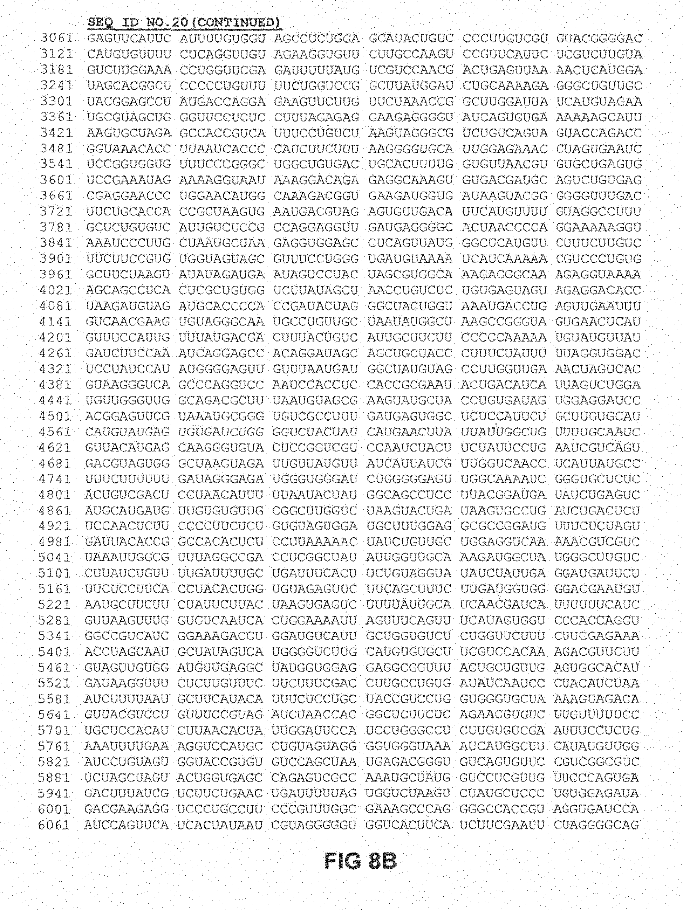

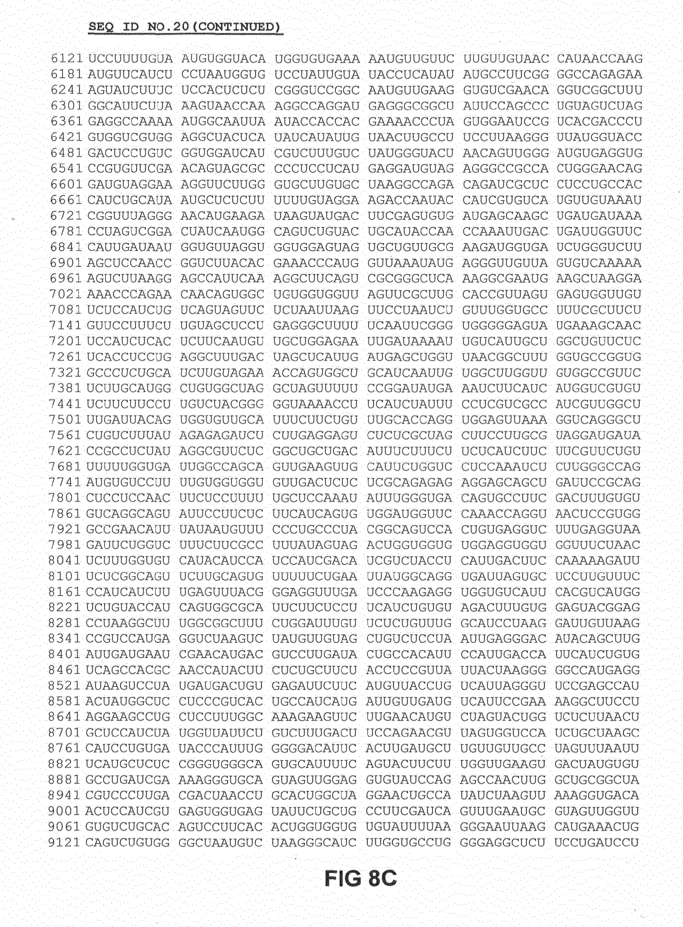

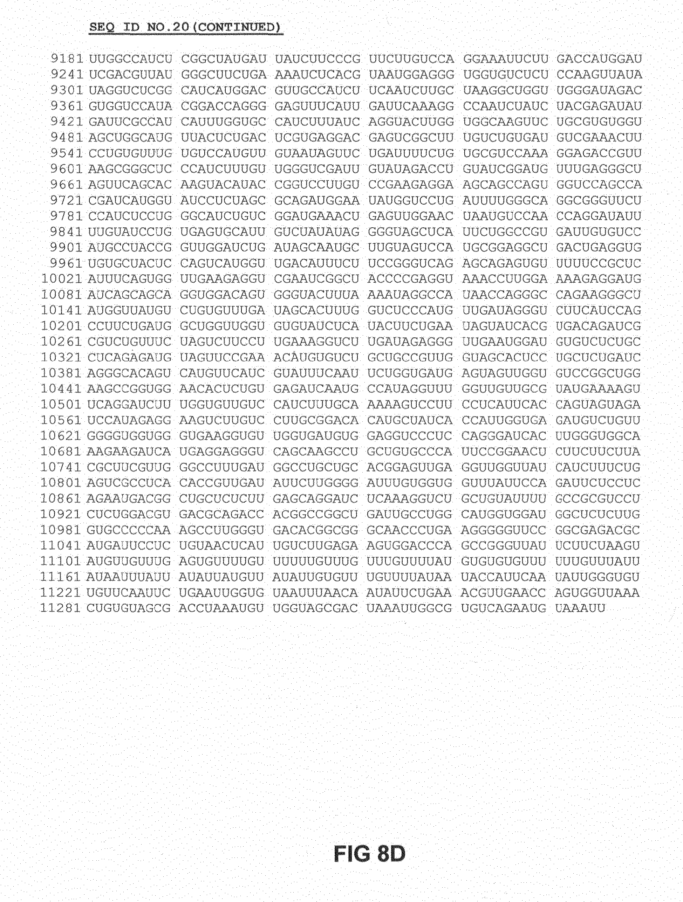

Figure 8A-8D discloses the RNA sequence (SEQ ID NO.8) corresponding to the RNA genome of the Lumpfish flavivirus, which is 11336 nucleotides in length.
Figure 9 discloses the DNA sequence (SEQ ID NO. 21) corresponding assembled sequence of the sequence of the LFV strain 1 isolate of Lumpfish flavivirus.
Figure 10 discloses the DNA sequence (SEQ ID NO. 22) corresponding assembled sequence of the sequence of the LFV strain 2 isolate of Lumpfish flavivirus.
Figure 11 discloses the DNA sequence (SEQ ID NO. 23) corresponding assembled sequence of the sequence of the LFV strain 3 isolate of Lumpfish flavivirus.
Figure 12 discloses the DNA sequence (SEQ ID NO. 24) corresponding assembled sequence of the sequence of the LFV strain 4 isolate of Lumpfish flavivirus.
Figure 13 discloses the DNA sequence (SEQ ID NO. 25) corresponding assembled sequence of the sequence of the LFV strain 5 isolate of Lumpfish flavivirus.

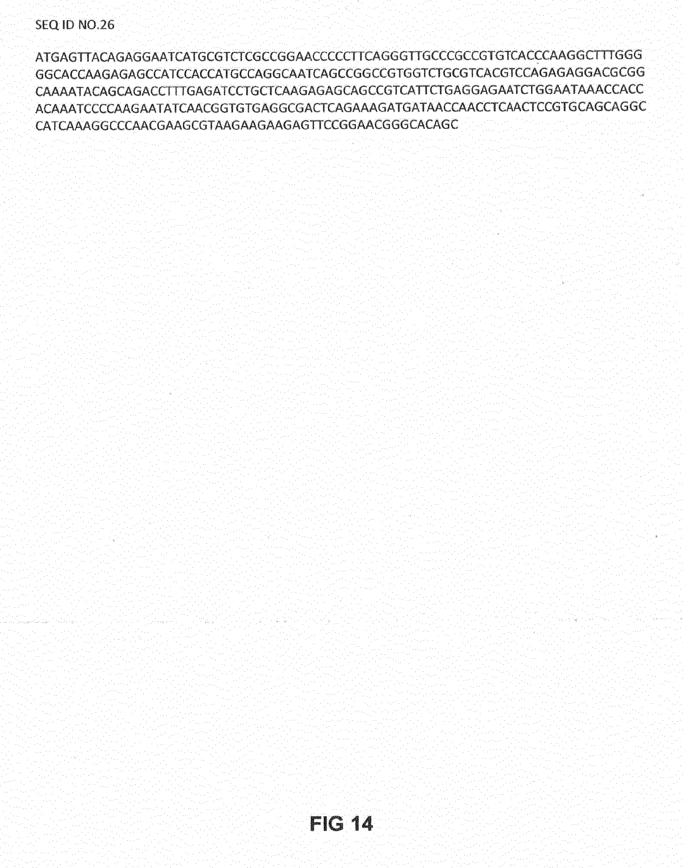

Figure 14 discloses the DNA sequence (SEQ ID NO. 26) corresponding assembled sequence of the sequence of the LFV strain reference of Lumpfish flavivirus.

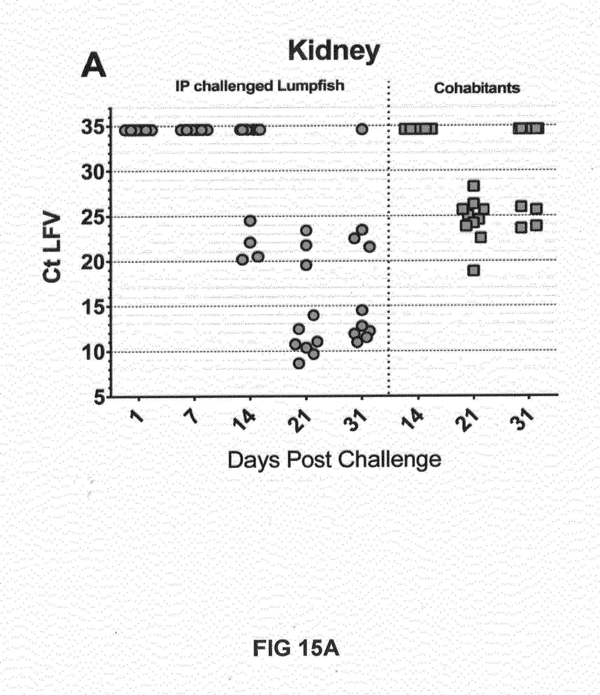

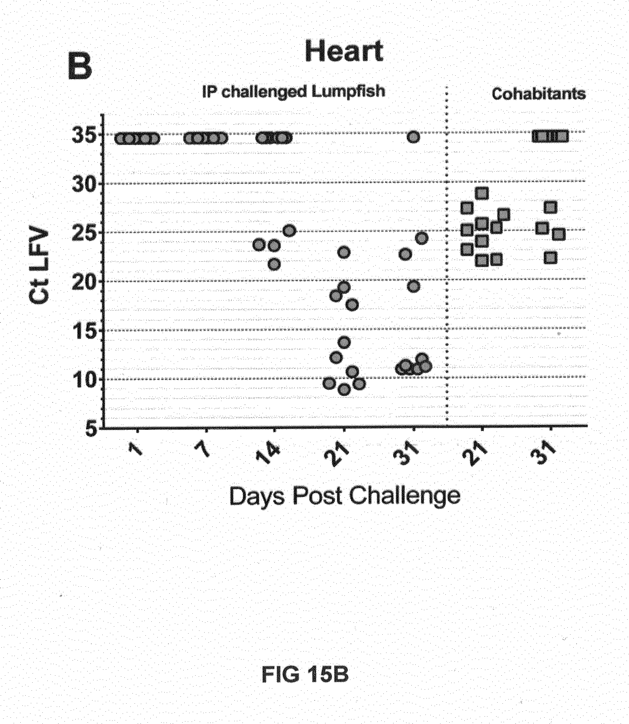

Figure 15A and B graphically discloses the LFV Ct values of Lumpfish tissue samples from (A) Kidney and (B) Heart tissue sampled at 1, 7, 14, 21 and 31 days post challenge. Each icon represents one fish. Ten fish are sampled per time point.

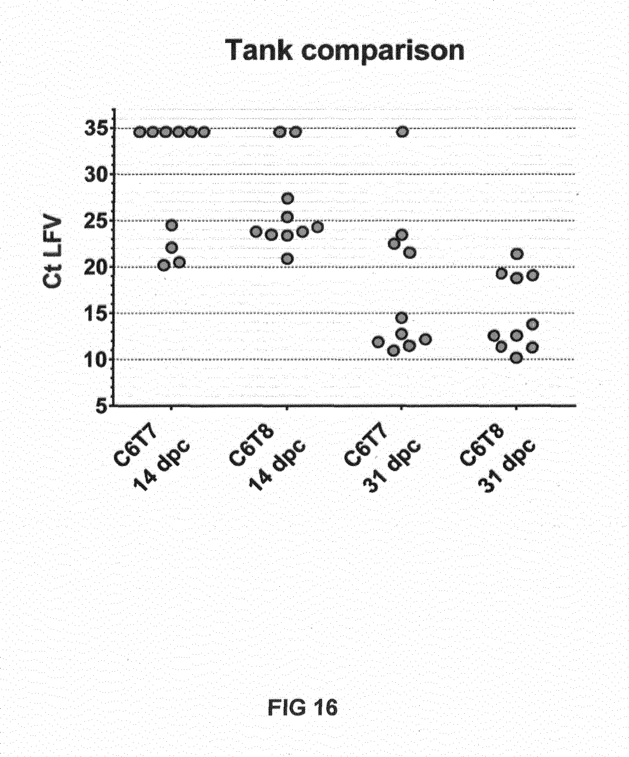

Figure 16 graphically discloses the LFV Ct values of Lumpfish kidney tissue samples sampled at 14 and 31 days post challenge. Each icon represents one fish. Ten fish are sampled per tank per time point.
Figure 17 discloses a photographic image of a histological section of a normal lumpfish liver.
Figure 18 discloses a photographic image of a histological section of a lumpfish liver with extensive tissue damage sampled at 21 days post IP challenge with LFV containing tissue homogenate.
Figure 19 discloses a photographic image of a histological section of a lumpfish liver with extensive tissue damage sampled at 31 days post IP challenge with LFV containing tissue homogenate.
Figure 20 discloses a photographic image of an ethidium bromide stained agarose gel showing the PCR product using the LFV1 forward and reverse primers, the products of which were sequenced and aligned to show variant LFV.
Figure 21 discloses a photographic image of an ethidium bromide stained agarose gel showing the PCR product using the LFV2 forward and reverse primers, the products of which were sequenced and aligned to show variant LFV.
Figure 22 discloses a photographic image of an ethidium bromide stained agarose gel showing the PCR product using the LFV3 forward and reverse primers, the products of which were sequenced and aligned to show variant LFV.
Figure 23 discloses a photographic image of an ethidium bromide stained agarose gel showing the PCR product using the LFV4 forward and reverse primers, the products of which were sequenced and aligned to show variant LFV.
Figure 24 represents a phylogenetic tree constructed from the nucleotide alignments of the five variant LFV strains isolated.
Figure 25 represents individual sequence alignment and consensus sequence of the putative structural proteins of the five variant LFV isolated

### DETAILED DESCRIPTION OF THE INVENTION

The present invention and the various embodiments thereof will now be described in detail in the following.

### Definitions

The present invention relates to isolated nucleic acid sequences and variants thereof which have at least 70% sequence identity to the isolated nucleic acid sequences. The terms "% sequence identity", "% identical", "% identity", "% homology", and similar terms, are to be understood to refer to the percentage of nucleotides that two or more sequences or fragments thereof contain that are the same. A specified percentage of nucleotides can be referred to as having, for example, 70%, 80%, 85%, 90%, 95%, 99% sequence identity or homology over a specified region when compared and aligned for maximum correspondence. Unless otherwise stated, the determination of sequence identity/homology is based on a comparison of the entire sequences, and not on selected portions. The skilled person will acknowledge that various means for comparing sequences are available. For example, one example of a useful computer sequence alignment program useful for determining the percent sequence identity/ homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

The term "fragments or variants thereof" used in respect of the nucleic acid sequences and recombinant proteins according to the present invention or disclosed herein is to be understood to encompass nucleic acid sequences and recombinant proteins that only differ from the isolated sequences SEQ ID NO. 1-26 by way of some amino acid or nucleotide additions, deletions or alteration that have little or no effect on the functional activity of the claimed sequences. The skilled person will acknowledge that modifications of a protein coding nucleotide sequence may be introduced which do not alter the amino acid sequence, e.g. the substitution of a nucleotide resulting in that the triplet affected by the substitution still codes for the same amino acid. Such alterations may, for example, be introduced to adapt the nucleic acid sequence to the codons preferably used by a host cell and thus to enhance the expression of a desired recombinant protein. Furthermore, the addition of nucleic acid sequences coding polypeptides which facilitate purification may be added without affecting the activity of the resulting recombinant protein.

The skilled person will further acknowledge that alterations of the nucleic acid sequence resulting in modifications of the amino acid sequence of the corresponding recombinant protein may have little, if any, effect on e.g. the protein's ability to induce protection against Lumpfish flavivirus infection, if the alteration does not have any impact on the resulting three dimensional structure of the recombinant protein. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a recombinant protein with substantially the same functional activity as the protein having the amino acid sequences as depicted in SEQ ID NO.s 3, 5, and 7, and thus to be expected to constitute a biologically equivalent product. Nucleotide changes which result in alteration of the N-terminal or C-terminal portions of the protein molecule would also not be expected to alter the functional activity of the protein. Each of the proposed modifications is well within the routine skill in the art, as is determination of retention of biological activity of the encoded products. Therefore, where the terms "nucleic acid sequences of the invention" or "recombinant protein of the invention" are used in either the specification or the claims each will be understood to encompass all such modifications and variations which result in the production of a biologically equivalent protein.

Thus, recombinant proteins and fragments thereof which differ in respect of amino acid substitutions, addition or deletions, compared with the recombinant proteins having the sequence of SEQ ID NO. 3, SEQ ID NO. 5, and SEQ ID NO. 7, respectively, but which exert substantially the same functional activity as the proteins having the sequence of SEQ ID NO. 3, SEQ ID NO. 5, and SEQ ID NO. 7.

Terms such as "antigen", "immunogenic protein or peptide", "antigenic determinant", and "epitope", when used herein, are to be understood to refer to a recombinant protein or fragment thereof which is able to induce protection against Lumpfish flavivirus infection in fish or which is able to bind to an antibody which recognises and binds to the Lumpfish flavivirus. A fragment of a recombinant protein having a sequence as depicted in SEQ ID NO. 3, SEQ ID NO. 5, and SEQ ID NO. 7, respectively may constitute such an antigen or epitope. A example of an antigenic fragment is a fragment constituting, for example, 8-11 amino acids of the sequence SEQ ID NO. 3, SEQ ID NO. 5, or SEQ ID NO. 7.

The term "originating from", as used herein, refers to a DNA sequence that was originally isolated from an RNA virus, reverse transcribed and made into double stranded DNA molecules. The DNA sequences of the present invention originated from the RNA genome of the virus of the invention.

The term "vaccine" as used herein refers to a material that can produce an immune response that blocks the infectivity, either partially or fully, of an infectious agent, which in respect of the present invention is the Lumpfish flavivirus affecting fish such as e.g. Lumpfish. Thus, when the vaccines are administered to a fish, the fish is immunised against the disease caused by Lumpfish flavivirus. The immunising component of the vaccine may be e.g. DNA as in a DNA vaccine, a recombinant protein or fragment thereof, a live recombinant microorganism, or an inactivated or attenuated virus.

When used in relation to the virus of the present invention, the term "isolated" refers to the virus when separated from its natural environment, such as the cells and tissues from its natural host. In particular, the term "isolated" refers to a culture, such as a pure culture of the virus derived from a tissue sample from an infected host. As viruses cannot reproduce on their own and use host cell machinery to make both the viral genome and capsids, the term "isolated" may in particular refer to a culture of cells infected with the virus. The term "isolated" may further refer to a virus which is substantially free of other viral or microbial material. The term "substantially free of other viral or microbial material" as used herein refers to a preparation of a virus in which other viral or microbial material cannot be detected using conventional techniques like seeding on TSA or cysteine heart agar spread plates, seeding in cell cultures known to support the growth of fish viruses like Pancreas Disease Virus, Infectious Salmon Anemia virus and Infectious pancreatic Necrosis virus and PCR with primers designed against known sequences from fish pathogens. Further, it is to be understood that when "substantially free of other viral or microbial material" the virus of the invention is in a form wherein it may be used for therapeutic purposes. This definition does not exclude applications in which a pure culture of the virus according to the invention is combined with other vaccine antigens, e.g. other bacterial or viral antigens in compositions, e.g. compositions intended for vaccination purposes.

As outlined below, primers (i.e. oligonucleotide sequences) useful as diagnostic tools are disclosed. The term "primer" as used herein refers to an oligonucleotide produced from a longer sequence (e.g. as a restriction fragment) or produced synthetically, which is significantly complementary to a Lumpfish flavivirus target sequence and thus capable of hybridizing to a nucleic acid sequence of the present invention. The primer may be labelled to facilitate detection, e.g. using fluorescent labels or other label means known to the skilled person.

### Detailed description of the embodiments of the invention

### Lumpfish flavivirus

The present inventors have identified and isolated a new virus, referred to herein as "Lumpfish flavivirus" (LFV). According to specific embodiments of the invention, the virus comprises in its genome a ribonucleic acid sequence, SEQ ID NO. 20 and which, by reverse transcription and two-strand synthesis provides the DNA sequence set forth in SEQ ID NO: 1, or a closely related or variant sequence.

### Nucleic acid sequences

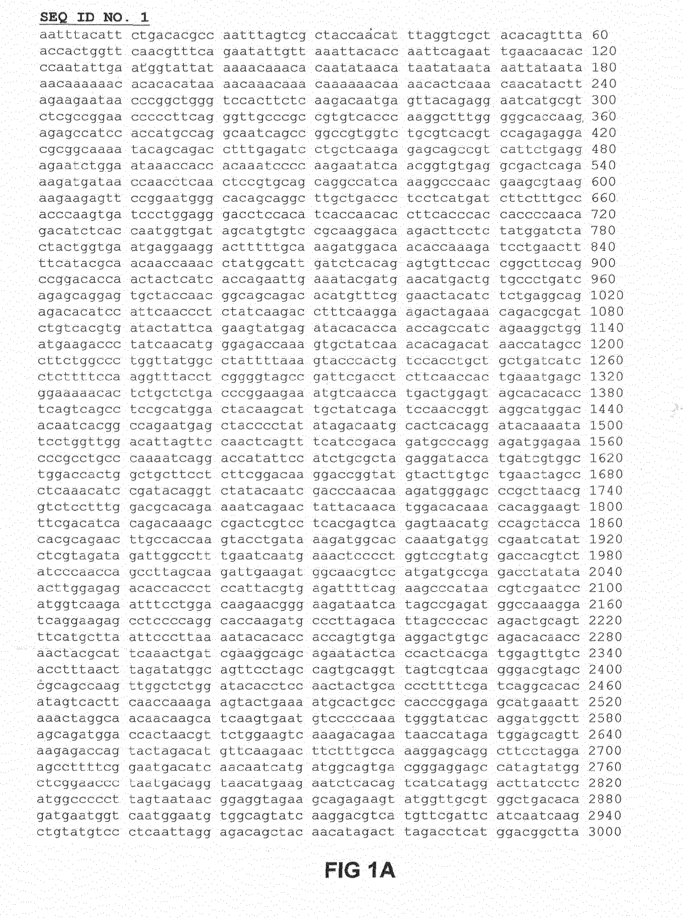

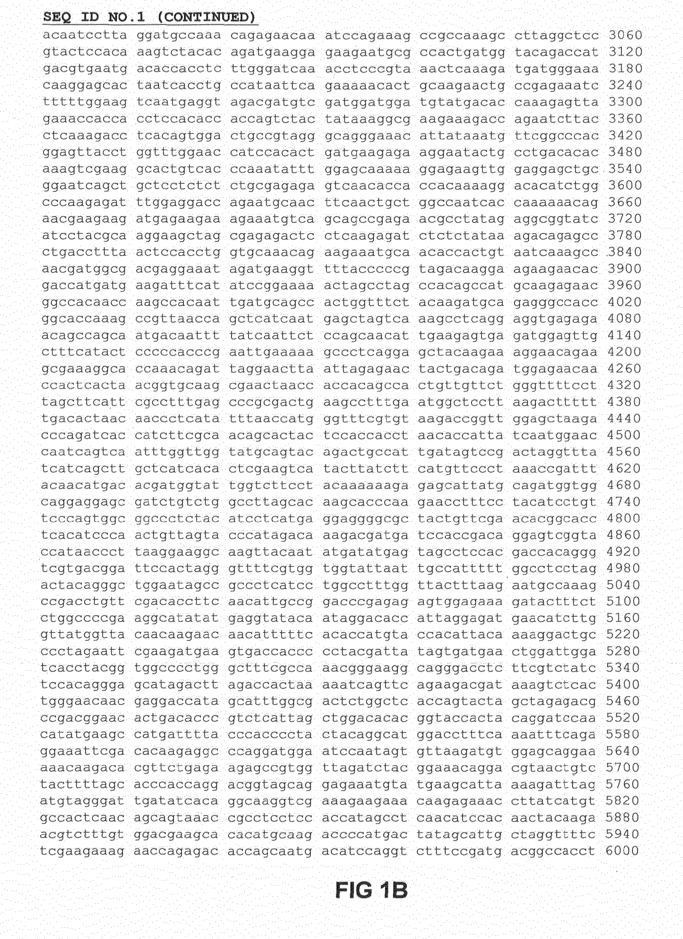

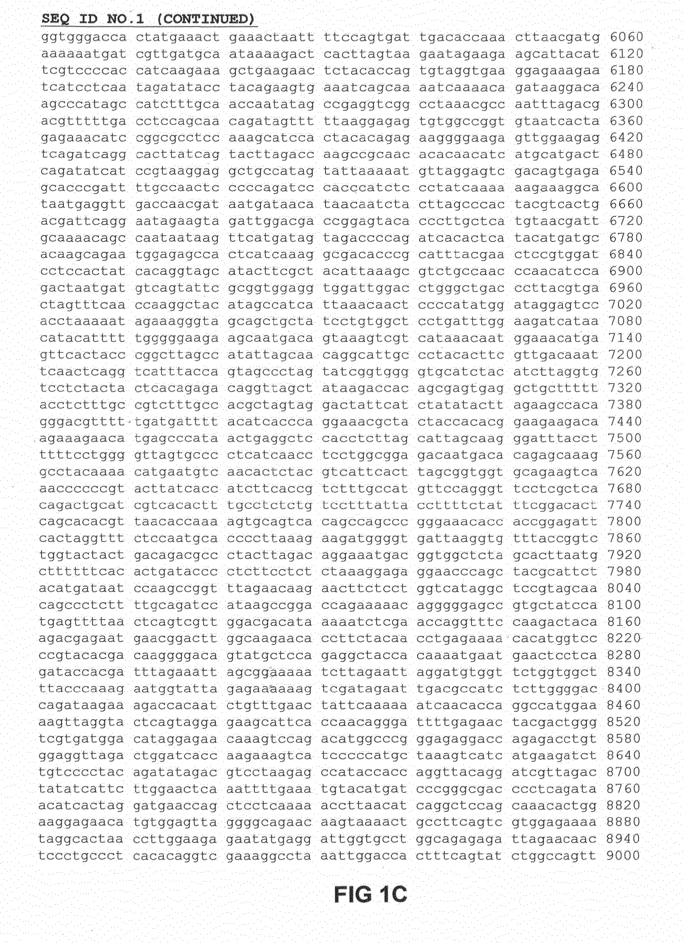

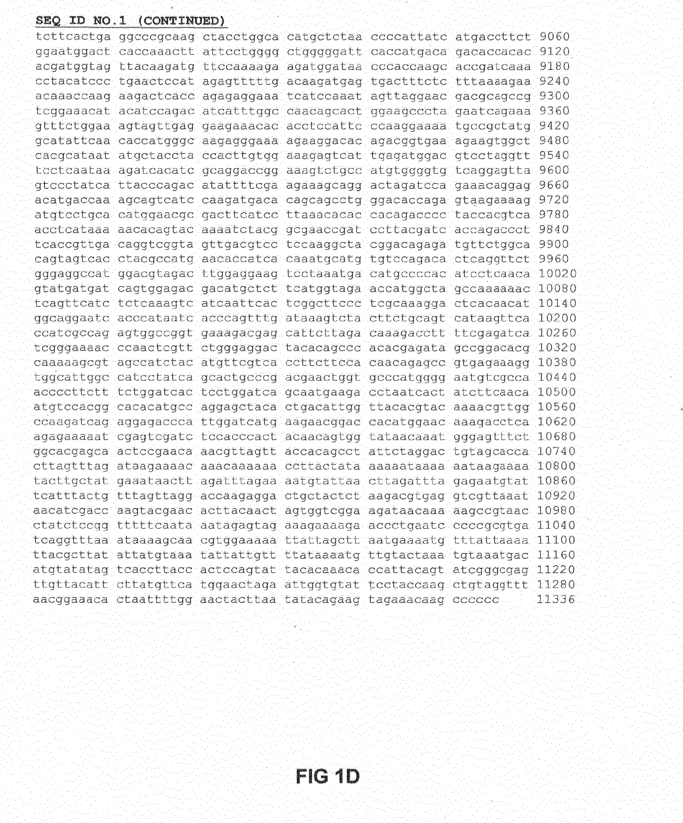

The present invention describes the genomic sequence of the new Lumpfish flavivirus (LFV). Tissue was collected from lumpfish showing clinical signs of disease, with extensive liver necrosis. Tissue was homogenized, pelleted by centrifugation, and the supernatant subjected to DNase and RNase treatment. RNA was then isolated and cDNA synthesised using random primers. Thereafter, double stranded DNA was provided and amplified by PCR using random primers, and the obtained products were sequenced and analysed to obtain part of the genomic sequence. The sequence of the rest of the genome was obtained by PCR with specific primers and 3' and 5' RACE (Rapid Amplification of cDNA Ends), and the DNA sequence corresponding to the full genomic sequence of the virus is given in figure 1 and SEQ ID NO. 1 and further represented as the ribonucleic acid sequence of the RNA genome as set forth in SEQ ID NO.20..

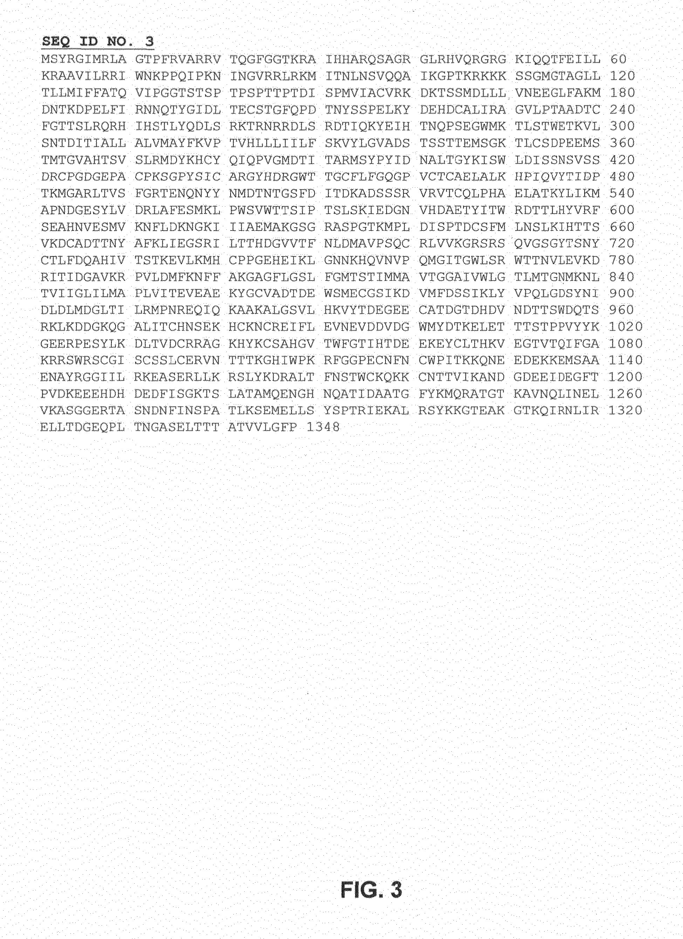

The further analysis of SEQ ID NO. 1 by the present inventors revealed two open reading frames which are believed to include the coding region for several viral proteins. Sequence comparison with other known viral sequences has revealed that the open reading frame 1 (SEQ ID NO. 2) according to the present invention shows very weak homology to other viral sequences. However, certain putative protein motives can be identified, and an organisation corresponding to the common outline of Flaviviridae is suggested. Furthermore, the analysis of the open reading frame 1 (SEQ ID NO. 2) indicates that it comprises a nucleic acid sequence coding an envelope protein (SEQ ID NO. 6). The amino acid sequence of the open reading frame 1 is shown in figure 3 and SEQ ID NO. 3. Said sequence shows only very weak homology with other known proteins.

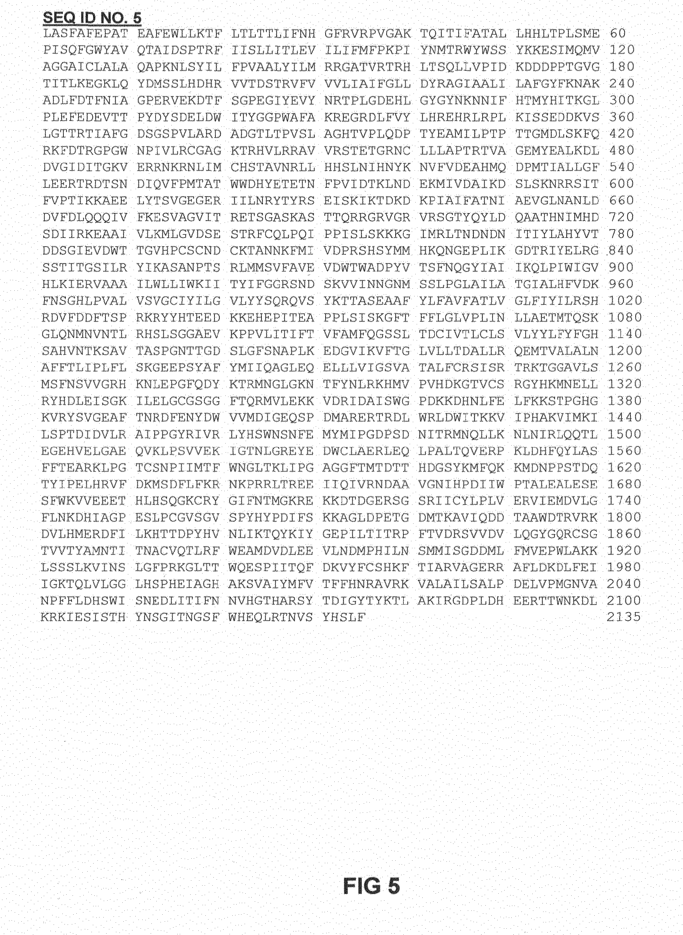

A further isolated nucleic acid sequence of the invention (open reading frame 2, SEQ ID NO. 4) comprises a sequence coding putative proteins and the amino acid sequence thereof is depicted in figure 5 and SEQ ID NO. 5

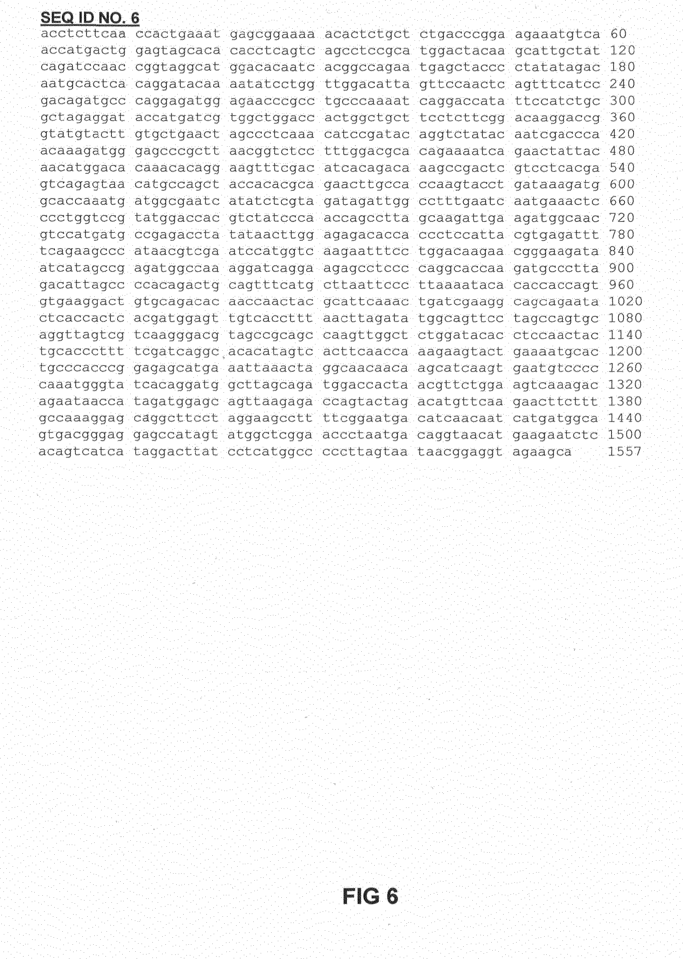

The SEQ ID NO. 6 of the invention comprises a sequence which is also believed to include the coding sequence of a putative envelope protein, the amino acid sequence thereof being depicted in figure 6/SEQ ID NO. 6. Surprisingly, said sequence shows only very weak homology with other viral envelope proteins, and no other members of the Flaviviridae family comprise this particular open reading frame.

The discovery of the Lumpfish flavivirus and the determination and disclosure of the viral nucleotide sequences advantageously facilitates substantial progress in respect of controlling and preventing liver necrosis in fish. For example, the sequences of the open reading frames 1, 2, or the putative envelope protein (SEQ ID NO. 2, SEQ ID NO.4, or SEQ ID NO. 6), may be used in the development of recombinant proteins to be used in vaccines, or the development of DNA vaccines or a live recombinant microorganism.

Recombinant proteins which are coded for by sequences based on the open reading frames 1, 2, or the putative envelope protein (SEQ ID NO. 2, SEQ ID NO.4, or SEQ ID NO. 6), may also be used in the development of diagnostic tools, for example, through the production of antibodies and the development of immunoassays for the detection of the presence of Lumpfish flavivirus in biological samples.

In addition, the sequences provided may be used in the development of diagnostic tools. For example, the nucleic acid sequences or fragments thereof may be used to detect the presence of Lumpfish flavivirus in fish or other biological samples.

Various aspects and embodiments of the invention resulting from the disclosed viral nucleic acid sequences are outlined further below. The skilled person will acknowledge that said embodiments should not be understood to represent a limit on the scope of the uses of the claimed nucleic acid sequences. Other uses of the claimed nucleic acid sequences are also understood to be covered by the scope of the present invention.

### Recombinant proteins

The skilled person is well familiar with the various available biotechnological techniques providing for the expression of isolated nucleic acid sequences for the preparation of recombinant proteins by heterologous expression in various host cell systems using commonly available genetic engineering techniques and recombinant DNA expression systems. Various protocols for the expression of recombinant protein are known, cf. e.g. "Recombinant Gene Expression Protocols, in Methods in Molecular Biology, 1997, Ed. Rocky S Tuan, Human Press (ISSN 1064-3745) or Sambrook et al., Molecular Cloning: A laboratory Manual (third edition), 2001, CSHL Press, (ISBN 978-087969577-4). For example, the nucleic acid sequences of the open reading frames 1 and 2 or fragments or variants thereof may be inserted in suitable expression vectors comprising all the necessary transcriptional and translational regulatory sequences specifically adapted for directing the expression of the desired protein coding nucleic acid sequence in a suitable host cell. Suitable expression vectors are e.g. plasmids, cosmids, viruses or artificial yeast chromosomes (YAC's). A non-limiting list of examples of suitable expression vector systems is e.g. pET-type of vectors (available from Invitrogen), pcDNA3.1 vectors (available from Invitrogen), or the pBR-type, pUC-type or pGEM-type of expression vectors. The obtained expression vector including the open reading frames (SEQ ID NO. 2, SEQ ID NO. 4 or SEQ ID NO. 6) or a fragment thereof is then introduced in suitable host cells for the production of the desired recombinant protein.

According to one embodiment of the invention, a vector is provided comprising a sequence selected from the group consisting of SEQ ID NO. 2, SEQ ID NO. 4 or SEQ ID NO. 6, and variants or fragments thereof which have at least 70 % sequence identity to any of the sequences SEQ ID NO. 2, SEQ ID NO. 4, or SEQ ID NO. 6, operatively linked to control sequences directing the expression of said sequences. The term "operatively linked", means that the desired sequence coding for a recombinant protein is linked to all the necessary transcriptional and translational regulatory sequences specifically adapted for directing the expression of the desired protein coding nucleic acid sequence in a suitable host cell.

It should be understood that various modifications may be introduced in the nucleic sequences of the present invention utilising techniques well known to the skilled person for example to facilitate expression. By the use of site directed mutagenesis, modification may be introduced to adapt the coding sequence to the desired host used to express the sequence and thus produce the recombinant protein. The skilled person is well aware of the fact of the presence of host specific codons, and that the adaption of a heterologous nucleic acid sequence to the host specific codons increase the expression efficiency. Other modification may also be introduced, e.g. to facilitate isolation and purification, i.e. by adding a sequence coding for a peptide or protein useful for such purposes. Also, nucleic acid sequences coding signal peptide providing for secretion of the desired recombinant protein from the host cell may also be linked to the nucleic acid sequences of the present invention. In case the recombinant protein is expressed in a live recombinant microorganism (see below), the nucleic acid sequence may be modified to enable the anchorage of the recombinant protein in the membrane or cell wall of the microorganism resulting in the presentation of the recombinant protein on the surface of the live recombinant microorganism. Accordingly the nucleic acid sequence of the present invention is modified by adding a nucleic acid sequence coding a protein or peptide resulting in the anchorage of the resulting recombinant protein in the membrane or cell wall of the live recombinant microorganism.

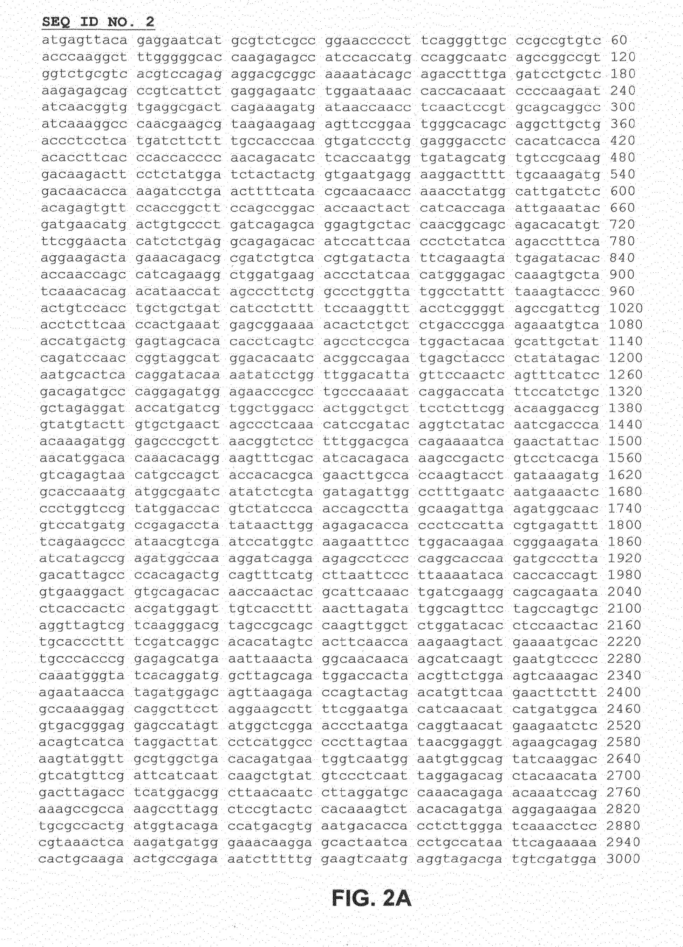

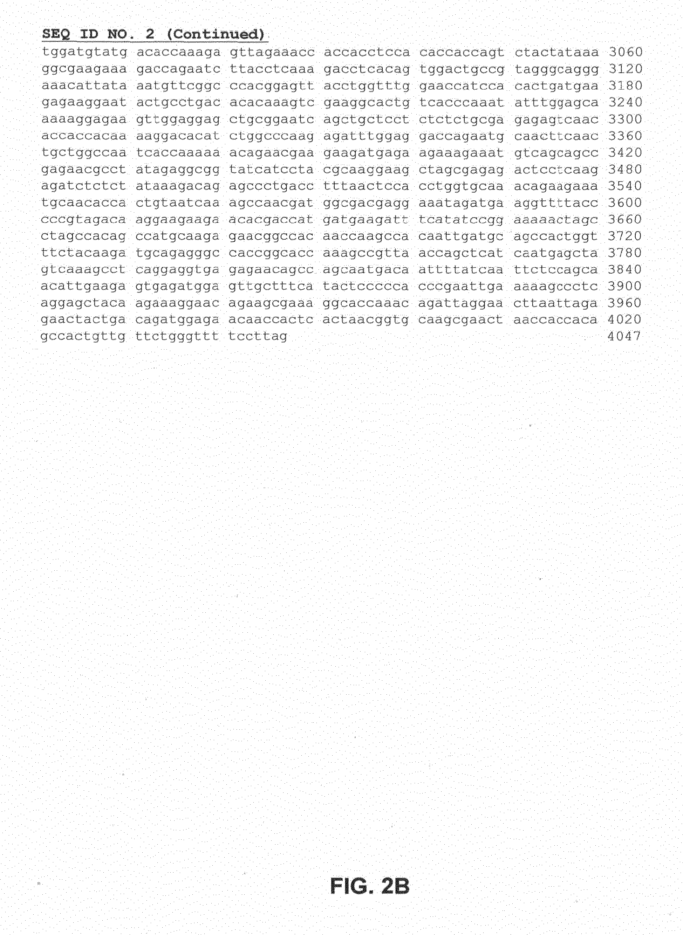

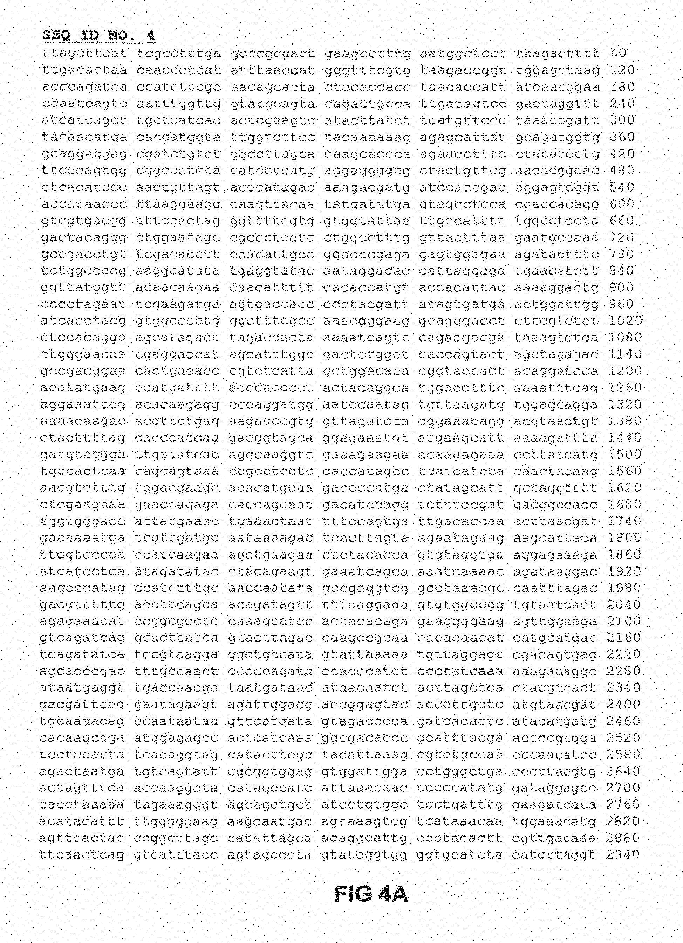

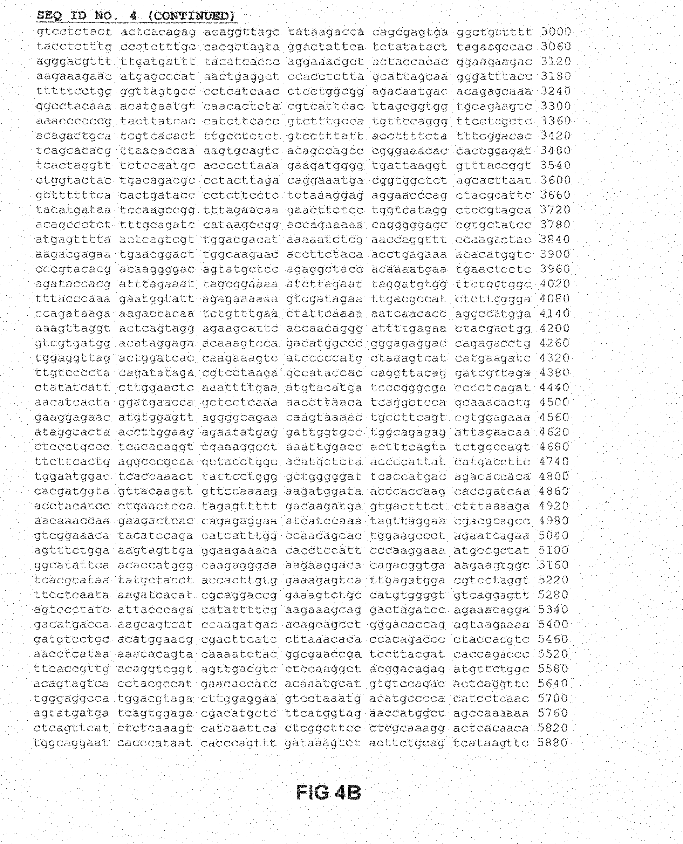

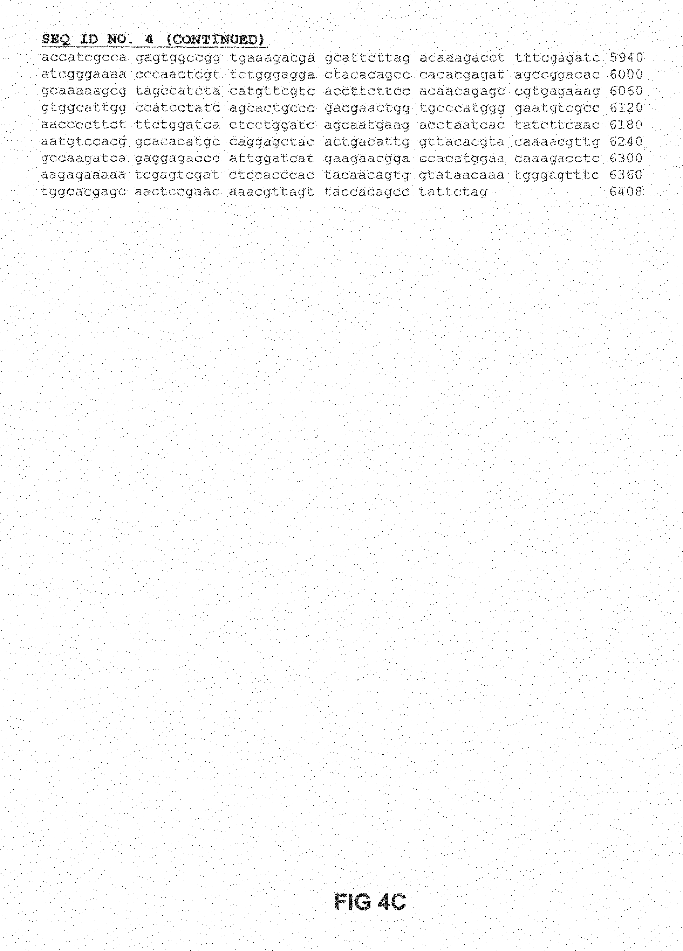

The present invention thus provides vectors comprising the nucleic sequence as depicted in figure 2 and/or 4 and/or 6 (SEQ ID NO. 2, SEQ ID NO. 4 or SEQ ID NO. 6) or variants thereof. The present invention also provides host cells comprising the vectors of the invention.

Various commercially available host cells specifically adapted for the production of recombinant proteins may be used, including both prokaryotic host cells and eukaryotic host cells. Non-limiting examples of suitable prokaryote host cells are *Escherichia Coli, Bacillus substilis, Lactobacilus sp, Caulobacter cresentus, Yersinia ruckeri, or Vibrio anguillarum.* Examples of suitable eukaryote host cells are yeast such as *Saccharomyces cerevisiae,* or *Chinese hamster ovary cells.* Viruses may also be used for the recombinant expression of a desired protein, cf. e.g. alphavirus, adenovirus or baculovirus vector systems (Bac-to-Bac® Baculovirus Expression Systems, Invitrogen, PichiaPink™ Yeast Expression System, Invitrogen).

According to one embodiment of the invention, a pET vector available from Invitrogen and specifically adapted for the expression of recombinant proteins in *Escherichia Coli* is used. The pET vector is an example of a bacterial expression vector and the invention is not limited to one vector system. One of skill in the art will recognize that other bacterial expression vectors may be used in place of a pET vector and yield positive results.

Recombinant protein may also be provided by using cell free expression systems. Recombinant proteins being the results of expression of the open reading frames 1 or 2 or putative envelope protein (SEQ ID NO. 2, 4 or 6) or fragments, variants, or combinations thereof may be used in a vaccine to obtain protection against further Lumpfish flavivirus infections.

### Recombinant vaccine composition

Recombinant proteins prepared by heterologous expression of the nucleic acid sequences according to the present invention may be used as immunogenic component(s) of a recombinant vaccine composition.

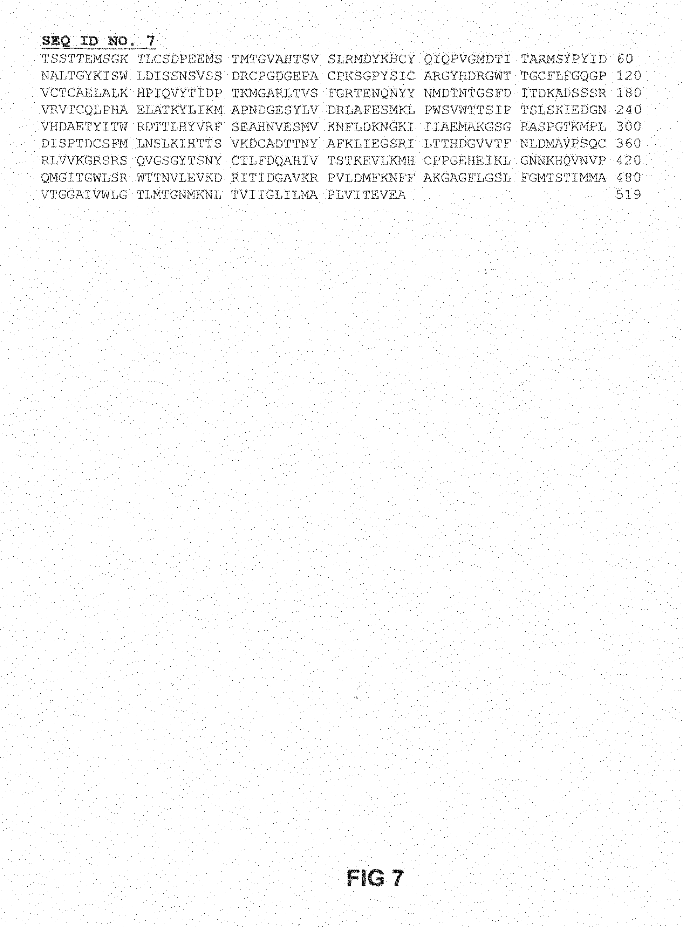

A vaccine composition comprising at least one recombinant protein having an amino acid sequence as depicted in figure 3, 5 or 7 (SEQ ID NO. 3, SEQ ID NO. 5 or SEQ ID NO. 7), or fragments or variants thereof is disclosed herein.

The at least one recombinant protein used in a vaccine consists of or comprises a fragment of the envelope protein coded for by the sequences of the SEQ ID NO. 6 that induce an immune response in the individual being administered the recombinant. Such fragments may constitute an epitope of the envelope protein coded for by SEQ ID NO. 6, is also known as an *antigenic determinant.* The skilled person is well familiar with the fact that the envelope proteins of a virus may comprise one or more epitopes that constitutes the part of the envelope protein that is recognized by the immune system and thus result in the development of protection against further infection. The epitopes may be found as linear, continuous sequence of amino acids, such as e.g. 8-11 amino acids, within an envelope protein or another surface structure of a pathogen organism. Such an epitope is commonly named a linear epitope. An epitope of an envelope protein may also be the results the three dimensional structure of the envelope protein, e.g. formed by the assembling of amino acids of the sequence that are not sequential. Such epitopes are commonly named discontinuous epitope, and are often larger than a linear epitope.

Linear and discontinuous epitopes comprised in the envelope protein may be determined by the use of epitope mapping technology available to the skilled person, c.f. Methods in Molecular Biology: Epitope Mapping Protocols, 1996, vol. 66, Ed. Glenn E Morris, Human Press (ISBN 978-0-89603-375-7). It is to be understood that vaccines comprising recombinant proteins which constitute protecting epitopes of the envelope protein of Lumpfish flavivirus, prepared by the expression of fragments of the putative envelope protein open reading frame (SEQ ID NO. 6), or variants or fragments thereof, are disclosed herein. More specifically, epitopes which are recognized by neutralizing antibodies will be of particular interest.

Epitopes that are found in those parts of the recombinant proteins according to the present invention that are particularly specific for the Lumpfish flavivirus are preferred.

The recombinant vaccine composition comprising recombinant proteins or fragments thereof as outlined above may further comprise an adjuvant. Examples of adjuvants frequently used in fish and shellfish farming are muramyldipeptides, lipopolysaccharides, several glucans and glycans, mineral oil, Montanide™ and Carbopol®. An extensive overview of adjuvants suitable for fish and shellfish vaccines is given in the review paper by Jan Raa, 1996, Reviews in Fisheries Science 4(3): 229-228.

The vaccine may further comprise a suitable pharmaceutical carrier. Preferably the vaccine is formulated as an emulsion of water in oil. The vaccine may also comprise a so-called "vehicle". A vehicle is a device to which the antigen adheres, without being covalently bound to it. Such vehicles include, for example, biodegradable nano/micro-particles or -capsules of PLGA (poly-lactide-co-glycolic acid), alginate or chitosan, liposomes, niosomes, micelles, multiple emulsions and macrosols, all of which are known in the art. A special form of such a vehicle, in which the antigen is partially embedded in the vehicle, is the so-called ISCOM (European patents EP0109942, EP0180564 and EP0242380.

In addition, the vaccine may comprise one or more suitable surface-active compounds or emulsifiers, e.g. Cremophore®, Tween® and Span®. Adjuvants such as interleukin, CpG and glycoproteins may also be used.

It is to be understood that the vaccine may further be in a formulation comprising an antigen from a bacterial source, an antigenic material obtained from a viral source other than the fish virus as described herein, an antigenic material obtained from a parasitical source, and/or an antigenic material obtained from a fungal source. Polyvalent vaccines containing antigens from typical fish pathogens other than Lumpfish flavivirus are well known in the art and are already commercially available. In addition, representative isolates of relevant fish pathogens are available from various sources.

An antigen from a bacterial source is selected from the group consisting of: live, attenuated or killed bacteria of the species *Piscirickettsias sp. Aeromonas sp., Vibrio sp., Listonella sp., Moritella viscosa, Photobacterium damsela, Flavobacterium sp., Yersinia sp., Renibacterium sp., Streptococcus sp., Lactococcus sp., Leuconostoc sp., Bifidobacterium sp., Pediococcus sp., Brevibacterium sp., Edwarsiella sp., Francisella sp., Pseudomonas sp., Cytophaga sp., Nocardia sp., Mycobacerium sp.,* parts or subunits of these bacteria, and any combination hereof.

Isolates of such bacteria are available, e.g. from LGC Promochem/American Type Culture Collection ATCC repository and distribution center (ATCC) including strains of *A. salmonicida* (ATCC 33658), *V. salmonicida* (ATCC 43839), *V. anguillarum* serotype O1 (ATCC 43305) and O2(ATCC 19264), and *Moritella viscosa* (ATCC BAA-105). In addition, cultures of *Piscirickettsias salmonis* have been deposited in the European Collection of Cell Culture (ECACC), Health Protection Agency, Porton Down, Salisbury, Wiltshire (UK), SP4 0JG UK on the 9 June 2006 under the following accession numbers: 06050901, 06050902, 06050903 and 07032110.

Other specific disclosures pertain to a vaccine, wherein said antigenic material obtained from a viral source other than the Lumpfish flavivirus described herein is from a virus selected from the group consisting of: Viral Hemorrhagic Septicemia Virus (VHSV), Viral Hemorrhagic Septicemia Virus (VHSV), Infectious Hematopoietic Necrosis virus (IHNV), Infectious Pancreatic Necrosis Virus (IPNV), Spring Viremia of Carp (SVC), Channel Catfish Virus (CCV), Infectious Salmon Anaemia virus (ISAV), pancreatic disease virus (SPDV), Iridovirus, and heart and skeletal muscle inflammation virus (HSMIV), parts or subunits of any one of these viruses, and combinations hereof. Representative species of such viruses are available to the skilled artisan, for instance from the following deposits: infectious pancreatic necrosis virus (IPNV, ATCC VR-1318, country of origin: unknown), Viral Hemorrhagic Septicemia Virus (VHSV, ATCC VR_1389, country of origin: Denmark); Infectious Hematopoietic Necrosis virus (IHNV, ATCC VR-1392, country of origin: USA)); Pancreatic Necrosis Virus; Spring Viremia of Carp (SVC, ATCC VR-1390, country of origin: Denmark); Channel Catfish Virus (CCV) (ATCC VR-665, country of origin: USA); Infectious Salmon Anaemia (ISA) virus (ATCC VR-1554, country of origin: Canada).

Patent deposits have previously been made by the present applicant of the following viral species: Heart and Skeletal Muscle Infection Virus (HSMIV, patent deposit nr ECACC 04050401, country of origin: Norway).

In more specific disclosures, said antigenic material obtained from a viral source other than the Lumpfish flavivirus as described herein is from the group consisting of: Glycoprotein of Viral Hemorrhagic Septicemia Virus (VHSV), nucleoprotein of Viral Hemorrhagic Septicemia Virus (VHSV), glycoprotein of Infectious Hematopoietic Necrosis virus (IHNV), nucleoprotein structural proteins of Infectious Pancreatic Necrosis Virus (IPNV), G protein of Spring Viremia of Carp (SVC), and a membrane-associated protein, tegumin or capsid protein or glycoprotein, of Channel Catfish Virus (CCV), antigenic fragments of any one of these proteins and combinations thereof.

In other embodiments said antigenic material from a parasitic source is from a source selected from the group consisting of *Lepeophtheirus Sp., Caligus Sp.,* and *Ichthyophthirius Sp,* parts of any one of these parasites, and combinations thereof.

In yet other embodiments said antigenic material is from a fungal source selected from the group consisting of *Saprolegnia Sp., Branchiomyces sanguinis, Branchiomyces demigrans* and *Icthyophonus hoferi.*

The vaccine may in particular be formulated for administration to a fin fish. More specifically the vaccine may be (formulated) for administration to a telostei. The teleostei include, but are not limited to salmonids, lumpfish, wrasse, basses, breams, cods, snappers, flatfish, catfish, yellowtails and tilapias.
Preferably the vaccine is formulated for administration to lumpfish (*Cyclopterus lumpus*)

In further disclosures the vaccine is formulated for administration by a route selected from the group consisting of: Bath, immersion, intraperitoneal injection, intramuscular injection and oral administration.

### Live recombinant microorganism

The sequences of SEQ ID NO. 6, or variants or fragments thereof, may also be used to develop a live recombinant microorganism to be used in a vaccine against Lumpfish flavivirus infection. A live recombinant organism functions as a carrier for genetic information by the insertion of genetic material of the SEQ ID NO. 6, or variants or fragments thereof. Said sequence or variants or fragments thereof may be inserted in the microorganism in such a way that the microorganism is capable of expressing said nucleic acid sequences. The nucleic acid sequences or variants thereof according to the invention may be inserted in the genome of the microorganism or by the introduction of a vector comprising the desired nucleic acid sequences. The live recombinant microorganism may thus be a carrier of nucleic acid sequences coding for the recombinant proteins or variants or fragments thereof according to the present invention. As mentioned above, the live recombinant organism may e.g. carry one or more recombinant proteins on the surface, e.g. wherein said recombinant proteins are anchored in the membrane or the cell wall of the live recombinant organism.

The live recombinant microorganism may be e.g. a virus, a bacteria or a parasite. The live recombinant microorganism may function in the same way as a live fish vaccine, i.e. the microorganism may replicate in the target fish and result in an immunological response against the recombinant proteins coded by the nucleic acids of the invention.

The live recombinant microorganism may have the advantage of being efficiently cultured and thus being optimised for desired fermentation processes and high yield production of vaccine.

The skilled person will be able, by the use of common biotechnological techniques for cloning and constructions of recombinant organisms, to construct a live recombinant microorganism according to the present invention.

The live recombinant microorganism may also be the carrier of nucleic acid coding for other fish pathogens thus providing for protection against a variety of pathogens. The live recombinant organism may thus include nucleic acids coding e.g. coating proteins or fragments thereof of other fish viruses, such as e.g. SAV, IPNV, IHNV or HSMIV.

The live recombinant microorganism is a virus able to replicate in fish, preferably lumpfish. For example, IHNV may be used as live recombinant microorganism, c.f. WO 03/097090 reporting the use of a modified novirhabdovirus to obtain vaccines. The use of live recombinant microorganism as carrier for nucleic acids coding antigens is also suggested for other fish pathogens, e.g. in WO 2007/031572.

A vaccine composition comprising a live recombinant microorganism may further comprise adjuvants, carrier, surface-active compounds or emulsifiers, including as those agents described above in relation to recombinant vaccines. Furthermore, the advantage of combining different vaccine components is equally applicable to the live recombinant vaccine according to the present invention as to recombinant vaccine composition as disclosed above.

### DNA vaccine

A DNA vaccine is disclosed herein comprising nucleic acids according to the present invention.

DNA vaccines are based on the administration of nucleic acids sequence(s) coding an antigen to a patient, wherein the expression of said nucleic acid(s) results in an immune response in said patient. For the purpose of the present invention, the patient is a fish. A DNA vaccine may consist of a naked plasmid DNA comprising a nucleic acid sequence coding the desired antigen of a pathogen operably linked to suitable promoter sequences and other suitable control sequences enabling the expression of said nucleic acid sequence by the cells of the patient after administration of the vaccine. A DNA vaccine may also be carried in a live host microorganism which is involved in delivering the genetic material to the patient.

Various DNA vaccines to combat fish pathogens are known. Heppell et al. reports of the protection against live virus after injection with a DNA vaccine comprising viral haemorrhagic septicaemia virus G and N genes cloned into an expression plasmid and injected into rainbow trout (Fish and Shellfish Immunology, 1998, vol 8, issue 4, pp 271-286). Lorenzen and LaPatra describes a DNA vaccine against a fish rabdhovirus in "DNA vaccines for aquacultured fish", Rev. sci.tech. Off int. Epiz., 2005 (1), 201-213. A DNA vaccine resulting in protective effects is further more reported by Mikalsen et al., 2005, "Protective effects of a DNA vaccine expressing the infectious salmon anaemia virus hemagglutinin esterase in Atlantic salmon", Vaccine, 23:30, pp 4895-4905.

In EP 1 818 406 A1, a DNA expression system that may be used to produce a DNA vaccine that prevents infectious disease in aquatic animals is described, wherein the expression is directed by a cytomegalovirus promoter.

WO 2007/31572 discloses inter alia a DNA vaccine useful to combat SAV infections in salmonid fish.

WO 2009/002376 discloses a DNA vaccine useful to combat viral infection in carp (Spring viremia of carp virus).

EP 2 011 876 regards the development of a DNA fish vaccine useful to combat KOI Herpes disease that affects carps.

Based on the prior art and the common general knowledge referred to above, the skilled person is able to construct a DNA vaccine comprising the SEQ ID NO. 6 of the present invention or fragments or variants thereof utilizing methods commonly used in the field of genetic engineering.

DNA vaccines consisting of naked plasmid comprising the SEQ ID NO. 6 or fragments or variant thereof may be administered to fish by intramuscular injection or particle mediated delivery by gene gun. The latter involves the coating of small gold particles with the DNA vaccine, and the administration is then effected by air-pressured mediated intradermal delivery. Intramuscular injection of a DNA vaccine according to the present invention is preferred as it is more cost effective than the gene gun method, cf. Lorenz and LaPatra, *supra.* Methods for the administration of DNA vaccines to fish is furthermore disclosed in "Intramuscular Injection of DNA vaccines in Fish" by Heppel and Davis in "DNA Vaccines: Methods and Protocols", Methods of Molecular Medicine, 2000, vol. 29 (ISBN 978-0-89603-508-5).

### Inactivated or attenuated vaccine

The isolated Lumpfish flavivirus of the invention may be used in the preparation of a vaccine, for example, in which the virus is in attenuated or inactivated form.

Various methods are known, including chemical or physical means for inactivation of viruses. Chemical inactivation may for instance be carried out by treatment of the virus with enzymes, with formaldehyde, β-propiolactone or ethyleneimine or a derivative thereof, with organic solvent (e.g. 30 halogenated hydrocarbon) and/or detergent, e.g. Triton® or Tween®. Physical inactivation can advantageously be carried out by subjecting the viruses to energy-rich radiation, such as UV light, gamma irradiation or X-rays.

### Antibodies and immunoassays

The recombinant proteins as disclosed herein or isolated virus according to the present invention may be used to obtain antibodies capable of binding a Lumpfish flavivirus.

Another disclosure thus relates to an antiserum or an isolated antibody or antibodies (monoclonal or polyclonal) which selectively binds to a Lumpfish flavivirus or to a component or part of said virus. Antiserum is conventionally obtained for instance by immunising a laboratory animal with the appropriate antigenic determinant. Once the concentration of antibodies in serum from the animal reaches a desired level, the animal is bled. The serum thus obtained should contain antibodies produced in response to the immunogenic stimulus.

Likewise, techniques for the preparation of antibodies are known to the skilled person. The techniques include the traditional hybridoma technology and alternative techniques such as mRNA display, ribosome display, phage display and covalent display.

The isolated antibody may comprise a marker, e.g. a radiolabel, a fluorescent tag, a chemiluminescent label or an enzyme. The antibody may be a polyclonal or monoclonal antibody. Protocols for the labelling of antibodies by coupling of the various markers mentioned above are well known to the skilled person.

For sufficient protection and control of the disease caused by Lumpfish flavivirus, means for efficient diagnosis and detection of Lumpfish flavivirus is needed including immunoassays, useful for the diagnosis and detection of Lumpfish flavivirus in fish. An immunoassay within the meaning of this disclosure is a biochemical test that measures the presence and/or concentration of the Lumpfish flavivirus in a biological sample, such as e.g. serum. An immunoassay thus utilises the specific binding of an antibody to its antigen. Both the presence of antigens (e.g. present on the Lumpfish flavivirus) and the presence of antibodies against the Lumpfish flavivirus may be measured in the biological sample.

The present disclosure further provides an immunoassay for the detection of antibodies specific for Lumpfish flavivirus in a biological sample, comprising (a) contacting the recombinant proteins disclosed herein and a biological sample suspected to comprise antibodies against a Lumpfish flavivirus; and (b) determining whether the one or more antibodies binds to a Lumpfish flavivirus present in the biological sample wherein said binding represents an indication that the sample contains antibodies specific for Lumpfish flavivirus.

An immunoassay for the detection of Lumpfish flavivirus in a biological sample is disclosed, comprising (a) contacting one or more antibodies that specifically recognise and bind to a recombinant protein according to the invention and a biological sample suspected to comprise Lumpfish flavivirus; and (b) determining whether the antibody binds to Lumpfish flavivirus present in the biological sample wherein said binding represent an indication that the sample contains a Lumpfish flavivirus.

The complex formed in the immunoassays, e.g. the binding of an antibody to a Lumpfish flavivirus present in a biological sample or the binding of an antibody present in a biological sample to a recombinant protein or fragment thereof, may be determined by various means known to the skilled person such as, for example, densitometry, fluorimetry, or colorimetry.

The immunoassays as described above may be a standard Enzyme-linked immunosorbent assay (ELISA). The skilled person will be able to provide ELISA protocols using antibodies developed against the the recombinant proteins. Reference is in this respect made to the ELISA Guidebook, Methods in Molecular biology, vol. 149, ed. By John R Crowther, Humana Press, 2001.

A diagnostic kit for carrying out the immunoassays is disclosed. The kit comprises antibodies recognizing a recombinant protein as disclosed herein and usual means for carrying out an immunoassay, such as e.g. an ELISA plate, and a reagent capable of producing a detectable colour change upon the binding of the antibodies to a Lumpfish flavivirus in a biological sample to be tested. Also a kit is disclosed comprising one or more recombinant proteins according to the invention and usual means for carrying out an immunoassay, such as e.g. an ELISA plate, and a reagent capable of producing a detectable colour change upon the binding of said recombinant proteins to antibodies in a biological sample to be tested.

### Primer sequences and method of detection of Lumpfish flavivirus infection

The nucleic acid sequences of the present invention may be used to detect the presence of Lumpfish flavivirus in fish. The disclosure therefore also relates to a diagnostic method wherein primers specifically hybridize to a nucleic acid in a test sample that is similar to the nucleic acid sequences of the invention. Such tests may take the form of a PCR test or a hybridization test without amplification.

The detection of nucleic acids present in a biological sample is widely applied in both human and veterinary diagnosis, wherein nucleic acids from e.g. pathogens present in biological samples are isolated and hybridized to one or more primers. The one or more primers to be used in hybridization based detection methods is constructed so that they are able to specifically bind to a nucleic acid sequence if present in the sample to be tested. The primers may be linked to a signalling unit or probe, such as e.g. a radiolabel, a luminescent molecule, or a fluorescent molecule, enabling the visualisation of the binding of the primers to a target sequence.

A primer as used herein refers to a nucleic acid sequence, which can be used as a probe in a method of detection of the presence of Lumpfish flavivirus in fish. The primers disclosed herein consist of at least 10, preferably at least 15, and more preferably at least 20 nucleotides that are able to hybridize to nucleic acid sequences isolated from a Lumpfish flavivirus.

The primer is able to hybridize to another nucleic acid molecule, such as genomic RNA originating from Lumpfish flavivirus or cDNA prepared from said genomic RNA, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a primer to target nucleic acids depends on inter alia the length of the primer and the degree of complementation, variables well known to the skilled person. For example, high stringency conditions may comprise hybridization in a buffer consisting of or comprising 6 X SSC, 50 mM Tris-HCL (pH=7.5), 1 mM EDTA, 0.02% PVP, 0.02% Ficoll, 0.02% BSA and 100 µg/ml denatured salmon sperm DNA, for 48 hours at 65°C, washing in a buffer consisting of 2 X SSC, 25 0.01% PVP, 0.01% Ficoll, 0.01% BSA for 45 minutes at 37°C, and washing in a buffer consisting of 0.1 X SSC, for 45 minutes at 50°C. A minimum length of a hybridisable primer is usually at least about 10 nucleotides, preferably about 15 nucleotides and more preferably about 20 nucleotides.

Various protocols for the detection of nucleic acid sequences by PCR are available to the skilled person; cf. e.g. Methods in Molecular Biology, vol. 226, PCR protocols, second ed., Ed. Barlett and Stirling, Humana Press, 2003. The skilled person will furthermore, with the knowledge of the genomic Lumpfish flavivirus sequence depicted in figure 1 (SEQ ID NO. 1) be able to provide suitable primers for the purpose of using said primers in a method for detection of the presence of Lumpfish flavivirus according to the present invention. Reference is in this respect made to the textbook PCR Primer Design, vol. 402 of the series Methods in Molecular Biology, Ed. A. Yuryev, 2007, Humana Press.

The following primers are disclosed:

**Table 1**

| |
|---|
| 5'- CCTCCATTCCCAAGGAAAAT-'3 (SEQ ID NO. 8) |
| 5'- GGTCCTGCGATGTGATCTTT -'3 (SEQ ID NO. 9) |
| 5'- GAGAACAGCCAGCAATGACA -'3 (SEQ ID NO. 10) |
| 5'- TTCGAGTGTGATGAGCAAGC -'3 (SEQ ID NO.11) |

### EXAMPLES

### Example 1

### Identification of viral sequences.

Lumpfish in a culture facility were showing signs of disease and dying. Samples were collected for histopathological analysis. Several organs were collected, and the specific histopathological change that was observed was necrosis in the liver.

Livers from the diseased fish were homogenized, centrifuged at 3000xg and passed through a 0.22 µm filter. The homogenate was digested with approximately 250U DNase l/ml and 5µg RNase A/ml in RDD-buffer (included with Qiagen DNase I) for 1h at 37°C.

RNA was prepared from the sample using QIAamp viral kit (Qiagen) according to the manufacturer's instructions. Single stranded cDNA was synthesized using SuperScript® III reverse transcriptase kit (Invitrogen) and random primers with a fixed 5' overhang (FR26RVN primer 5' GCC GGA GCT CTG CAG ATA TCN NNN NN 3'; Djikeng et al., BMC Genomics 2008, 9:5). The RNA was incubated at 95°C prior to cDNA synthesis to avoid problems caused by a possible secondary structure of the RNA. After synthesis the RNA strand was digested using RNase H before double stranded DNA was made using a combination of the 2^{nd} strand buffer components of the Universal RiboClone® cDNA Synthesis System (Promega) and Exo-Klenow polymerase and including additional supply of the random primers. A single primer PCR reaction using primer against the random primer fixed overhang (FR20RV 5' GCC GGA GCT CTG CAG ATA TC 3'; Djikeng et al., BMC Genomics 2008, 9:5) and standard *Taq* polymerase (Invitrogen) was performed (initial denature of cDNA 95°C for 5 minutes, 40 cycles of 94°C for 30 seconds, primer annealing 60°C for 15 seconds and synthesis 72°C for 1 minute and then final synthesis 72°C for 10 minutes), and the resulting products were separated by 1% agarose gel electrophoresis. PCR products in the size range 500 - 1000 bp were excised from the gel in one piece, purified and cloned using the TOPO-TA cloning system (Invitrogen). Approximately 300 clones were sequenced and subjected to analysis.

Sequences were subjected to analysis in the ContigExpress program of Vector NTI software, resulting in identification of different sequences. The complete genome sequence was obtained by PCR amplification of genome sequence located between the initially obtained sequences, as well as 3' and 5' RACE using standard procedures.

Only weak homology with known virus sequences was detected. Finally, a genome of 10779 bp was assembled (as set forth in SEQ ID NO.1), containing two open reading frames. This is the first time a putative member of the *Flaviviridae* is identified in a teleost.

The analysis of SEQ ID NO. 1, as described, revealed two open reading frames which are believed to include the coding region for several viral proteins. Sequence comparison with other known viral sequences revealed that the open reading frame 1 (SEQ ID NO. 2) according to the present invention shows very weak homology to other viral sequences. However, certain putative protein motives can be identified, and an organisation corresponding to the common outline of Flaviviridae is suggested. Furthermore, the analysis of the open reading frame 1 (SEQ ID NO. 2) indicates that it comprises a nucleic acid sequence coding an envelope protein (SEQ ID NO. 6).

### Example 2

### Expression and use of recombinant proteins

Gene sequences from SEQ ID NO. 6 were amplified by PCR and cloned into a pET vector system using standard methods readily known by one of skill in the art. An *E.coli* host cell was transformed, and recombinant protein expressed. Recombinant protein was purified using standard techniques. Additionally, SEQ ID NO. 6 was amplified by PCR and cloned into a eukaryotic vector. A eukaryotic host cell was transformed and recombinant protein expressed.

Recombinant proteins, as described herein, were formulated into a suitable formulation, preferably but not limited to, a water-in-oil emulsions and will be used as a formulation to produce an immune response in a lumpfish. Any variants related to SEQ ID 6. that can be construed as having conservative amino acid substitutions are included in this description. Additionally, any open reading frames expressing proteins from a lumpfish flavivirus, or any variant of a lumpfish flavirus having about 70% sequence identity to the open reading frames described herein, may be used as part of a formulation to produce and immune response. Additionally, rabbits and mice will be immunized with recombinant proteins cloned and expressed, as described herein. The antibody responses against the proteins will be assessed at various time points. Serum from the immunized animals will be tested in virus neutralization assays and used for additional *in vitro* assays.

### Example 3

### Vaccination and challenge of lumpfish with a recombinant lumpfish flavivirus protein

The effect of a vaccine based on the recombinant putative envelope protein (SEQ ID NO.6) from Lumpfish flavivirus will be assessed.

Lumpfish will be vaccinated by intraperitoneal injection with a vaccine comprising recombinant putative Lumpfish flavivirus envelope protein, SEQ ID NO.6, or variants thereof. After an immunization period, vaccine efficacy will be tested through i.p. challenge of vaccinated and unvaccinated lumpfish with Lumpfish flavivirus (LFV). Tissue samples from vaccinated and unvaccinated fish will be collected for histopathological evaluation. Samples will also analyzed for the presence of virus by PCR-screening and other methods.

Mortality will be registered daily and tissue samples will be collected at different time points from the vaccinated and unvaccinated groups for histopathological evaluation, measurement of antibody response and PCR-screening for the presence of virus.

### Example 4

### DNA vaccine protecting against infection with Lumpfish flavivirus

SEQ ID NO. 6 and parts, fragments, and variants thereof, will be cloned into a vaccine vector suitable to be used as part of a DNA vaccine. The resulting vector will be injected intramuscularly into lumpfish. After an immunization period, vaccine efficacy will be tested through i.p. challenge of vaccinated and unvaccinated lumpfish with Lumpfish flavivirus. Tissue samples from vaccinated and unvaccinated fish will be collected for histopathological evaluation. Samples will also be analyzed for the presence of virus by PCR-screening and other methods.

### Example 4

### Testing the infectivity of LFV

The aim of this study was to reproduce disease in Lumpfish by experimental infection to investigate if Lumpfish virus (LFV) could be transmitted horizontally by infected Lumpfish to naïve Lumpfish by cohabitation, and to investigate whether the virus could be transmitted to Atlantic salmon by cohabiting with infected Lumpfish.

### Study design

This was a comparative, randomized, double tank laboratory study. The study period lasted 4 weeks. The fish were acclimatized to the conditions to be used in the study prior to start. Below is a table of characteristics of the fish (Table 2).

**Table 2**

| **Species** | **Lumpfish (*Cyclopterus lumpus*)** | **Atlantic salmon (*Salmo salar*)** |
|---|---|---|
| Strain | Fjordforsk AS | Stofnfiskur Iceland |
| Origin | Fjordforsk AS | Egghouse Vogavik, Iceland |
| Batch no. | - | ILAB_15_500 |
| Number of fish | 160 (incl. 10 fish for baseline | 30 (incl. 10 fish for baseline |
| Size | 16.8 grams in average at challenge | 94.3 grams in average at challenge |
| Vaccination status | Unvaccinated | Unvaccinated |
| Disease history | None | None |

The study fish were unvaccinated and were free from disease. The Atlantic salmon were already smoltified by ILAB, in order to be ready for cohabiting with infected Lumpfish. Head kidney samples from 15 Lumpfish were screened for the presence of atypical *A*. *salmonicida, Pasteurella* sp. and Lumpfish Flavivirus upon introduction to the facility. All samples were negative for all the investigated disease agents.

The fish were acclimatized for minimum 1 week to the conditions to be used in the study period (12 °C, 34 % salinity sea water, 12:12 light:darkness). The fish were kept in two 150 L tanks during the challenge period. Environmental parameters during the study period are described in Table 3

**Table 3**

| **Week** | **Salinity** | **Temp. °C** | **O₂ (%)** | **Tank size/ density** | **Photoperiod (L : D)** |
|---|---|---|---|---|---|
| 41-45 | 34 ‰ | 12±1°C | 70-100 | 150 L | 12:12 |

### Challenge

### Challenge isolate

The challenge material ALV 1224 consisted of tissue homogenate from Lumpfish originating from a field outbreak of LFV. Histopathological examination revealed changes dominated by acute and chronic inflammation in the liver, necrosis and loss of normal tissue structure of the kidney, loss of normal tissue structure in the spleen, and changes in the heart. The fish were strongly positive for LFV in all investigated organs (gills, brain, heart, skin, liver, spleen, kidney, intestines) with Ct values averaging 10-14. The liver, spleen and kidney were aseptically extracted from the infected fish. Liver from nine fish were pooled, and spleen plus kidney from the same nine fish were pooled, homogenized in L-15 medium with gentamicin, cell debris removed by centrifugation, and the supernatant filtered through a 0.45 µm filter. The resulting challenge material was strongly positive for LFV, with Ct-values of 17 and 18 from liver and head-kidney/spleen respectively. However, when RNAse treating the samples, the Ct values increased to 30 and 23 respectively, indicating that the majority of intact (and therefore probably infectious) viral particles can be found in the kidney/spleen rather than the liver.

### Challenge procedure

The Lumpfish were marked by VIE-tagging at the time of marking, according to Table 4. 3x1 ml ampulles of spleen/kidney and liver homogenate each was thawed on ice, and pooled prior to challenge. Disposable syringes were used to infect 50 Lumpfish by IP injection of 0.05 ml challenge material to each tank. To C6T7, 50 uninfected Lumpfish were added to be infected by cohabitation, while 20 uninfected Atlantic salmon were added to C6T8. These were to be infected by cohabitation.

**Table 4**

| Challenge study design | | | | | |
|---|---|---|---|---|---|
| **Species** | **Marking** | **Challenge mode** | **Cell** | **Tank** | **No. of fish** |
| Lumpfish | 2x Orange | IP | C6 | 7 | 50 |
| Lumpfish | 2x Yellow* | Cohabitation | C6 | 7 | 50 |
| Lumpfish | 2x Red | IP | C6 | 8 | 50 |
| Atlantic salmon | - | Cohabitation | C6 | 8 | 20 |
| | | | | **Total** | **170** |

### Collection of tissue samples for Reverse Transcriptase quantitative PCR

The fish were euthanized prior to collection of tissue samples. Kidney tissue was collected on RNAlater from minimum ten (10) unvaccinated fish prior to challenge. The collected kidney tissue was screened by Reverse Transcriptase quantitative-PCR (RT-qPCR) to confirm absence of Lumpfish Flavivirus in the test population prior to vaccination. Liver, heart, gill and kidney tissue was collected on RNAlater from 10 Lumpfish from C6T8 the day after challenge, in order to determine the quantity of LFV in the respective tissues as a consequence of injection of the challenge material. Also, liver, heart, gill and kidney tissue samples was collected on RNAlater from 10 fish per group at one, two, three and four weeks post challenge for C6T7, and at two and four weeks post challenge for C6T8. The samples were analysed.

### Collection of tissue samples for histopathology

Tissue samples (liver, kidney, spleen and heart) were collected from ten (10) Lumpfish and Atlantic salmon on formalin for histopathological evaluation prior to challenge. Also, tissue samples were collected from ten (10) fish per group at one, two, three and four weeks post challenge for C6T7, and at two and four weeks post challenge for C6T8. Histopathological changes developing after challenge were investigated for a selection of groups and time points after evaluating the results obtained by RT-qPCR:
- Baseline samples of ten Lumpfish prior to challenge
- ten IP injected Lumpfish from C6T7 at 21 dpc
- ten IP injected Lumpfish from C6T7 at 31 dpc

The scoring was performed blind by a third-party assessor in order to evaluate the degree of histopathological changes developing in the respective tissue as a consequence of infection with LFV.

### Results- Prevalence of LFV in Fish Post Challenge

### IP injected Lumpfish

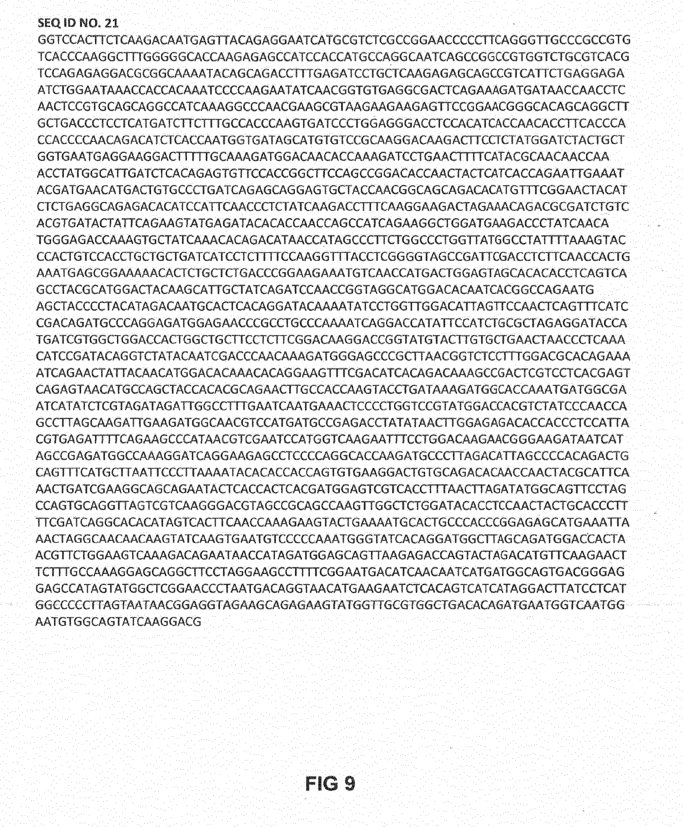

The prevalence of LFV in IP injected Lumpfish increased throughout the study. The results are shown in Figure 9A and 9B. No fish were positive for LFV at introduction to the study facility, one day post challenge (dpc) or 7 dpc. However, at 14 dpc, 4/10 fish were positive for LFV in kidney, heart and gills, and 5/10 fish were positive for LFV in liver. At 21 dpc 10/10 fish were positive for LFV in kidney and heart. At 31 dpc, 9/10 fish were positive for LFV in kidney, heart and liver.

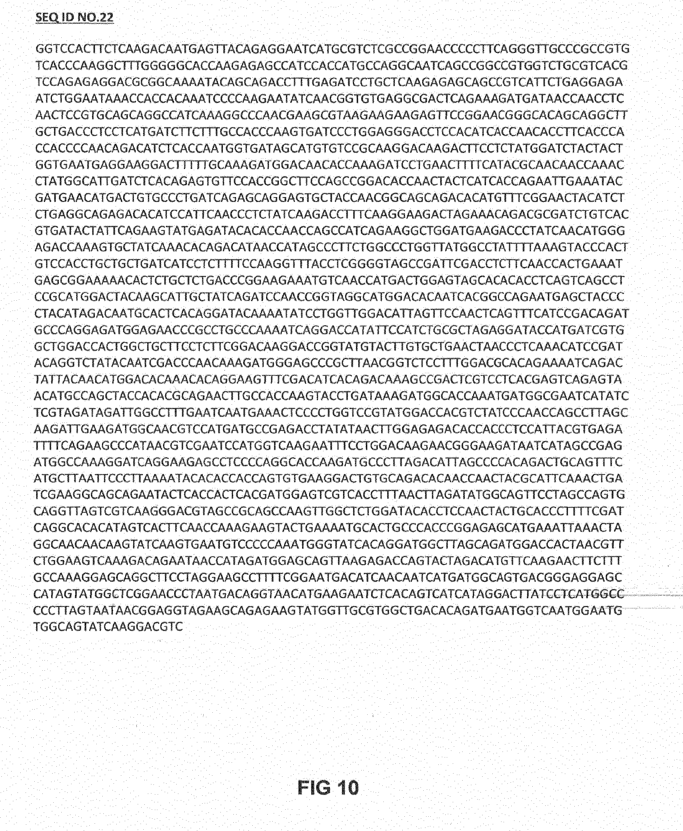

The quantity of LFV in IP-infected Lumpfish increased from 14 dpc to 21 dpc. However, there was no apparent change in the quantity of LFV from 21 to 31 dpc. The quantity of LFV in kidney samples was investigated from 10 Lumpfish from C6T8 at 14 dpc and at termination at 31 dpc, in order to investigate if there were any relevant differences between the two tanks. As can be seen in Figure 10, there is a slightly higher prevalence of LFV-positive Lumpfish at 14 dpc in C6T8 compared to C6T7 (8/10 compared to 4/10). The Lumpfish in C6T8 have been cohabiting with Atlantic salmon, with expected higher stress levels for these fish compared to C6T7. This might be the reason for a higher prevalence. At 31 dpc, there were no relevant differences between the two challenge tanks with regards to the prevalence of LFV-positive Lumpfish (9/10 vs 10/10).

### Cohabiting Lumpfish

None of the Lumpfish cohabitants were positive for LFV (kidney) when investigated at 14 dpc, Figure 9. However, 10/10 Lumpfish were positive for LFV (kidney and heart) when investigated at 21 dpc showing that LFV can be horizontally transmitted from IP injected Lumpfish to naïve Lumpfish. At 31 dpc, the prevalence of LFV positive fish had decreased to 4/10 fish (kidney and heart).

### Cohabiting Atlantic salmon

Atlantic salmon were investigated for the presence of LFV in kidney, in order to investigate whether LFV could be horizontally transmitted from IP infected Lumpfish. At 14 dpi, 0/10 Atlantic salmon were positive for LFV by PCR. 1/9 Atlantic salmon sampled at 31 dpc was positive for LFV in the kidney. However, as this sample was negative when re-analyzed, and gill, liver and heart samples from the same fish were all negative, it is apparent that this was a false positive sample.

### Histopathological changes developing post challenge with LFV

The histopathological changes developing after IP infection with LFV-containing tissue homogenate was investigated at 21 and 31 dpc (Table 5 below). Kidney, heart, liver and spleen were investigated. The most interesting findings were in the liver. 4/10 fish exhibited severe histopathological changes of the liver, dominated by diffuse necrosis at 21 dpc. This is characteristic for what is observed in field outbreaks of LFV. In addition, 2/10 fish exhibited mild changes, while 4/10 were negative. Only minor histpathological changes were detected in the kidney and heart at 21 dpc. While the spleen showed some histopathology, this might
not be related to the disease, as the spleen is a difficult organ to evaluate. Investigation of 10 Lumpfish prior to challenge showed the absence of necrotic or inflammatory changes in the liver prior to infection with LFV. However, minor changes were observed in the spleen and kidney, which might be related to the sampling procedure or other factors not related to LFV.

At 31 dpc, similar histopathological changes were observed as at 21 dpc. Severe necrosis of the liver was observed in 6/10 fish IP injected Lumpfish. Also, inflammatory changes were observed, and infiltration of connective tissue. No significant changes were detected for the Lumpfish infected by cohabitation sampled at 31 dpc.

The relationship between the Ct value of LFV in kidney tissue and the histopathological changes observed in the different organs was investigated by non-parametric Spearman correlation. A strong correlation was observed between histopathological changes in liver and the LFV tissue burden in kidney at 21 dpc (Spearman r -0.9342, p<0.0001) and 31 dpc (-0.8528, p-value <0.0001). For the other tissues, no significant correlation was observed between LFV kidney burden and the histopathological changes in the respective organs.

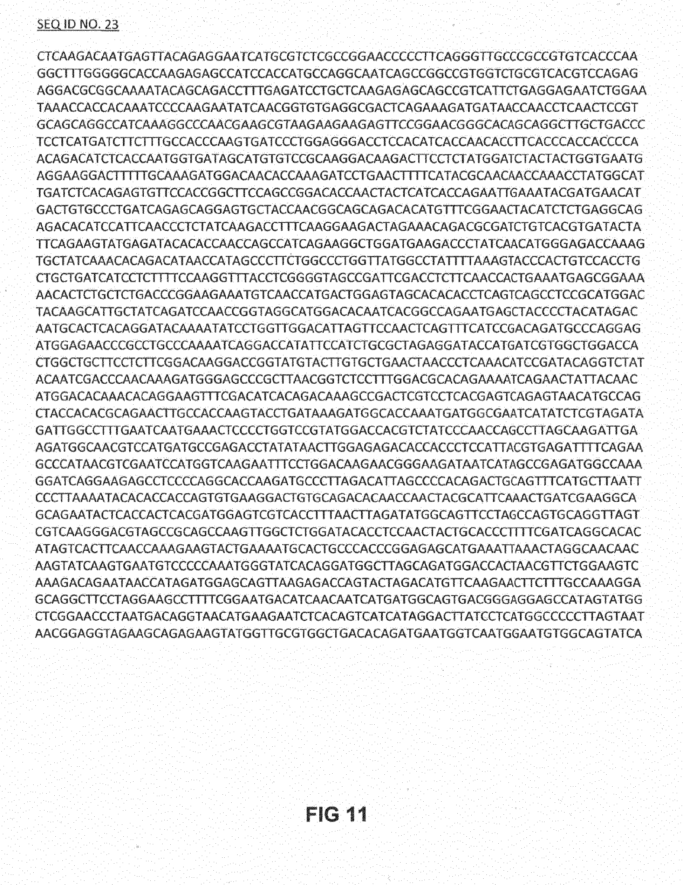

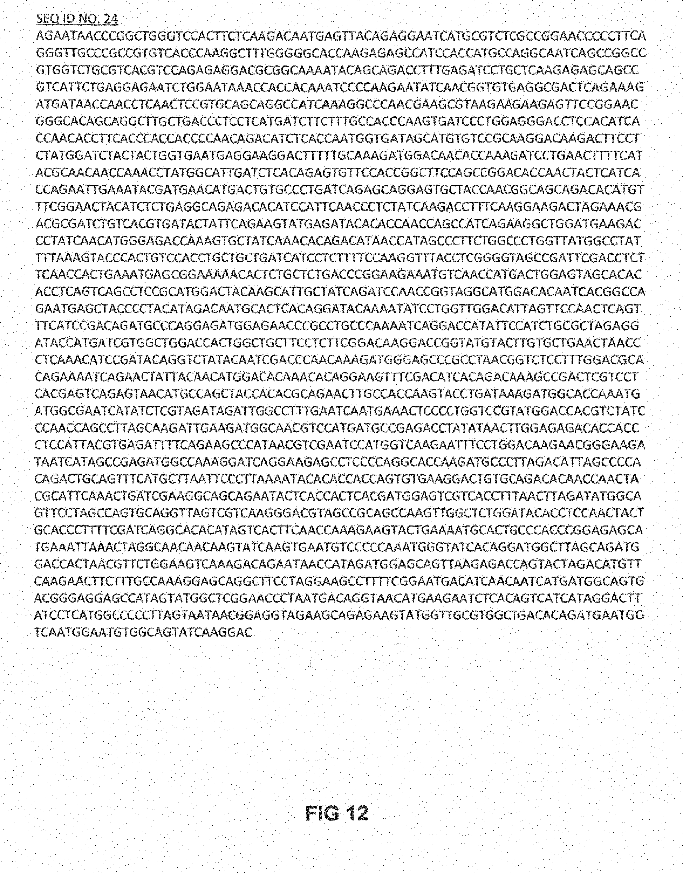

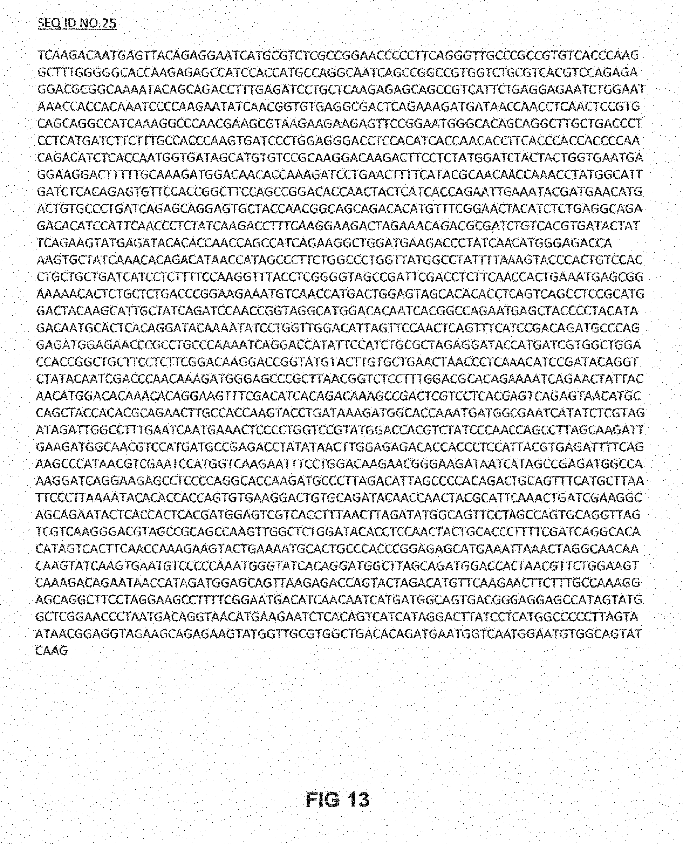

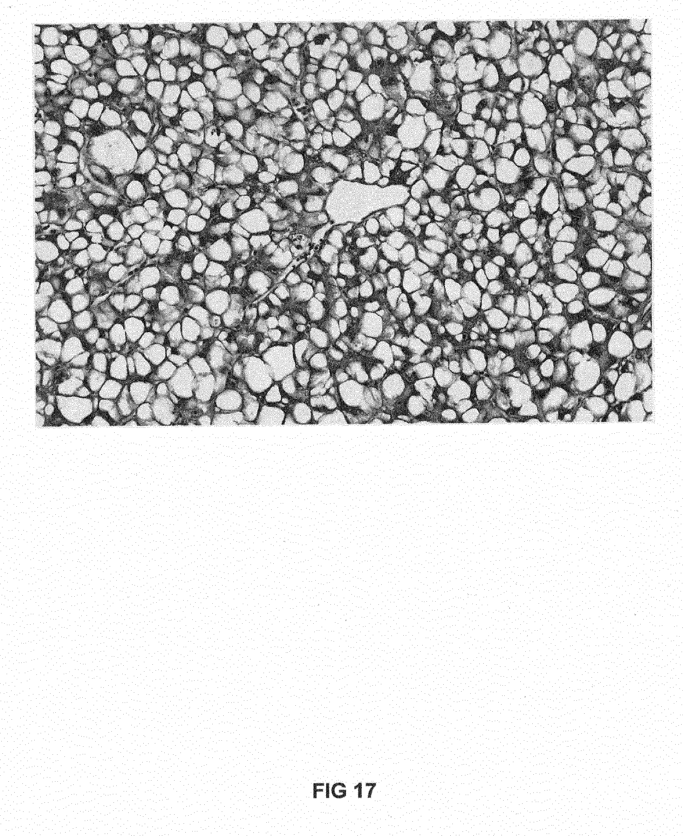

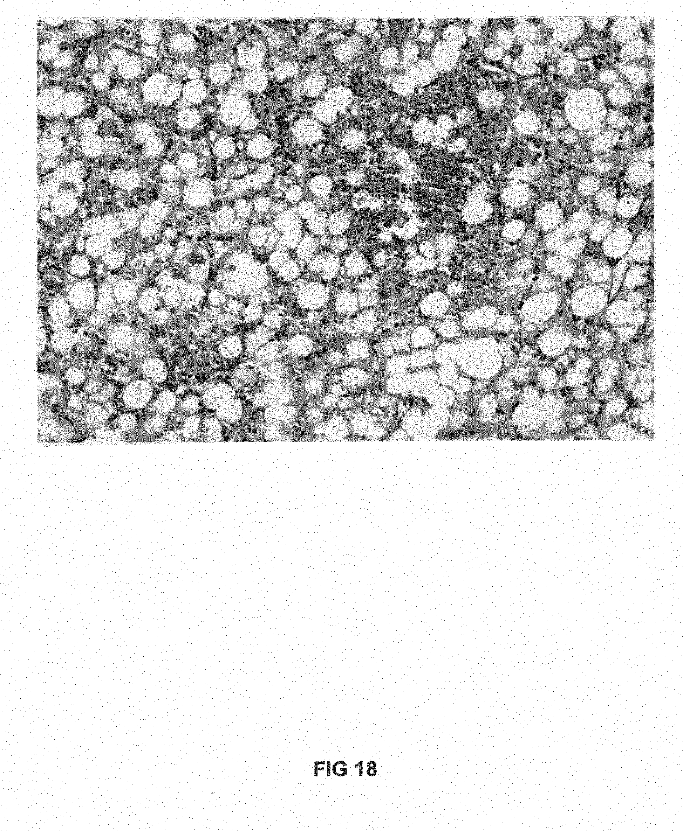

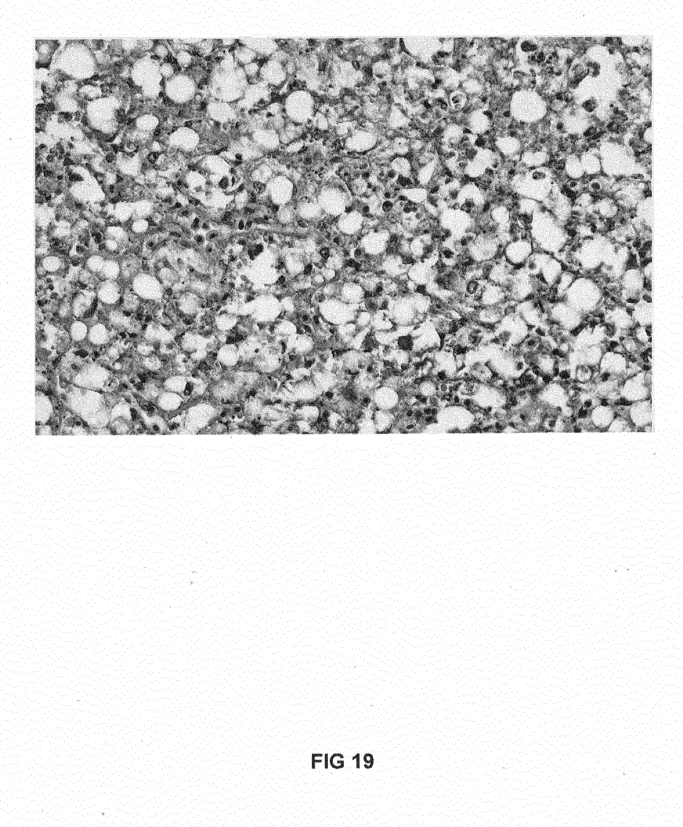

As can be seen in Figures 11-13, there is significant histological differences between the normal liver from a control fish (Fig 17), the liver having extensive tissue damage sampled at 21 days post IP challenge with LFV containing tissue homogenate (Fig 18), and a liver with extensive tissue damage sampled at 31 days post IP challenge with LFV containing tissue homogenate (Fig 19)

**Table 5**

| Histopathology of Lumpfish IP injected with LFV-containing tissue homogenate (C6T7) | | | | | | | | | | |
|---|---|---|---|---|---|---|---|---|---|---|
| **Fish no.** | **21 dpc** | | | | | **31 dpc** | | | | |
| | **Ct kidney** | **Kidney** | **Heart** | **Liver** | **Spleen** | **Ct kidney** | **Kidney** | **Heart** | **Liver** | **Spleen** |
| **1** | 11.03 | 0.5 | 0.0 | 1.0 | 0.0 | 34.60 | 0.0 | 0.0 | 0.0 | 1.0 |
| **2** | 9.70 | 0.5 | 0.5 | 3.0 | 0.0 | 14.51 | 0.0 | 0.0 | 3.0 | 1.0 |
| **3** | 12.44 | 0.0 | 0.0 | 1.0 | 0.0 | 11.90 | 1.0 | 0.0 | 3.0 | 1.0 |
| **4** | 10.75 | 1.0 | 0.0 | 3.0 | 2.0 | 12.19 | 1.0 | 0.0 | 3.0 | 3.0 |
| **5** | 23.41 | 0.5 | 0.5 | 0.0 | 2.0 | 23.45 | 1.0 | 0.0 | 0.0 | 1.0 |
| **6** | 21.77 | 0.0 | 0.0 | 0.0 | 2.0 | 21.56 | 2.0 | 1.0 | 0.0 | 1.0 |
| **7** | 10.38 | 0.5 | 0.0 | 3.0 | 3.0 | 12.76 | 1.0 | 0.0 | 3.0 | 3.0 |
| **8** | 19.55 | 0.0 | 0.0 | 0.0 | 2.0 | 22.51 | 0.0 | 0.0 | 0.0 | 3.0 |
| **9** | 13.96 | 0.5 | 0.0 | 0.0 | 2.0 | 10.97 | 1.5 | 0.0 | 3.0 | 3.0 |
| **10** | 8.66 | 0.0 | 0.0 | 3.0 | 2.0 | 11.50 | 1.0 | 0.0 | 3.0 | 2.0 |
| **Mean** | | 0.35 | 0.1 | 1.4 | 1.5 | | 0.85 | 0.1 | 1.8 | 1.9 |
| **Spearman r** | | - | 0.08 | - | 0.097 | | - | 0.17 | - | - |
| **p-value** | | 0.4397 | 0.40 | <0.0 | 0.797 | | 0.0917 | 0.20 | <0.0 | 0.114 |

| | | | | | | | | | | |
|---|---|---|---|---|---|---|---|---|---|---|
| *Weight at challenge* | | | | | | | | | | |

The mean weight at challenge was 17.8 and 94.3 grams for Lumpfish and Atlantic salmon respectively. The results are given in Table 6.

**Table 6**

| Weight at challenge | | | |
|---|---|---|---|
| **Species** | **n** | **Mean weight (± 1 SD)** | **95 % CI of mean** |
| Lumpfish | 30 | 17.8 ± 5.3 g | 16.8 - 18.8 g |
| Atlantic salmon | 30 | 94.3 ± 12.6 g | 92.0 - 96.6 g |

The described study showed that Lumpfish injected IP with tissue-homogenate containing LFV reproducibly developed characteristic signs of disease caused by LFV. Strong replication of LFV in IP injected Lumpfish was verified, as a function of time post challenge. Also, a clear correlation between the amount of LFV and the degree of histopathological damage to the liver was shown, verifying that the tissue damage observed was caused by LFV. To further support this proof, ten Lumpfish were shown to be negative for potential differential diagnoses (*Nucleospora cyclopteri*).

When non-challenged Lumpfish were cohabited with Lumpfish IP injected with LFV-containing tissue-homogenate, the virus was efficiently transmitted to the naíive Lumpfish, verifying that the virus can be transmitted horizontally to Lumpfish by natural exposure. No evidence of transmission of LFV to Atlantic salmon was observed throughout the study, indicating that LFV is unlikely to be able to be transmitted to Atlantic salmon by cohabiting with infected Lumpfish.

### Conclusion

LFV has been demonstrated that it can cause disease in Lumpfish upon IP challenge, and can further be transmitted horizontally to naive Lumpfish by natural exposure through cohabitation. Additionally, there are no indications that LFV can be transmitted to Atlantic salmon by cohabiting with infected Lumpfish.

### Example 5

### PCR Diagnostics and Analysis of Lumpfish flavirus (LFV) Structural Region Diversity Between Isolated Strains

The following primers described in Table 7, below, have been tested and found suitable for PCR diagnostics and identification of the Lumpfish flavivirus (LFV).

**Table 7**

| **PRIMER** | **FORWARD (5' - 3')** | **REVERSE (5' - 3')** |
|---|---|---|
| POL | 5'-CCTCCATTCCCAAGGAAAAT-'3 (SEQ ID NO.8) | 5'- GGTCCTGCGATGTGATCTTT - '3 (SEQ ID NO.9) |
| FRAME | 5'- GAGAACAGCCAGCAATGACA-'3 (SEQ ID NO.10) | 5'- TTCGAGTGTGATGAGCAAGC-'3 (SEQ ID NO.11) |
| LFV 1 | 5'-ACATACTTAGAAGAATAACCCGG-'3 (SEQ ID NO12) | 5'-TGCCGTTGGTAGCACTCCT-'3 (SEQ ID NO.13) |
| LFV 2 | 5'-TACGCAACAACCAAACCTATGG-'3 (SEQ ID NO.14) | 5'-GCAGATGGAATATGGTCCTG-'3 (SEQ ID NO.15) |
| LFV 3 | 5'-GACAATGCACTCACAGGATAC-'3 (SEQ ID NO.16) | 5'-GCATGAAACTGCAGTCTGTG-'3 (SEQ ID NO.17) |
| LFV 4 | 5'-TCAGAAGCCCATAACGTCGA-'3 (SEQ ID NO.18) | 5'-GATGATCGAACATGCGTCC-'3 (SEQ ID NO.19) |

The study, as described herein, examined the diversity in field isolates of LFV. Variations of nucleotide sequences of the region coding for the structural proteins of five different LFV strains isolated is described. Fourteen samples of LFV were isolated from five different sites, as outlined in Table 8, below.

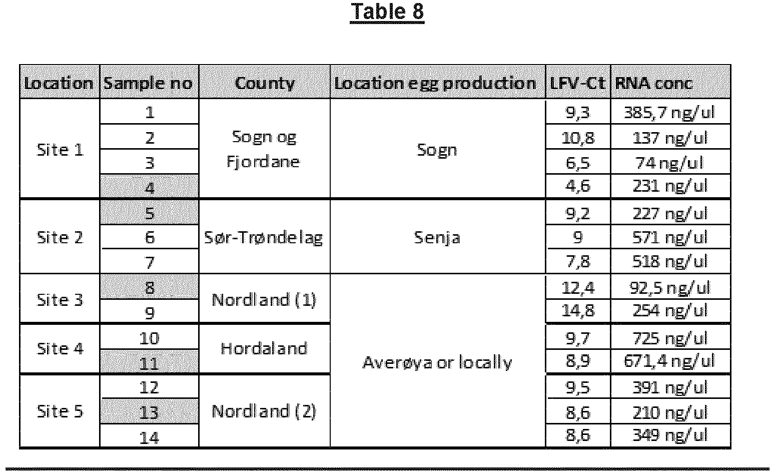

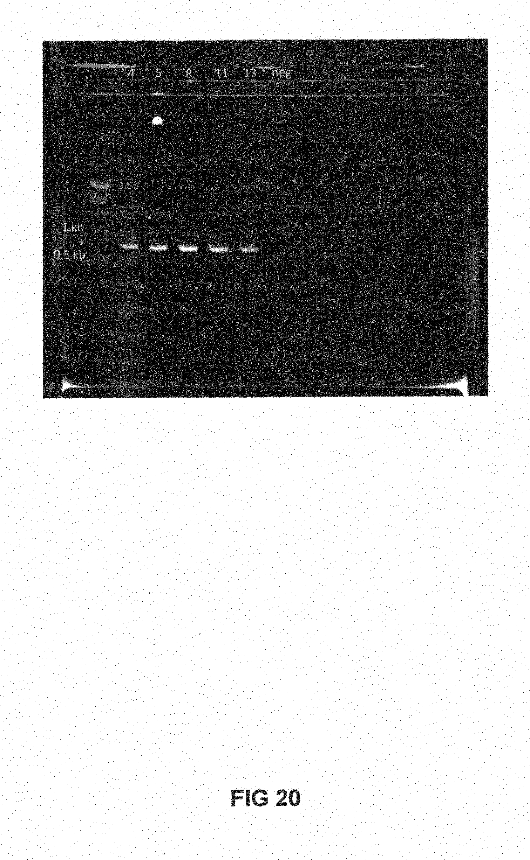

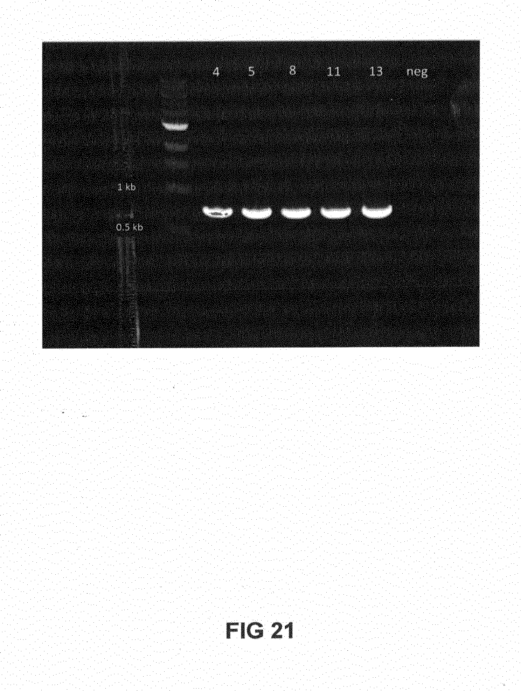

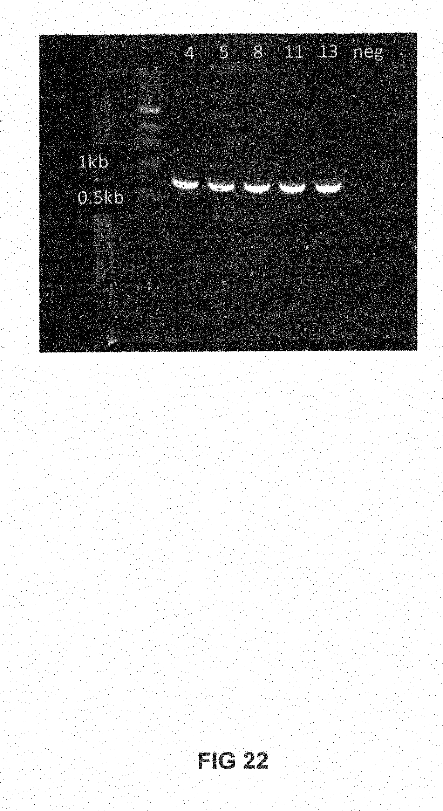

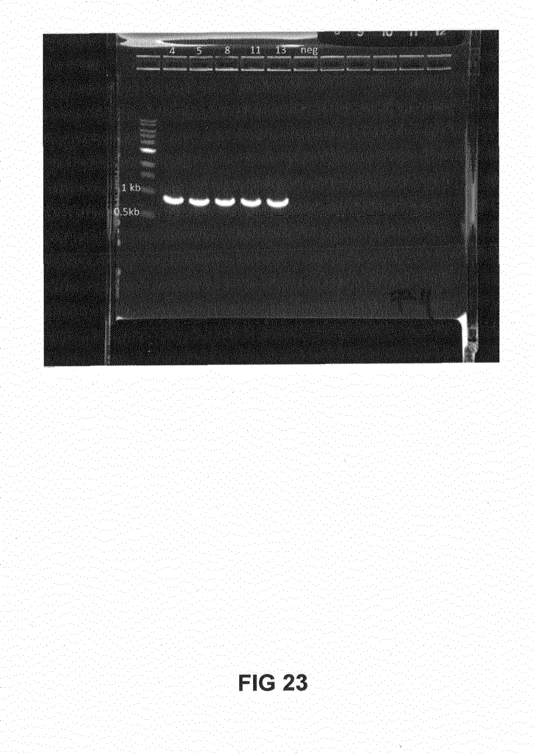

RNA was isolated and cDNA synthesis was performed using First Strand cDNA Synthesis (Invitrogen). PCR using Hot Start Taq DNA Polymerase (Qiagen) was performed with primers (described in Table 7 above) that were designed to cover the structural proteins of the LFV with 4 fragments overlapping each other. cDNA samples from fish numbers 4, 5, 8, 11 and 13 were used for analysis. The PCR products were visualized on an ethidium bromide stained 1.2% agarose gel, which confirmed the correct approximately 750 bp sized fragments. Figures 20-23 show the PCR product using the LFV1-4 forward and reverse primers.

The PCR fragments were isolated and sequenced. The sequence data was analyzed and fragments 1-4 from each fish was assembled and the five different virus strains were aligned.

### Results

The variation at nucleotide level in the 2577 nt coding for the structural proteins of LFV is generally low. Table 9 summarizes the number of nucleotide differences between the different strains.

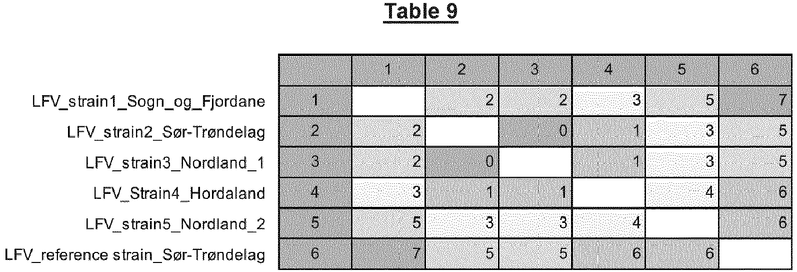

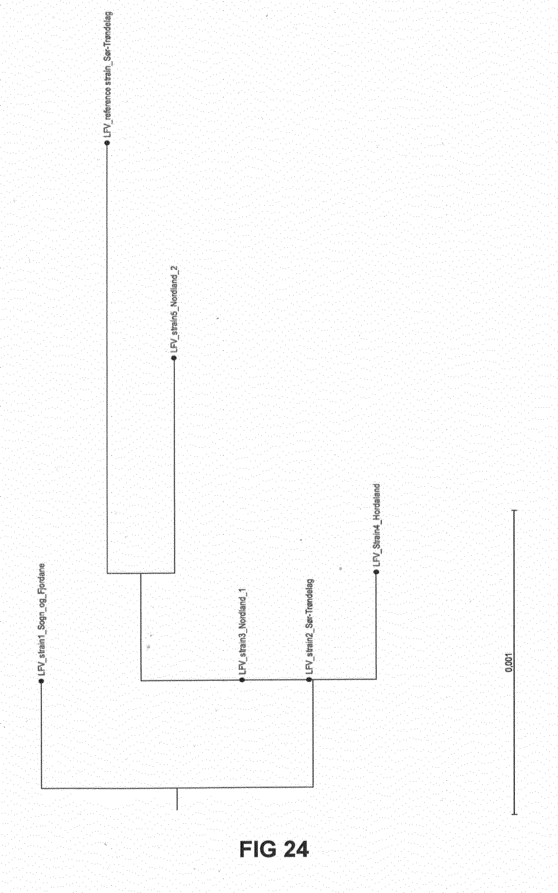

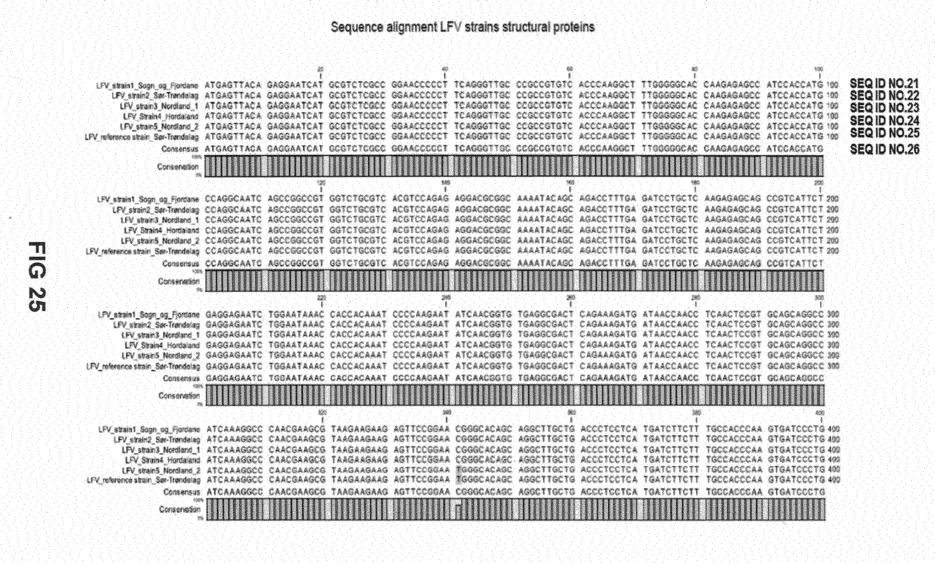

Figure 24 describes a phylogenetic tree constructed from the 2557 nucleotide sequences of the five strains studied in addition to the reference strain. Figure 25 describes the sequence alignment and the consensus sequence of the putative structural proteins of the five LFV strains studied.

The geographically spread field strains of LFV show low nucleotide variation in the region coding for the structural proteins. Although low variation, the reference strain from Sør-Trøndelag is the most distant with 5-7 nt differences compared to the other strains. It should be emphasized that a predominant part of the Lumpfish eggs in Norway are produced at one single localization in Norway (Averøya), where wild Lumpfish ready for spawning are caught locally and the eggs distributed to farms in Norway.
## Claims

1. An isolated flavivirus comprising a ribonucleic acid genome sequence having at least 70% sequence identity to SEQ ID NO.20.

2. The virus of claim 1 wherein the genome sequence comprises ribonucleic acid sequence having at least 80% sequence identity to SEQ ID NO.20.

3. The virus of claim 1 wherein the genome sequence comprises ribonucleic acid sequence having at least 90% sequence identity to SEQ ID NO.20.

4. The virus of claim 1 wherein the genome sequence comprises ribonucleic acid sequence having at least 95% sequence identity to SEQ ID NO.20

5. An isolated DNA nucleic acid sequence originating from the virus of any of claims 1 to 4 wherein the nucleic acid sequence is selected from the group consisting of: SEQ ID NO. 1, SEQ ID NO.2, SEQ ID NO. 4, SEQ ID NO. 6, and variants thereof having at least 70% sequence identity to any of the sequences SEQ ID NO. 1, SEQ ID NO. 2, SEQ ID NO. 4, or SEQ ID NO. 6.

6. An isolated DNA nucleic acid sequence according to claim 5 wherein the nucleic acid sequence has at least 80% sequence identity to a nucleic acid selected from the group consisting of: SEQ ID NO. 1, SEQ ID NO. 2, SEQ ID NO. 4, and SEQ ID NO. 6.

7. An isolated DNA nucleic acid sequence according to claim 5 wherein the nucleic acid sequence has at least 90% sequence identity to a nucleic acid selected from the group consisting of: SEQ ID NO. 1, SEQ ID NO. 2, SEQ ID NO. 4, and SEQ ID NO. 6.

8. An isolated DNA nucleic acid sequence according to claim 5 wherein the nucleic acid sequence has at least 95% sequence identity to a nucleic acid selected from the group consisting of: SEQ ID NO. 1, SEQ ID NO. 2, SEQ ID NO. 4, and SEQ ID NO. 6.

9. A vector comprising a nucleic acid sequence according to claims 5 to 8.

10. A vector according to claim 9 wherein the nucleic acid sequence is operatively linked to control sequences directing the expression of said nucleic acid sequences.

11. A host cell comprising a vector according claim 9 or claim 10.

## Patentansprüche

1. Isoliertes Flavivirus, umfassend eine Ribonucleinsäure-Genomsequenz, welche mindestens 70% Sequenzidentität zu SEQ ID NO. 20 besitzt.

2. Virus gemäß Anspruch 1, wobei die Genomsequenz eine Ribonucleinsäuresequenz umfasst, welche mindestens 80% Sequenzidentität zu SEQ ID NO. 20 besitzt.

3. Virus gemäß Anspruch 1, wobei die Genomsequenz eine Ribonucleinsäuresequenz umfasst, welche mindestens 90% Sequenzidentität zu SEQ ID NO. 20 besitzt.

4. Virus gemäß Anspruch 1, wobei die Genomsequenz eine Ribonucleinsäuresequenz umfasst, welche mindestens 95% Sequenzidentität zu SEQ ID NO. 20 besitzt.

5. Isolierte DNA-Nucleinsäuresequenz, welche aus dem Virus gemäß einem der Ansprüche 1 bis 4 stammt, wobei die Nucleinsäuresequenz ausgewählt ist aus der Gruppe, bestehend aus SEQ ID NO. 1, SEQ ID NO. 2, SEQ ID NO. 4, SEQ ID NO. 6 und Varianten davon, welche mindestens 70% Sequenzidentität zu einer der Sequenzen SEQ ID NO. 1, SEQ ID NO. 2, SEQ ID NO. 4 oder SEQ ID NO. 6 besitzen.

6. Isolierte DNA-Nucleinsäuresequenz gemäß Anspruch 5, wobei die Nucleinsäuresequenz mindestens 80% Sequenzidentität zu einer Nucleinsäure, ausgewählt aus der Gruppe, bestehend aus SEQ ID NO. 1, SEQ ID NO. 2, SEQ ID NO. 4 und SEQ ID NO. 6, besitzt.

7. Isolierte DNA-Nucleinsäuresequenz gemäß Anspruch 5, wobei die Nucleinsäuresequenz mindestens 90% Sequenzidentität zu einer Nucleinsäure, ausgewählt aus der Gruppe, bestehend aus SEQ ID NO. 1, SEQ ID NO. 2, SEQ ID NO. 4 und SEQ ID NO. 6, besitzt.

8. Isolierte DNA-Nucleinsäuresequenz gemäß Anspruch 5, wobei die Nucleinsäuresequenz mindestens 95% Sequenzidentität zu einer Nucleinsäure, ausgewählt aus der Gruppe, bestehend aus SEQ ID NO. 1, SEQ ID NO. 2, SEQ ID NO. 4 und SEQ ID NO. 6, besitzt.

9. Vektor, umfassend eine Nucleinsäuresequenz gemäß den Ansprüchen 5 bis 8.

10. Vektor gemäß Anspruch 9, wobei die Nucleinsäuresequenz operativ verknüpft ist mit Kontrollsequenzen, welche die Expression der Nucleinsäuresequenzen steuern.

11. Wirtszelle, umfassend einen Vektor gemäß Anspruch 9 oder Anspruch 10.

## Revendications

1. Flavivirus isolé comprenant une séquence génomique d'acide ribonucléique présentant au moins 70 % d'identité de séquence avec SEQ ID NO. 20.

2. Virus selon la revendication 1, dans lequel la séquence génomique comprend une séquence d'acide ribonucléique présentant au moins 80 % d'identité de séquence avec SEQ ID NO. 20.

3. Virus selon la revendication 1, dans lequel la séquence génomique comprend une séquence d'acide ribonucléique présentant au moins 90 % d'identité de séquence avec SEQ ID NO. 20.

4. Virus selon la revendication 1, dans lequel la séquence génomique comprend une séquence d'acide ribonucléique présentant au moins 95 % d'identité de séquence avec SEQ ID NO. 20.

5. Séquence isolée d'acide nucléique d'ADN provenant du virus selon l'une quelconque des revendications 1 à 4, dans laquelle la séquence d'acide nucléique est sélectionnée dans le groupe constitué de : SEQ ID NO. 1, SEQ ID NO. 2, SEQ ID NO. 4, SEQ ID NO. 6, et les variants de celles-ci présentant au moins 70 % d'identité de séquence avec l'une quelconque des séquences SEQ ID NO. 1, SEQ ID NO. 2, SEQ ID NO. 4, ou SEQ ID NO. 6.

6. Séquence isolée d'acide nucléique d'ADN selon la revendication 5, dans laquelle la séquence d'acide nucléique présente au moins 80 % d'identité de séquence avec un acide nucléique sélectionné dans le groupe constitué de : SEQ ID NO. 1, SEQ ID NO. 2, SEQ ID NO. 4 et SEQ ID NO. 6.

7. Séquence isolée d'acide nucléique d'ADN selon la revendication 5, dans laquelle la séquence d'acide nucléique présente au moins 90 % d'identité de séquence avec un acide nucléique sélectionné dans le groupe constitué de : SEQ ID NO. 1, SEQ ID NO. 2, SEQ ID NO. 4, et SEQ ID NO. 6.

8. Séquence isolée d'acide nucléique d'ADN selon la revendication 5, dans laquelle la séquence d'acide nucléique présente au moins 95 % d'identité de séquence avec un acide nucléique sélectionné dans le groupe constitué de : SEQ ID NO. 1, SEQ ID NO. 2, SEQ ID NO. 4, et SEQ ID NO. 6.

9. Vecteur comprenant une séquence d'acide nucléique selon les revendications 5 à 8.

10. Vecteur selon la revendication 9, dans lequel la séquence d'acide nucléique est liée de manière fonctionnelle à des séquences de contrôle dirigeant l'expression desdites séquences d'acide nucléique.

11. Cellule hôte comprenant un vecteur selon la revendication 9 ou la revendication 10.
